# EUROPEAN PATENT APPLICATION

(11) **EP 4 108 298 A1**
(43) Date of publication of application: **28.12.2022**
(21) Application number: 21181202.9
(22) Date of filing: 23.06.2021
(51) Int. Cl.: A61P 31/20, C07K 16/08, G01N 33/569

(54) **APPLICATION OF HCMV GP34- AND GP68-SPECIFIC ANTIBODIES AND FRAGMENTS THEREOF FOR PREVENTION, THERAPY AND DIAGNOSTICS OF HCMV DISEASE**

(71) Applicant: Albert-Ludwigs-Universität Freiburg, 79085 Freiburg im Breisgau (DE); University of Rijeka, Faculty of Medicine, 51000 Rijeka (HR)
(72) Inventor: Jonjic, Stipan, 51000 Rijeka (HR); Lenac Rovis, Tihana, 51215 Kastav (HR); Hengel, Hartmut, 79194 Grundelfingen (DE); Hoffmann, Katja, 79106 Freiburg (DE); Kolb, Philipp, 79102 Freiburg (DE)
(74) Representative: Lederer & Keller Patentanwälte Partnerschaft mbB

(57) **Abstract**

The present application relates to antibodies which bind to the extracellular domain of an Fc-gamma-binding glycoprotein gp34 and gp68 of human cytomegalovirus.

This patent application was supported by the grant "Strengthening the capacity of CerVirVac for research in virus immunology and vaccinology", KK.01.1.1.01.0006, awarded to the Scientific Centre of Excellence for Virus Immunology and Vaccines and co-financed by the European Regional Development Fund.

## Description

Treatment of CMV-related diseases and prevention of damage due to congenital CMV (cCMV) infection are one of the most pressing challenges of cytomegalovirus research. The US Institute of Medicine of the National Academy of Science declared the development of a vaccine to prevent cCMV as one of the highest priorities. Due to the high health impact of CMV disease, many research consortia and pharmaceutical companies are actively pursuing the development of an efficient CMV vaccine.

As human cytomegalovirus (HCMV) is best known to efficiently evade immune responses including humoral immunity it was assumed that HCMV encoded FcyR antagonists gp34 and gp68 to be responsible for limiting the efficacy of endogenous and therapeutic IgG antibodies directed against HCMV antigens. gp34 and gp68 were identified as HCMV *RL11*/*gp34* and *UL119_118*/*gp68* encoded Fcy-binding glycoproteins and their ability to mediate evasion from FcyR-dependent IgG effector functions was described. The experiments performed according to the present invention were usually made with the soluble gp34 (sgp34), the sequence thereof is shown in SEQ ID NO:1.

Interfering with IgG binding by gp34 and gp68 may thus restore FcyR-mediated immune responses (e.g. Antibody-dependent cellular cytotoxicity (ADCC) of NK-cells or Antibody-dependent cellular Phagocytosis (ADCP) of phagocytic cells). gp34 and gp68 are well-conserved among HCMV wild-type strains and patient isolates and make them perfect targets for therapeutic intervention. Utilizing this knowledge for gp34 and gp68 targeting first mouse monoclonal antibodies (mAbs) were generated to block their immunoevasive function(s) and restoring HCMV-IgG effector functions. Evidence is provided showing that gp34 interacts with additional human immune ligands, extending its spectrum for immunoevasive functions. On this basis, the generated mAbs to gp34 may gain an even broader therapeutic and diagnostic applicability.

In the first round of experiments parts of the glycoproteins gp34 and gp68 (only extracellular domain, ECD) were used for the generation of mouse monoclonal antibodies. The glycoprotein gp34 binds to the Fc part of human IgG1, IgG2, IgG3, IgG4, mouse IgG1, mouse IgG2a and mouse IgG3, rat IgG and rabbit IgG. For the immunization experiments two different proteins derived from gp34 were used.

The amino acid sequence having the designation sol gp34 wt (SEQ ID NO:1) was used for immunization.

The amino acid sequence ID NO:1 has the following sequence:

MQTYSTPLTLVIVTSLFLFTTQGSSSNAVEPTKKPLKLANYRATCEDRTRTLVTRLNTSHH
SVVWQRYDIYSRYMRRMPPLCIITDAYKETTRQGGAAFACTRQYTGDVEAFYLIIHPRSFC
RALETRRCFYPGPGRVVVTDSQEADRAIISDLKRQWSGKPIPNPLLGLDSTRTGHHHHHH

(SEQ ID NO:1) comprising:
a signal peptide; 1-23 aa = SP
an Ig-like domain (32-137 aa)
a minimal IgG-Fc binding domain (based on analysis using truncated gp34 variants): 1-140 aa truncation variant: 1-124 aa → lost its ability to bind IgG-Fc,
a V5 Tag (GKPIPNPLLGLDSTRTG) and
poly-His Tag (HHHHHH).

In addition thereto a modified sequence from gp34 having the designation sol gp34mtrp (more precisely gp34 1-179 W65F) (SEQ ID NO:2) was used. This protein corresponds with the extracellular domain of gp34 wt having amino acid sequences 1-179 whereby, however, the amino acid tryptophan at position 65 (in the one letter code "W") was changed to phenylalanine (in the one letter code "F"). The correct designation of the mutant is therefore W65F.

SEQ ID NO:2 has the following amino acid sequence: 
MQTYSTPLTLVIVTSLFLFTTQGSSSNAVEPTKKPLKLANYRATCEDRTRTLVTRLNTSHH
SVVFQRYDIYSRYMRRMPPLCIITDAYKETTRQGGAAFACTRQNLTLYNLTVKDTGVYLLQ
DQYTGDVEAFYLIIHPRSFCRALETRRCFYPGPGRVVVTDSQEADRAIISDLKRQWSGKPI
PNPLLGLDSTRTGHHHHHH (SEQ ID NO:2)
with an SP (signal peptide; 1-23 aa),
an Ig-like domain (32-137 aa),
a minimal IgG-Fc binding domain (based on analysis using truncated gp34 variants): 1-140 aa truncation variant: 1-124 aa → lost its ability to bind IgG-Fc,
with mtrp position: W65F (Trp → Phe),
a V5 Tag (GKPIPNPLLGLDSTRTG) and
a His Tag (HHHHHH).

The purpose of using those two antigens sol gp34 wt and sol gp34 mtrp was that the binding of the gp34 wt is different from the binding properties of the mutant. gp34 wt binds to human IgG1, IgG2, IgG3, IgG4, to mouse IgG1, to mouse IgG2a and mouse IgG3 and to rat IgG and rabbit IgG. The mutated gp34 has impaired binding properties for human IgG binding, looses the binding capability to all mouse IgGs, which was important since the immunization with gp34 wt protein might not result in the generation of specific antibodies.

As a further antigen for the immunization of mice in order to produce monoclonal antibodies the antigen derived from glycoprotein 68 having the designation sol gp68 which corresponds to the extracellular domain of gp68 was used. The sequence of this protein is shown as SEQ ID NO:3.

SEQ ID NO:3 has the following amino acid sequence: 
MCSVLAIALVVALLGDMHPGVKSSTTSAVTSPSNTTVTSTTSISTSNNVTSAVTTTVQTST
SSASTSVIATTQKEGHLYTVNCEASYSHDQVSLNATCKVILLNNTKNPDILSVTCYARTDC
KGPFTQVGYLSAFPPDNEGKLHLSYNATAQELLISGLRPQETTEYTCSFFSWGRHHNAT
WDLFTYPIYAVYGTRLNATTMRVRVLLQEHEHCLLNGSSLYHPNSTVHLHQGNQLIPPWN
ISNVTYNGQRLREFVFYLNGTYTVVRLHVQIAGRSFTTTYVFIKSDPLFEDGKPIPNPLLGL
DSTRTGHHHHHH (SEQ ID NO:3)
 with
an SP (signal peptide; 1-23 aa),
an Ig-like domain(32-137 aa),
a V5 Tag (GKPIPNPLLGLDSTRTG) and
a poly His Tag (HHHHHH).

The present invention relates to antibodies or antigen binding fragments thereof which bind to the extracellular domain of an Fc-gamma-binding glycoprotein of human cytomegalovirus. In one preferred embodiment the antibody or antigen binding fragment thereof binds to the Fc-gamma-binding glycoprotein which is gp34. The sequence to which the antibody or antigen binding fragment binds is the Ig-like domain of SEQ ID NO:1. The Ig-like domain stretching from amino acid 32 to 137 is part of the extracellular domain of gp34.

It should be noted that the term "antibody or antigen binding fragment thereof" encompasses all derivatives of antibodies which may be used for therapeutic and/or diagnostic purposes such as for example single-chain antigen binding fragments or nanobodies.

In an alternative embodiment the antibody and/or antigen binding fragments thereof bind to the mutated sequence of gp34 having SEQ ID NO:2. The mutated sequence differs from the wild type sequence only in position 65 whereby the wild type has a tryptophan at amino acid position 65 and the mutated sequence has a phenylalanine at position 65. The two sequences were used in the course of the present invention in order to investigate the different functional parts of the glycoprotein gp34.

The preliminary experiments were performed with monoclonal antibodies derived from mice. It is, however, plainly evident that mouse antibodies cannot be used in therapy. It is possible to use mouse antibodies for diagnostic purposes, but when applied to human patients the human body develops antibodies directed against the mouse-specific structures.

In order to overcome those difficulties it is common practice to humanize the antibodies. For the humanization of antibodies which can be done by using suitable computer programs it is searched for human antibodies which have the highest similarity or homology to the effective mouse antibodies. The framework of such human antibodies is used and the CDRs are "transplanted" from the mouse antibodies into the human framework. Very often it is required to adapt the sequences in order to maintain the best binding properties. Therefore, it may be required that one or two of the CDR sequences are replaced in the course of humanization. The advantage of having good working mouse monoclonal antibodies is the knowledge which CDRs can be best used for the humanization.

It is therefore a preferred embodiment of the present invention that the humanized antibodies and/or antigen binding fragments thereof have a very high homology to the preferred amino acid sequences of the best working monoclonal antibodies. Preferably the sequence homology or sequence identity is at least 80% with the mouse sequences disclosed herein. In more preferred embodiments the sequence identity or sequence homology is at least 85% and in especially preferred embodiments the sequence identity and/or sequence homology is at least 95% to the mouse sequences. The highest homology and/or identity is required in the CDR regions whereas in the framework areas a lower homology is acceptable. Other properties like the efficient expression in host cells or the further processability for pharmaceutical uses may require additional adaptations of the amino acid sequences. Such preferred sequences are in particular SEQ ID NO:14 - SEQ ID NO:59.

The property of the antibodies and antigen binding fragments thereof is important as such antibodies are able to block the binding of the glycoprotein gp34 to the Fc part of the immunoglobulin-gamma (IgG). By this interaction the counter-defense mechanism of the human cytomegalovirus by antagonizing the antibodies of the host is substantially weakened. It was demonstrated that gp34 and gp68 of human cytomegalovirus bind to the Fc parts of immunoglobulin-gamma whereby the host defense mechanisms are impaired. When this defense mechanism of HCMV is inhibited by the antibodies according to the invention the immune defense of the patient infected with HCMV is substantially improved.

Another aspect of the present invention is that the antibodies and/or antigen binding fragments thereof block the binding of glycoprotein gp34 to C-reactive protein. C-reactive protein is a well-known mediator of the immune response of the host cell (patient). By blocking the usual function of glycoprotein gp34 with the help of an antibody or antigen binding fragment thereof according to the invention the immune reaction of the infected patient is substantially improved.

A further very surprising effect of the present invention is that the antibodies and/or antigen binding fragments thereof according to the invention are able to block binding to the neonatal Fc receptor. This is of utmost importance in the treatment of pregnant women when adverse effects of the virus to the fetus are to be avoided.

In an alternative embodiment of the present invention the antibodies and/or antigen binding fragments thereof bind to glycoprotein gp68.

In addition to the therapeutic uses as described above the antibodies and/or antigen binding fragments of the present invention can be used for diagnostic purposes. In such diagnostic purposes the antibody and/or antigen binding fragments thereof are reacted with biological samples derived from a potentially infected subject. Such samples can be cell fragments obtained by biopsy or from biological fluids like blood, serum, plasma, saliva or liquor.

In general the antibody and/or antigen binding fragment thereof according to the invention is reacted with the biological sample in order to allow the binding of the antibodies to the antigen gp68 and/or gp34. Subsequently the unbound antibodies and/or antigen binding fragments thereof are removed usually by washing and thereafter the antigen/antibody complex is detected by label. Such a label can already be incorporated into the antibody and/or antigen binding fragment or it can be detected by a suitable detection means. When for example monoclonal mouse antibodies are used the detection can be achieved by using antibodies probably derived from rabbit or goat and directed against the Fc part of mouse antibodies.

The following mouse monoclonal antibodies were generated and characterized, the genes were sequenced and expressed to produce recombinant IgG:
- 8 mAbs raised against the ectodomain of gp68 (1-292aa) capable to block IgG binding by gp68
- 9 mAbs raised against the ectodomain (1-179aa) of gp34, some of which block the IgG-gp34 interaction
- 6 mAbs raised against the point-mutation W65F (position 65 Trp→Phe of the ectodomain (1-179aa) of gp34. The mAbs recognize sgp34wt protein but the mAbs have only minor or no effect on gp34-IgG binding but could prevent binding of further gp34 ligands (namely CRP and FcRn).

Antigen specificity of the generated mAbs for the immunizing antigen was approved by ELISA, flow cytometry, immunoprecipitation and Western Blot, resp., and specifically controlled for the contributing binding effects of Fcγ. Blocking activities of gp34 and gp68 by mAbs was assessed in FcyR reporter cell activation assays.

Specifically, all anti-gp68 mAbs detect gp68 in ELISA, whereas only two of them recognize gp68 in western blot (gp68.01 and gp68.02). All tested anti-gp68 mAbs exhibit a strong blocking capacity of Fcy binding to gp68 in a dose-dependent manner.

Two different antigens (SEQ ID NOs:1 and 2) were used to generate mAbs to gp34, resulting in two kinds of antibody groups. First, antibodies were raised against sgp34wt protein which shows a strong IgG-Fc binding and has a strong potential to antagonize human FcyR-activation, named sgp34wt mAbs (wt.07/wt.08/wt.09/wt.10/wt.11/wt.12/wt.13/wt.14 and wt.15).

Second antibodies raised against gp34 W65F (mutated sequence) which is impaired for Fcy-binding and lacks antagonization potential of FcyR-activation, named gp34mtrp mAbs (mtrp.01/mtrp02/mtrp.03/mtrp.04/mtrp.05 and mtrp.06) were provided.

Both groups of mAbs detect immobilized sgp34wt by ELISA. In contrast, none of the mAbs resulting from the immunization using native sgp34wt protein is able to detect structurally affected gp34 W65F in ELISA, as well as in western blot.

All gp34 W65F mAbs specifically recognize gp34 W65F protein, as well as the sgp34wt protein.

With regard to the potential of our anti-gp34 mAbs to interfere and block IgG binding by vFcyR gp34, only selected mAbs of the anti-sgp34wt mAb group show strong blocking capacities in a dose-dependent manner:
wt.09: IC50 63 ng/ml, wt.10: IC50 89 ng/ml, wt.11: IC50 77 ng/ml, wt.12: IC50 70 ng/ml, wt.15: IC50 120 ng/ml.

Some antibodies have an intermediate effect on sgp34wt-IgG binding at least reducing up to 50% of binding by the vFcyR:
wt.07: IC50 447 ng/ml, wt.08: IC50 494 ng/ml, mtrp.03: IC50 152 ng/ml, mtrp.04: IC50 147 ng/ml, mtrp.01: IC50 239 ng/ml; mtrp.02: IC50 330 ng/ml.

In contrast, IC50 values of mtrp.06 are IC50 4456 ng/ml and of mouse IgG1 isotype control IC50 9690 ng/ml.

To validate the functionality and blocking capacity of the mAbs using recombinantly generated Fab-fragments Fab-genes were sequenced and analyzed including framework (FR) and complementary-determining regions (CDRs). Furthermore, humanized Fab sequences were generated from selected, promising mAb candidates. Those might be further optimized by glyco-engineering and/or Fc-modifications addressing beneficial immune-reactions and avoiding unwanted actions. Also, half-life of humanized mAbs might be modified by introducing mutations within the Fc-region.

Besides IgG, FcyR antagonist gp34 recognizes further ligands involved in crucial defense mechanisms including
i) the B-cell receptor of IgG+ memory B-cells,
ii) C-reactive protein (CRP), playing important functions in complement activation but also host FcyR-dependent phagocytosis and
iii) the neonatal FcR (FcRn) potentially involved in the transplacental transmission of HCMV leading to congenital HCMV infection.

The potential of the sgp34wt and gp34W65F mAbs to interrupt the interaction of gp34 with IgGFc as well as other above mentioned ligands was investigated. It is assumed that no immunomodulatory protein of microbial or pharmaceutical origin comparable to gp34 has been reported so far.

In addition, mAbs or binding fragments thereof according to the invention may be useful for highly sensitive and specific detection of the HCMV antigens gp34 and gp68 in clinical specimens as a diagnostic tool either in tissue sections or in immunoassays.

Taken together, the monoclonal antibodies or binding fragments thereof represent a novel, unique and promising approach to block immunoevasive functions of gp34 and gp68, thereby restoring immune effector functions of IgG and further ligands of gp34 including CRP, FcRn and IgG-BCR. Antibodies or antigen binding fragments specific for gp34 and gp68 can
1) improve the efficacy of HCMV-IgG,
2) improve B cell immunity in HCMV-infected patients,
3) prevent the transmission of HCMV by cellular transcytosis and
4) detect gp34 and gp68 in clinical specimens.

The monoclonal antibodies were raised against the IgG-Fc-binding ectodomains (ECDs) of the glycoproteins gp34 (encoded by the gene *RL11*) and gp68 (encoded by the gene *UL119-*118) of human Cytomegalovirus (HCMV). gp34 and gp68 are immune evasion molecules acting as viral Fcy-Receptors (vFcyRs) antagonizing IgG-mediated immune responses of host FcyR-positive immune cells e.g. phagocytes and NK-cells.

gp34 and gp68 were identified as HCMV immune evasive molecules and further characterized. gp34 and gp68 both antagonize host FcγRI,-II and -III activation through interactions with the ligand IgG-Fc. Some of the mAbs according to the invention possess the potential to block this interaction between vFcyR gp34 or gp68 with IgG-Fc. By blocking vFcyR - IgG interaction, some of the mAbs described herein are able to restore IgG-mediated host FcyRs activation.

The following mAbs were generated:
- 8 mAbs raised against vFcyR gp68 with varying inhibitory potential to bind and block gp68-IgG binding.
- 9 mAbs raised against vFcyR gp34 wildtype (wt), with varying inhibitory potential to block IgG-gp34 binding; some mAbs that show no effect on IgG interaction however interfere with other gp34 ligands (vide infra).
- 6 mAbs raised against a point-mutation of gp34 (gp34mtrp). The mAbs recognize sgp34wt but differ in their inhibitory potential from mAbs raised against gp34 wt.

Surprisingly additional interaction partners of the viral glycoprotein gp34 were identified which includes binding to (i) the IgG-B-cell-receptor (BCR) on memory B-cells, (ii) C-reactive protein (CRP) and (iii) the neonatal Fc-Receptor (FcRn). The findings allow further uses of gp34 as a target to prevent HCMV dissemination and pathogenesis. No other protein of microbial or pharmaceutical origin seems to have comparable features as gp34.

Many experiments were performed and the results thereof are shown in the Figures. The following explanation of the Figures illustrates together with the graphs the present invention. In the present application the following abbreviations were used:
- BCR: B cell receptor
- BSA: bovine serum albumin
- cCMV: congenital cytomegalovirus
- CDR: complementary Determining Region
- CFA: complete Freund's adjuvant
- CMV: cytomegalovirus
- CRP: C-reactive protein
- ECD: ectodomain
- ELISA: Enzyme-linked immune-sorbent assay
- Fab: fragment antigen binding
- Fc: fragment crytallizable
- FcRn: neonatal Fc-receptor
- Fcγ: fragment crytallizable of immunoglobulin G subclass
- FcγR: Fcy (gamma)-receptor
- FR: frame work region
- gp: glycoprotein
- HeLa: human epithelial cells, cervix
- hCMV: human cytomegalovirus
- HEK 293T: Human embryonic kidney 293 cells
- IC50: half maximal inhibitory concentration
- ICOS-L: Inducible T Cell co-stimulator ligand
- IFA: incomplete Freund's adjuvant
- IgG: immunoglobulin G
- mAb: monoclonal antibody
- Moi: multiplicity of infection
- Mtrp: mutated tryptophan
- MW: mother-well
- SDS: Sodium dodecyl sulfate
- SP: Signal peptide
- TM: transmembrane domain
- VH: variable heavy chain
- VL: variable light chain

To protect from antibody-mediated anti-viral immune responses herpesviruses evolved immunomodulatory molecules, so-called Fcy-Receptors (vFcyRs), to counteract the host immune system.

IgG-dependent triggering of host FcyRs found on many immune cells leads to activation of phagocytic cells or antibody dependent cellular cytotoxicity (ADCC) by NK-cells. To antagonize IgG effector functions members of the herpesvirus family express their own, viral FcyRs to manipulate or compete with host FcyRs for ligand binding and thus interfere for example with IgG-mediated immune responses.

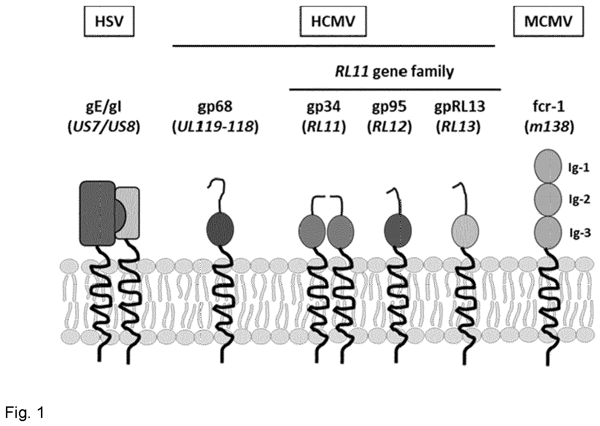

HCMV is equipped with several antagonists, i.e. gp34, gp68, gp95 and a fourth candidate, gpRL13 as shown schematically in Fig. 1. Those type-1 glycoproteins have in common to bind to the Fc-part of immunoglobulin G.

Fig. 1 shows herpesviral-encoded viral FcyRs. Several members of the herpesviral family express their own IgG-binding molecules to counteract and interfere with host FcyRs. Remarkably, in contrast to other members of this family, HCMV encodes not only one, but several of those antagonists: gp34 (*RL11*)*,* gp68 (*UL119-118*)*,* gp95 (*RL12*) and described only recently, gpRL13 (*RL13*)*.* Here, gp34, gp95 and gpRL13 belonging to the *RL11* gene family encoding several immune evasion molecules interfering with immune cells.

### Characterization of the interaction of vFcγR gp34 with human IgG

Viral glycoproteins gp34 and gp68 bind to the Fc-part of an IgG-molecule. For the experiments and the demonstration of their IgG-binding ability, recombinant proteins based on the HCMV AD169 strain were expressed by transient or stable transfection into HEK-293-T cells.

An ELISA-based assay was used to demonstrate binding of soluble, recombinant sgp34wt, a mutant thereof (gp34mtrp: containing one amino acid change from tryptophan to phenylalanine at position 65, leading to drastically impaired IgG-Fc-binding and inhibition), vFcyR gp68 and soluble ICOS-L as control protein (Inducible T Cell co-stimulator ligand for the T-cell-specific cell surface receptor ICOS).

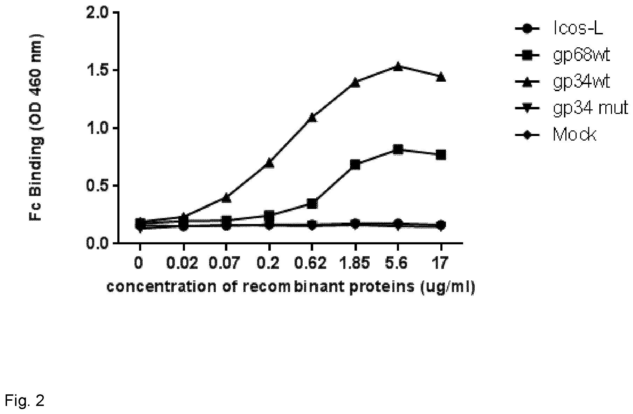

The binding to immobilized human IgG-Fc fragment by soluble, V5-His-tagged proteins was analyzed in ELISA in a dose-dependent manner. sgp34wt and gp68 are Fc-binding glycoproteins as shown in Fig. 2. A mutant version of sgp34wt, sgp34mtrp, lacks the ability for IgG-binding. In addition, the binding ELISA demonstrates ICOS-L to be a non-Fc-binding molecule serving as a suitable negative control during our assays.

Fig. 2: Dose-dependent IgG-Fc binding by vFcyRs gp34 and gp68. Binding to immobilized human IgG-Fc fragment by graded concentrations of recombinant vFcyRs sgp34wt and gp68 is demonstrated in ELISA. Hence, sgp34wt exhibits a stronger IgG-binding ability compared to sol gp68. The gp34 mtrp mutant shows no Fc binding as human ICOS-L and binding buffer (mock-treated control).

Viral Fc-binding molecules gp34 and gp68 interact and bind towards all human IgG-subclasses.

Surprisingly further ligands especially for glycoprotein gp34 were identified. Those interaction partners includes C-reactive protein (CRP), a key molecule of inflammation and part of the innate immune system as well as activator of the complement system, and the neonatal Fc-Receptor (FcRn).

C-reactive proteins share many structural but also functional features with antibodies, besides the obvious fact, to show binding towards Fc-Receptors. Also, since gp34 shows strong interference to the classical/canonical host FcyRs (FcγRI, FcyRlla and -Ilb, FcγRIII), this glycoprotein surprisingly interacts with the non-classical Fc-Receptor FcRn.

Beside manipulation(s) of IgG-mediated antiviral immune response, HCMV encodes for a molecule able to evade from CRP-mediated activation e.g. by phagocytes or by the complement system. On the other hand, interactions with the neonatal Fc-Receptor, involved in IgG half-life, transport (also placental transfer) and recycling represents new potential opportunities for the virus achieved by vFcyR gp34. This finding is the basis for new uses of the mAbs and binding fragments thereof according to the invention.

### Binding of vFcγR gp34 to C-reactive protein (CRP)

C-reactive protein (CRP) is a molecule of the innate immune system but has structural and functional features in common with antibodies (ref. Bang et al. 2005; Duncan et al. 1988). It has been reported that CRP binds to human FcyRs potentially leading to receptor activation. Since viral FcyRs like gp34 and gp68 were shown to bind to IgG, the binding potential of various HCMV vFcyRs towards the ligand CRP was investigated.

gp34, but neither gp68 nor gp95 were able to bind to immobilized CRP. This identifies a further host factor as an interaction partner of gp34 and provides evidence that the glycoproteins gp34 and gp68 differ in their features and functions. While gp34mtrp is strongly impaired regarding IgG-Fc-binding (see Fig. 2) it preserves binding capability to the CRP-ligand although at lower levels compared to the sgp34wt protein.

This suggests that different domains of gp34 are involved in IgG-versus CRP-binding. CRP-binding to gp34 was confirmed by pulldown experiments using western blot detection and flow cytometry as read-out system.

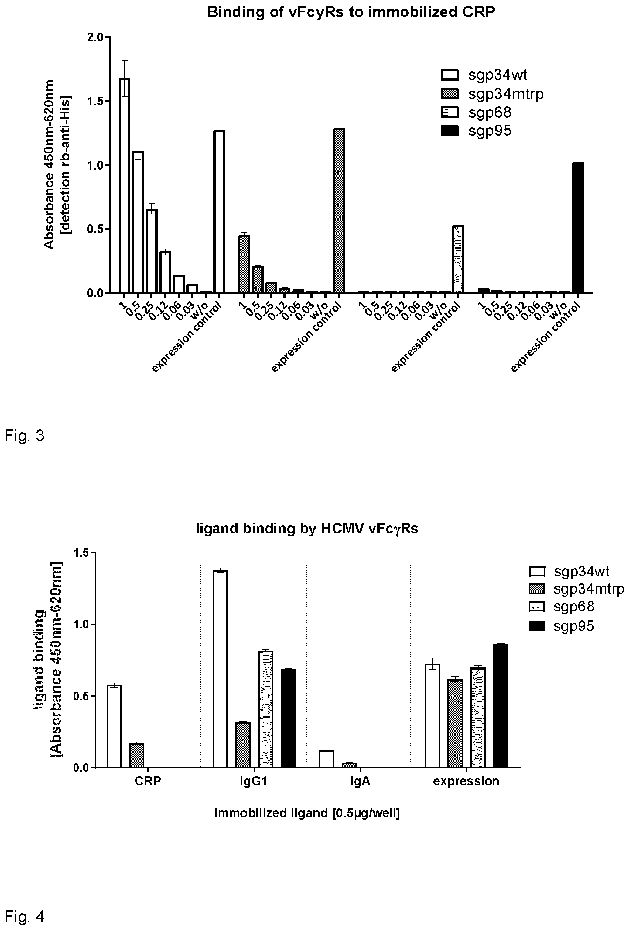

Fig. 3: Exclusive binding of C-reactive protein by vFcyR gp34. Various vFcyRs of HCMV were analyzed for their binding ability towards immobilized CRP in ELISA. While gp34 interacts with CRP, neither vFcyR sgp68, nor sgp95 bind to CRP. In addition, the IgG-Fc-deficient gp34 mutant sgp34mtrp shows residual binding to immobilized CRP.

Specificity of vFcyR binding to CRP was approved by comparing the binding to various immunoglobulins including human IgG1 (positive control) and human IgA (negative control). The data confirmed specific binding by sgp34wt as well as the mutant sgp34mtrp (Fig. 4).

Fig. 4: In addition to IgG, also CRP is a ligand of vFcyR gp34. vFcyRs of HCMV were analyzed for their binding ability to immobilized CRP, human IgG1 or human IgA in ELISA. While gp34wt (also sgp34mtrp to some extent) was found to interact with CRP, neither vFcyR gp68, nor gp95 were found to bind to CRP. In contrast, all tested vFcyRs including gp34, gp68 and gp95 are truly IgG-Fc-binding proteins as demonstrated. Binding of vFcyR to immunoglobulins is restricted to the IgG subclass whereas there is no binding observed to the IgA subclass.

Next, it was investigated whether gp34 is able to antagonize CRP-mediated host FcyR activation, as was observed before for IgG-mediated FcyR-activation. To this end, a reporter-cell-based assay was modified measuring IgG-mediated FcyR-activation to detect CRP-mediated FcyR triggering. The assay is based on mouse BW5147 thymoma cells lacking T cell receptor chains which can be stably-transduced to express extracellular domains (ECDs) of various human FcyRs fused to the signaling module of the T-cell receptor (CD3-zeta chain). Upon FcyR crosslinking the reporter cells secrete mouse IL2 (mIL2). Therefore, mIL-2 measured by ELISA indicates the level of host FcyR-activation and quantifies antagonizing effects of vFcyRs.

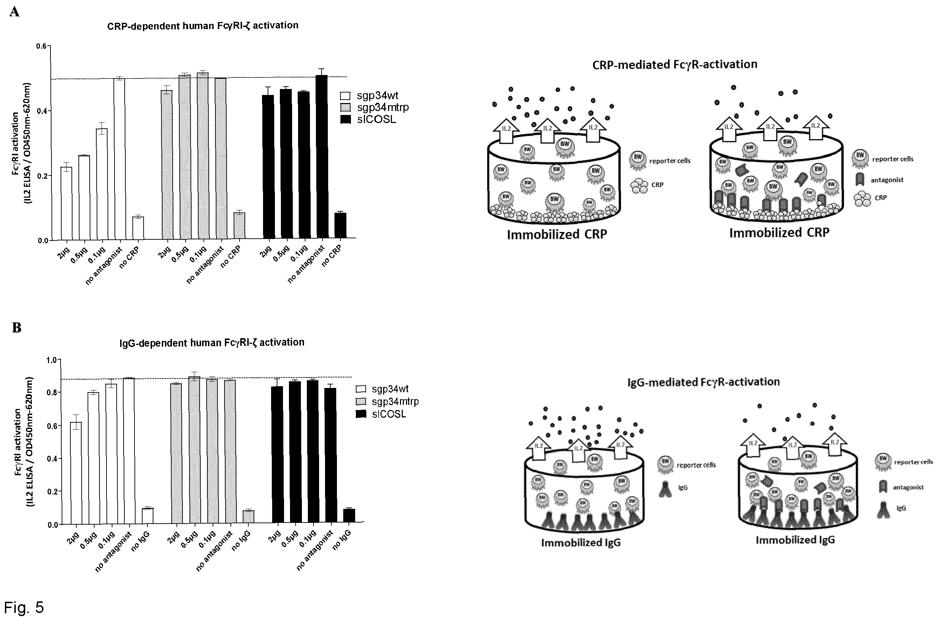

Fig. 5: Even stronger inhibitory effect by vFcyR gp34 on CRP-mediated host FcyRI activation, compared to IgG. Various recombinant, soluble proteins were used as potential antagonist to interfere with IgG- or CRP-dependent FcyR-activation using human FcγRI-expressing reporter cells. Our FcyR-activation assay was performed either on immobilized human IgG or on human CRP in presence or absence of vFcyR gp34 or control proteins. Gp34 showed an even stronger inhibitory effect on CRP-dependent FcyRI-activation compared to its antagonizing effect on IgG-dependent FcyRI-activation. sgp34mtrp or ICOSL ligand had no inhibitory effect on both ligands.

### Interaction of vFcγR gp34 with neonatal FcR (FcRn)

The neonatal Fc-receptor (FcRn) is belonging the Fc-receptor family although it does not represent one of the classical, canonical human FcyRs FcγRI,-IIa, -IIb or-III. This molecule is mainly found in placenta, facilitating the transport of maternal IgG to the fetus. In addition, FcRn plays a role in regulating IgG and serum albumin half-life and turnover. FcRn-mediated transcytosis of IgG across epithelial cells is possible because FcRn binds IgG at acidic pH(<6.5) but not at neutral or higher pH. Therefore, FcRn can bind IgG from the slightly acidic intestinal lumen and ensure efficient, unidirectional transport to the basolateral side where the pH is neutral to slightly basic.

First, it was investigated whether the viral FcyR gp34 is capable to interact IgG-dependent or independent with neonatal Fc-Receptor. Using immobilized recombinant FcRn protein binding of gp34 and control proteins were investigated for dose-dependent binding ability in ELISA. Our experiments identified FcRn, in addition to IgG, as a further ligand partner of vFcyR gp34, while control proteins like sgp34mtrp and ICOSL showed no binding.

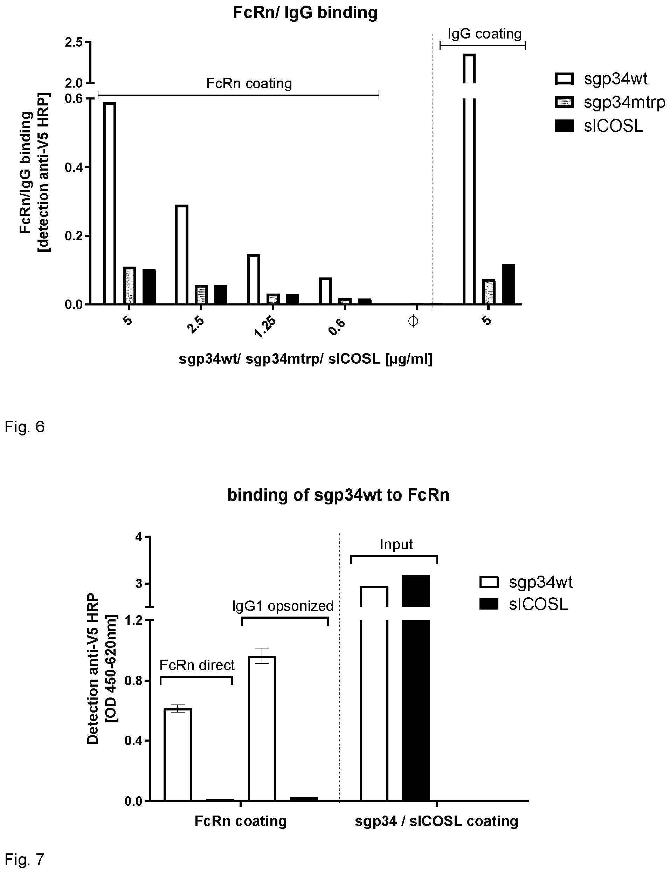

Fig. 6 shows that the neonatal Fc-receptor is bound by sgp34wt, but not sgp34mtrp. Binding ability of sgp34wt, sgp34mtrp and slCOSL as control protein where analyzed with regard to their ability to interact with FcRn. Recombinant FcRn was immobilized on ELISA plates and binding of sgp34wt, sgp34mtrp or slCOSL where detected via anti-V5-HRP antibody. Binding ELISA identified sgp34wt as a novel ligand of FcRn. In contrast, no binding of the mutant protein sgp34mtrp or slCOSL was observed, demonstrating specific interaction of sgp34wt with FcRn.

Next, it was investigated whether this binding is affected by the presence or absence of pre-bound IgG by FcRn. Indeed, ELISA binding studies showed enhanced binding of gp34 to IgG-opsonized FcRn. It is tempting to speculate if this is due to binding to the IgG-molecule itself or if pre-binding to IgG induces structural changes that further facilitate binding by gp34.

Fig. 7 demonstrates stronger binding of IgG-opsonized FcRn by gp34. Binding ability of sgp34wt and ICOSL as control protein where analyzed with regard to their ability to interact with FcRn opsonized with IgG or native FcRn. Recombinant FcRn was immobilized on ELISA plates and pre-incubated or not with human IgG. Binding of sgp34wt or ICOSL where detected via anti-V5-HRP antibody. Binding ELISA identified further enhanced FcRn binding by sgp34wt to FcRn decorated with human IgG. In contrast, no binding by ICOSL as control protein was observed, demonstrating specific interaction of sgp34wt with FcRn.

Next, interaction of sgp34wt and FcRn was analyzed in FACS experiments. Here, binding of gp34 to FcRn in absence or presence of antibodies was confirmed to be stable at neutral pH values as well as in low pH situations.

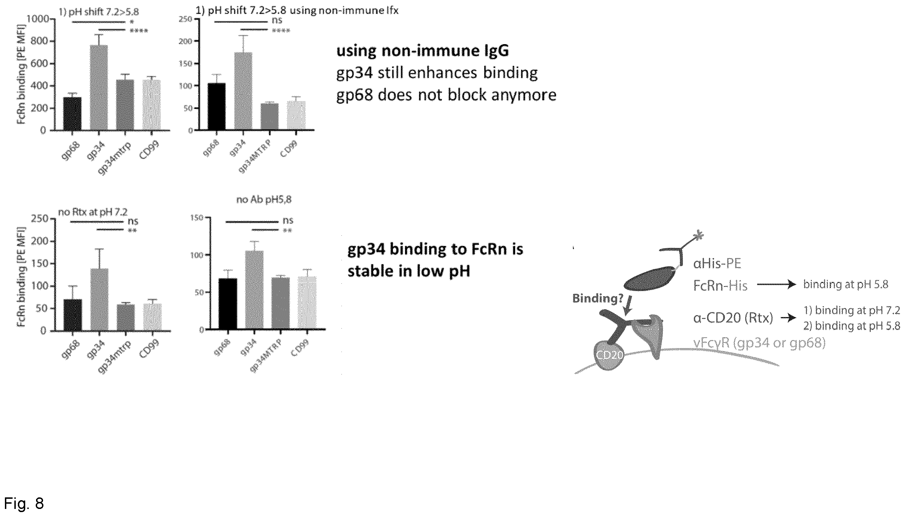

Fig. 8 shows that gp34 binding to FcRn is stable under low pH condition. Binding ability of sgp34wt, sgp34mtrp, sgp68 and slCOSL as control protein to FcRn where investigated by flow cytometry. The data revealed that vFcyR sgp34wt, but not gp34mtrp nor gp68 specifically interacts with FcRn. This interaction is observed on native, unbound FcRn, as well as in presence of antibodies. In addition, binding between sgp34wt and FcRn seems to be stable independent of the pH situation since FcRn still is bound by sgp34wt at low pH.

The observation that the HCMV-encoded Fc-binding protein gp34 shows now IgG-dependent but also direct binding towards FcRn led us to the speculation whether this interaction between gp34 and FcRn might have a role in transmission of HCMV from the mother to the fetus resulting in congenital infection.

Similar scenarios of viral transmission involving FcRn, with- or without IgG and viral particles have been shown recently. Studies demonstrated that FcRn-mediated transcytosis is involved with the trafficking of the HIV-1 virus across genital tract epithelium.

### Interaction of vFcγR gp34 to B-cell receptor (BCR)

The B-cell receptor (BCR) is a transmembrane protein on the surface of a B-cell. This receptor is composed of two molecules, CD79 and two immunoglobulin heavy and light chains. The membrane-bound immunoglobulin molecule of any given B-cell receptor is represented by one of the immunoglobulin isotypes: IgD, IgM, IgA, IgG, or IgE.

Experiments investigated binding of recombinant, soluble vFcyRs towards various immune cells to identify novel interaction partners/ligands. In brief, PBMCs were isolated from healthy donors, incubated with sgp34wt, sgp34mtrp, sgp68 or sICOSL. Next, immune cells were stained for characteristic surface markers and binding of soluble vFcyRs or slCOSL was determined using antibodies against the V5-His-tagged recombinant proteins.

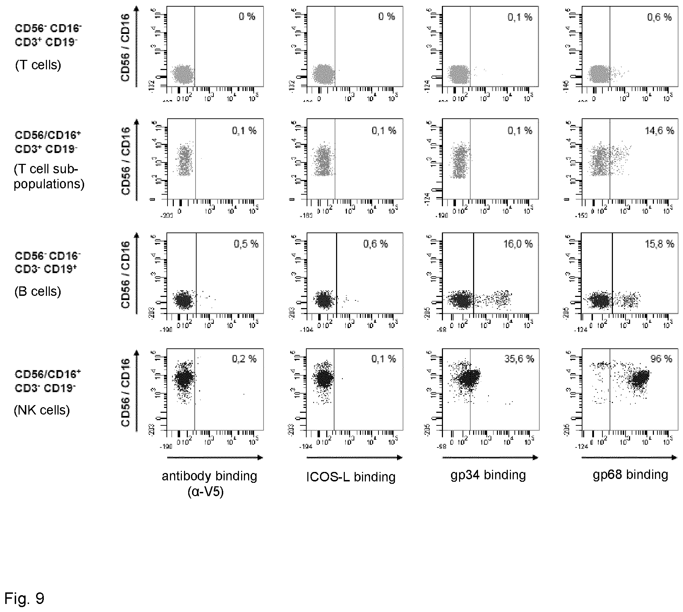

Fig. 9 shows the binding specificity of vFcyRs gp34 and gp68 to various subpopulations of immune cells. Recombinant, soluble proteins sgp34wt, sgp68 or slCOSL were incubated with various immune cell subpopulations. Binding analysis was done by flow cytometry using indicated surface markers for the identification of T-cells, B-cells or NK-cells. Binding of soluble proteins was determined by anti-V5-Tag as indicated. Both vFcyRs gp34 and gp68 show binding to a subpopulation of B-cells and NK-cells.

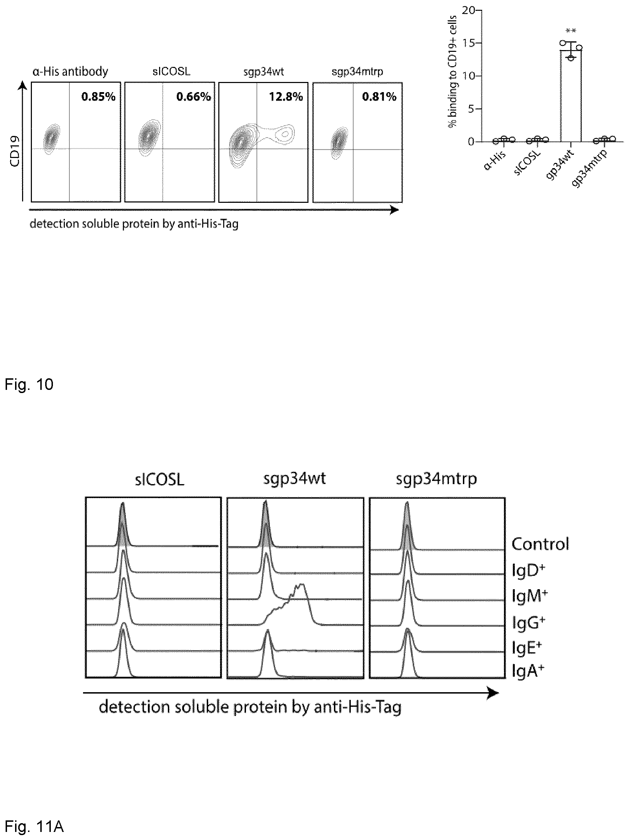

Fig. 10 describes binding specificity of vFcγRs gp34 to CD19+ B-cells. Recombinant, soluble proteins sgp34wt, sgp34mtrp or slCOSL were incubated with various immune cell subpopulations. Ex-vivo CD19+ human B cells were isolated from PBMCs via negative selection purification. Binding analysis was done by flow cytometry using indicated surface markers for the identification of T-cells, B-cells or NK-cells. Binding of soluble proteins was determined by anti-V5-Tag as indicated. Only vFcyRs gp34 shows binding to B-cells and NK-cells.

In a further experiment the immunoglobulin isotype of those CD19+ B-cells bound by gp34 was investigated. Therefore, in addition, isotype-specific antibodies were used for co-staining in flow cytometry.

Fig. 11A shows the exclusive binding of vFcyRs gp34 to IgG+/CD19+ B-cells. Recombinant, soluble proteins sgp34wt, sgp34mtrp or slCOSL were incubated with various immune cell subpopulations. Binding analysis was done by flow cytometry using indicated surface markers for the identification of T-cells, B-cells or NK-cells. B cell isotype populations were distinguished using conjugated anti-IgG, -IgA, -IgM, -IgD or-IgE antibodies in flow cytometry. A positive double-staining with conjugated anti-immunoglobulin isotype and anti-His antibody indicated binding between B-cell isotypes and sgp34wt, sgp34mtrp or sICOSL. Flow cytometry analysis confirmed selective binding of IgG+/CD19+ B-cells by sgp34wt, not sgp34mtrp or sICOSL.

Finally, we used another approach to confirm specific interaction with sgp34wt and CD19+ B-cells of IgG-isotype. Here, an ELISA-based assay was conducted.

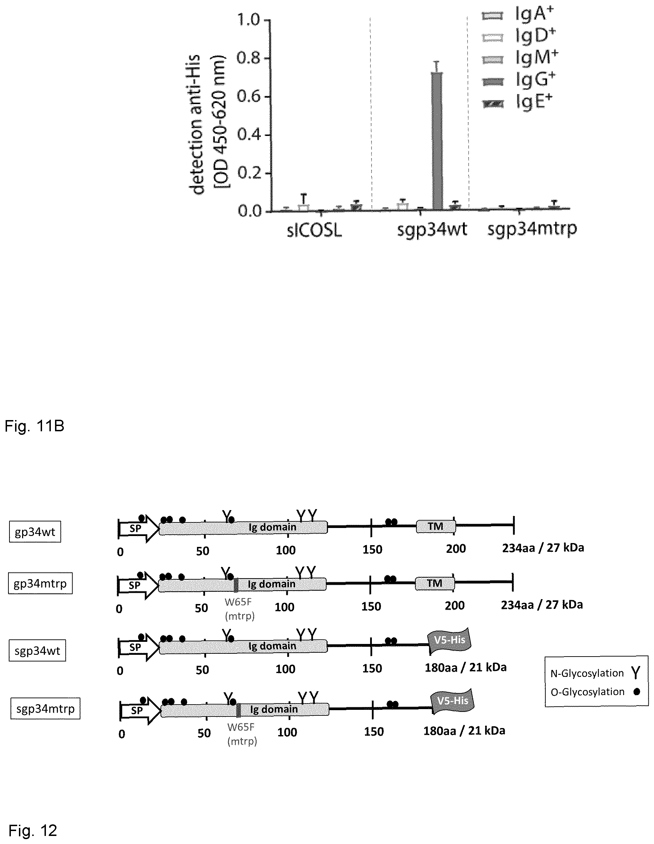

Fig. 11B illustrates the exclusive binding of vFcyRs gp34 to IgG+/CD19+ B-cells. Anti-IgG, -IgA, -IgM, -IgD or -IgE antibodies were coated onto an ELISA plate and incubated with ex-vivo isolated B-cells. sgp34wt, sgp34mtrp or slCOSL were co-incubated onto the ELISA plate. Recombinant proteins which bound indicated B-cell isotypes were detected with anti-His antibody. The optical density representing binding between proteins and B-cells was measured by spectrophotometry.

### Generation and characterization of HCMV gp34 (RL11) and gp68 (UL119-118) specific and antagonizing monoclonal antibodies (mAbs)

### Recombinant expression of soluble vFcyRs sgp34wt, sgp34mtrp and sgp68 for immunization

For generation of antigen specific and potential antagonizing monoclonal antibodies against vFcyR gp34 and gp68 of HCMV in total four different proteins where chosen to use as antigens for immunization. vFcγR gp34 and gp68 based on the sequence of HCMV AD169 strain, a non-functional mutant of gp34wt protein, gp34mtrp that will be further explained in detail later and ICOSL serving as control for antibody selection process containing the same protein-Tags (V5 and poly-His).

First, we have a closer look onto the recombinant protein of vFcyR gp34 and the point-mutation, gp34mtrp.

Fig. 12 shows schematically the domain structures of full length and soluble sgp34wt and gp34mtrp. Domain structure prediction based on protein sequences of HCMV AD169 vFcyR gp34 is depicted. Mutational analysis identified that a tryptophan on position 65 is important for oligomerization and impairs IgG-Fc-binding. This position (W65F, mtrp = mutated tryptophan) is highlighted in the gp34mtrp protein variants (highlighted in red letters). Full length versions of gp34wt and gp34mtrp are illustrated including the predicted signal peptide (SP), the Ig-like domain within the N-terminus, the transmembrane region (TM) followed by the cytoplasmic tail within the C-terminus. Glycosylation sites are predicted and illustrated: N-glycosylation (Y-shaped) and O-glycosylation (closed circle). In addition to full length versions, recombinant, soluble proteins are indicated, containing only the extracellular domain (ECD) and tagged with a V5-poly-histidine Tag. Soluble proteins are designated by the small letter "s" in front (sgp34wt and sgp34mtrp).

Next, we have a closer look onto the recombinant protein of vFcyR gp68.

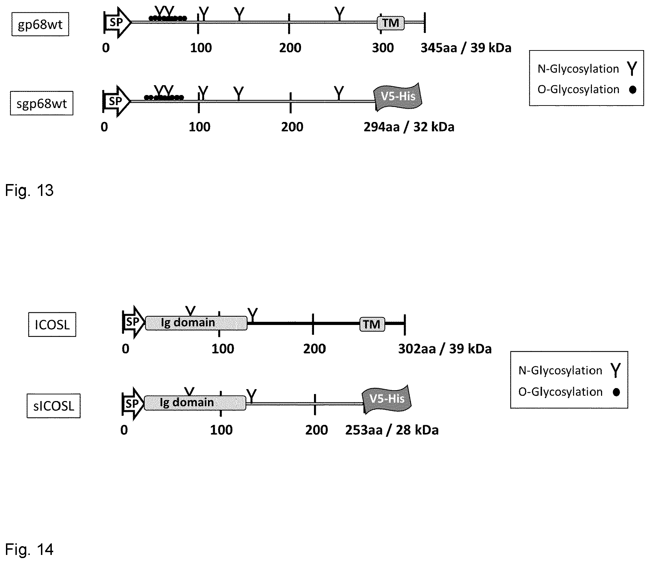

Fig. 13 shows domain structure of full length and soluble gp68. Domain structure prediction based on protein sequences of HCMV AD169 vFcyR gp68 is depicted. Full length version as well as a soluble variant of gp68 is illustrated including the predicted signal peptide (SP), the transmembrane region (TM) followed by the cytoplasmic tail within the C-terminus. Glycosylation sites are predicted and illustrated: N-glycosylation (Y-shaped) and O-glycosylation (closed circle). In contrast to the full-length gp68, the recombinant, soluble gp68 protein (sgp68) contains only the extracellular domain (ECD) and is V5-poly-His-tagged.

Finally, Fig. 14 illustrates the recombinant protein of human ICOSL, which was selected to serve as a negative control throughout experiments. Human ICOSL is also a type-I glycoprotein but without any known interactions to IgG.

The domain structure of full length and soluble human ICOSL is shown. Human ICOSL is a type-I glycoprotein, but without Fc-binding capacities. Domain structure prediction based on the protein sequence of human ICOSL (ICOS ligand). Full length version (ICOSL) as well as a soluble variant of ICOSL (sICOSL) is illustrated including the predicted signal peptide (SP), a predicted Ig-like domain, the transmembrane region (TM) followed by the cytoplasmic tail within the C-terminus. Glycosylation sites are indicated as following: N-glycosylation (Y-shaped) and O-glycosylation (closed circle). In contrast to the full-length ICOSL, the recombinant, soluble ICOSL protein (sICOSL) contains only the extracellular domain (ECD) and is tagged by V5-poly-histidine.

Only extracellular domains (ECDs) of the viral Fc-binding proteins gp34 and gp68 from HCMV AD169 strain were recombinantly expressed to serve as antigens for immunization of mice.

For expression, detection and purification purposes, soluble proteins were V5-His tagged on the C-terminal part of the ectodomains. N-terminus of gp34 was amplified by PCR using the following primers 5'-CGCGCTCGAGATGCAGACCTACAGCACCCC-3' (SEQ ID NO:4) and 5-CGCGGGATCCTCAATGGTGATGGTGATGATGACCGGTACGCGTAGAATCGAGACC GAGGAGAGGGTTAGGGATAGGCTTACCGGACCACTGGCGTTT-3' (SEQ ID NO:5). Thereby, restriction sites were introduced, as well as the V5-His Tag using revere primer to add in-frame Tag onto the C-terminus before the Stop-codon. Cloning of gp34mtrp version was performed using same primers as for the construction of the sgp34wt plasmids, but using an already existing plasmid as PCR-template containing the amino acid change for the gp34mtrp version (W65F). Correct mutation was confirmed by DNA sequencing.

In the cloning of the N-terminus of gp68 suitable primers were used. The N-terminus of human ICOS ligand (ICOSL) was amplified by PCR using the following primers 5'-GCGCCTCGAGATGCGGCTGGGCAGTCCTGGACTG-3' (SEQ ID NO:6) and 5'-GCGCGGATCCTCAATGGTGATGGTGATGATGACCGGTACGCGTAGAATCGAGACCG AGGAGAGGGTTAGGGATAGGCTTACCCGTGGCCGCGTT-3' (SEQ ID NO:7). Again, C-terminal V5-poly-His Tag was added by revers primer. Sequencing of the coding sequences showed an amino acid exchange in ICOSL from V128 to 1128 but with no detectable functional difference. Also, this V128 to 1128 mutation is an described single-nucleotide polymorphism (SNP) of human ICOSL (NP_056074.1.1:p.Val128Ile).

All oligos used for amplification are listed in the table below.

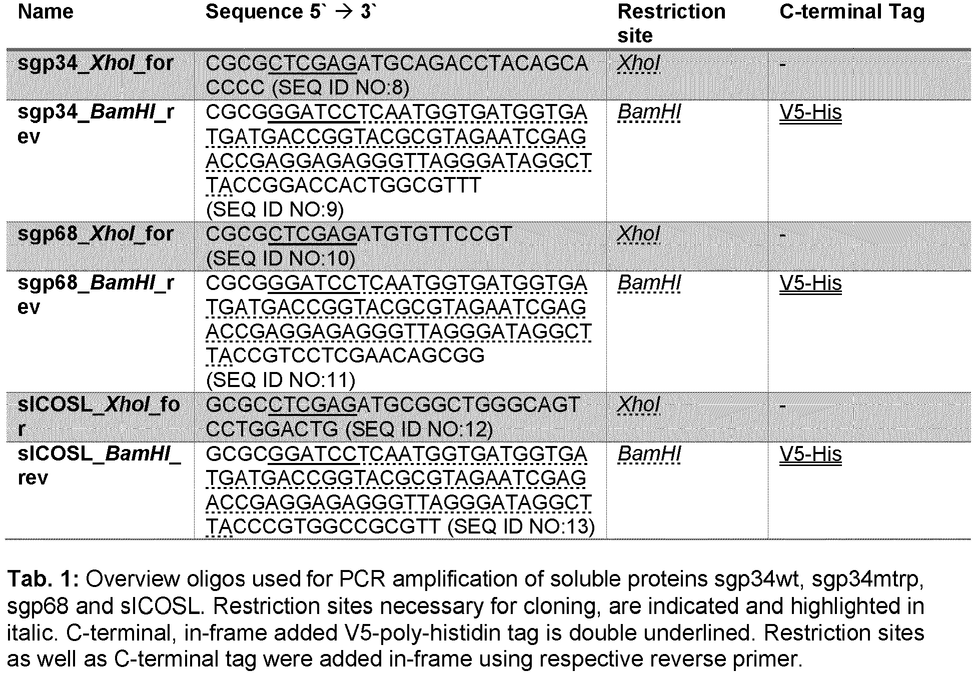

Briefly, each PCR product was amplified and cloning restriction sites were inserted by the forward and the revers primers. Both protein Tags (V5 and poly-histidine) were inserted in-frame using the revers primer. PCR-products were purified from agarose-gel using Monarch gel purification kit. Inserts and target vectors were digested for overnight and gel-purified again. Cloning was done, using the restriction sites Xhol and BamHI (italics in primer sequences) and then cloned into pIRES-EGFP vector (Clontech, USA). For enhanced protein expression, β-Globin cloned from pSG5 vector (Stratagene, USA) was inserted into pIRES-EGFP between the CMV-IE promoter and the coding sequences, named pIRE-β-EGFP. Inserts coding for tagged sgp34wt, sgp34mtrp, sgp68 and slCOSL were further sub-cloned into pUCIP plasmid using same restrictions sites as before to use them for lentiviral transduction of target cells like HEK-293-T.

Recombinant protein expression was conducted by two different expression strategies, both containing their own advantages and disadvantages. Proteins were expressed either (i) by transiently transfection of HEK-293T cells or (ii) by stable HEK-293T cells after lentiviral transduction using pUCIP plasmids and puromycin selection of stable clones after sub-cloning by limiting dilution technology. The plasmids were transfected in HEK-293-T cells using polyethylemine (PEI, branched, Sigma-Aldrich, Germany) and transfected cells were verified by expression of EGFP (expressed also on the pIRES-β-EGFP plasmid) by fluorescent microscopy (Sigma-Aldrich, Germany).

For protein expression in stable cells three human cell lines where tested for best quality and quantity. Therefore, HeLa cells, HEK-293-T and A549 cells where stably transduced by lentiviral transduction as described elsewhere and protein expression was compared and analyzed. On basis of those results, HEK-293-T cells where selected for further recombinant protein expression of soluble proteins to use for immunization.

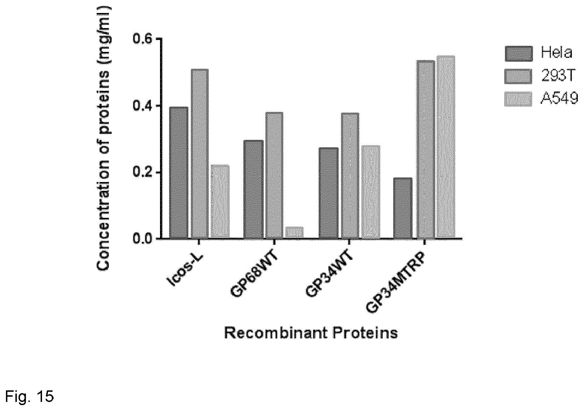

Fig. 15 shows a representative comparison of protein expression in various stable cell lines. Various cells were lentivirally transduced for stable expression of soluble proteins (sgp34wt, sgp34mtrp, sgp68 and sICOSL). HeLa (human epithelial cells, cervix, blue bars), HEK-293-T (human epithelial cells, embryonic kidney, green bars) and A549 (human epithelial cells, lung epithelium, orange bars), cells were chosen as target cells and cells were controlled for successful protein expression after puromycin selection. Expression levels of secreted proteins within the cell culture supernatant were analyzed by rabbit-anti 6xHis antibody in ELISA. Stable 293-T cells were used for further purposes, showing most convincing protein expression for all proteins.

Since earlier experiments demonstrated problems during purification process and affected recombinant protein quality, we decided to produce our recombinant without fetal calf serum (FCS) to avoid contamination with proteins like bovine serum albumin (BSA).

When cells were 90-100% confluent and well-attached media was carefully replaced by starvation medium without FCS (DMEM w/o phenol red, 1% Pen/Strep, 1% Sodium Pyruvat). After 5-7 days or when cells started to detach, supernatants were collected, remaining cells in the supernatant removed by centrifugation (40 min, 4.000g), sterile filtered and adjusted to a 10 mM Imidazole concentration and passed over a His-Trap FF crude column (GE Healthcare, USA). Proteins were eluted in Imidazole/Phosphate buffer (250 mM Imidazole, 20 mM sodium phosphate, 500 mM NaCl) and immediately dialyzed against PBS and concentrated via Amicon columns. Protein concentrations were determined by PIERCE BCA assay and protein quality was analyzed in various assays like IgG-Fc binding ELISA, coomassie stain and western blot using anti-V5 or anti-His-Tag antibodies for detection. For second expression strategy, using stable cell clones for expression of recombinant proteins, cells were kept under selection pressure with 2 mg/ml puromycin (selection marker encoded on the pUCIP plasmids). Cells were grown and expanded to a density of -100% per dish and put under Starvation as described before. Supernatants were collected usually between 7-10 days when cells started to detach from the surface. Further steps including collection, purification and dialysis was performed as mentioned before.

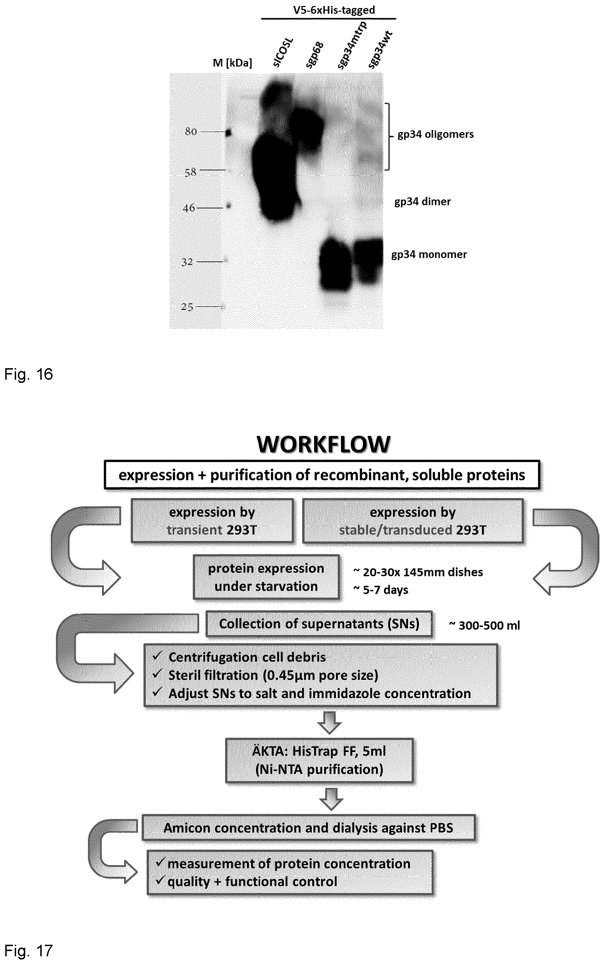

Fig. 16 shows the expression of soluble, V5-poly-His tagged glycoproteins. Purified, soluble glycoproteins sgp34wt, sgp34mtrp, sgp68 and slCOSL were characterized in western blot. Proteins expressed from stable 293-T cells were purified dependent on their V5-6xHis-Tag by NiNTA purification using ÄKTA system, dialyzed and concentrated against PBS. 5 µg total protein was loaded on SDS-PAGE and proteins detected in rabbit-anti-His western blot.

Protein expression and purification was further optimized to obtain proteins best in quality and quantity. Next, a scheme for a typical strategy for recombinant protein expression, supernatant collection and protein purification is illustrated.

Fig. 17 is a workflow of recombinant protein expression and purification. Essential Steps of recombinant protein expression and purification is illustrated. Quality and quantity controlled proteins were used for immunization process.

### Antigens used for immunization

Protein batches were generated, quality controlled and protein concentrations measured using PIERCE BCA kit. Selected samples were delivered on dry ice to our collaboration partners to performing immunization, fusion and sub-cloning of promising candidate clones. Protein batches used for immunization are illustrated following.

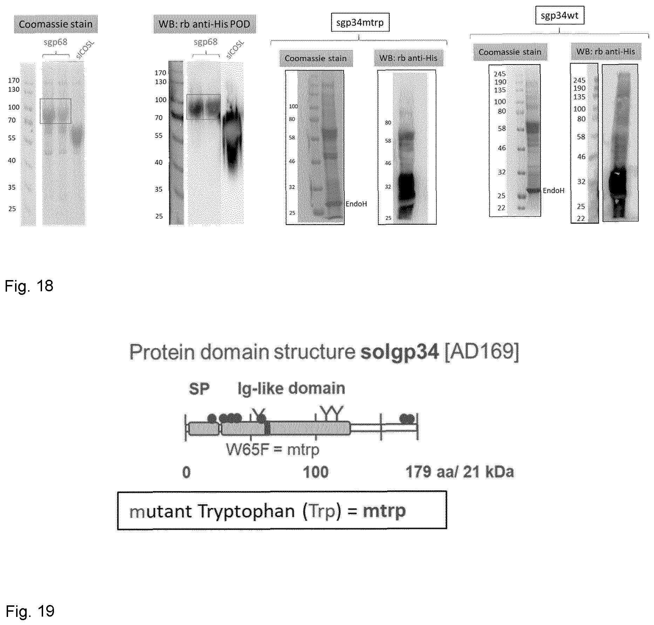

Fig. 18 shows the recombinant proteins used as antigens for immunization. Purified, soluble ectodomains of sgp34wt, sgp34mtrp, sgp68 and slCOSL were characterized in coomassie stain and western blot. 10 µg of purified proteins expressed from stable 293-T cells were loaded on SDS-page for coomassie stain. In parallel 10 µg of proteins were loaded for western blot analysis using rabbit-anti-His and goat-anti-rabbit IgG HRP for detection.

### Antibody selection process for gp34wt, gp34mtrp and gp68

The immunogen (sgp68, sgp34wt or sgp34mtrp) expression technology is based on recombinant protein production in HEK-293-T cells. In contrast to wildtype gp34, the mtrp version of gp34wt protein consists of a single amino acid change within the extracellular domain. The original tryptophan (Trp) at position 65 was mutated to phenylalanine (Phe) which leads to conformational consequences in the protein.

Fig. 19 emphasizes the tryptophan in gp34wt sequence which is essential for its function. sgp34wt and sgp34mtrp proteins were used for immunization of mice to enhance the chance of successful antibody generation. The tryptophan (trp) position within the extracellular domain (W65), important for IgG-Fc-binding is indicated.

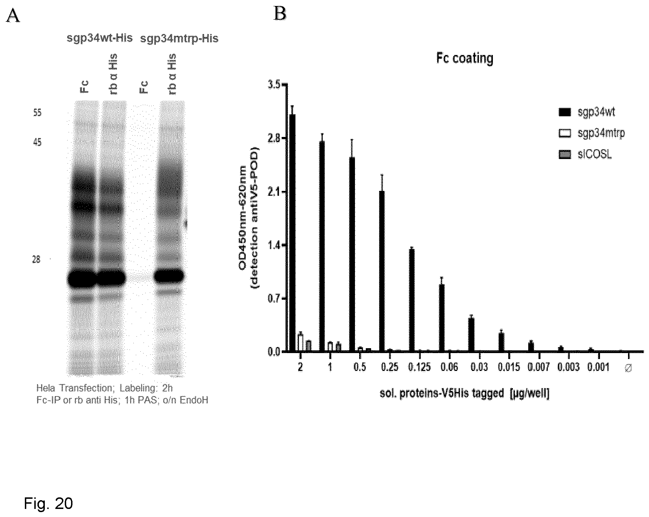

Usually gp34wt protein forms monomers, dimers and high molecular weight oligomers like tetramers. However, oligomerization seems to be essential for IgG interaction. In consequence, gp34mtrp forms only monomers and dimers and lacks IgG-Fc binding capacity as shown in Fig. 20.

Next, gp34wt and gp34mtrp variants were further characterized, regarding their ability to bind human IgG-Fc-fragment. The data demonstrate that gp34 is indeed a vFcyR able to bind to human IgG shown in precipitation experiments and binding ELISA. Both experiments show as well that gp34 with a mutated tryptophan (gp34mtrp) is impaired for IgG-Fc-binding.

Fig. 20: mutated tryptophan in gp34wt sequence is essential for IgG binding. A mutation at position 65 on amino acids level results in a Fc-binding impaired gp34 variant- gp34mtrp. Lack of IgG-binding by this point mutation is shown using precipitation of radioactive-labeled proteins (A) or via binding ELISA on immobilized human IgG-Fc-fragment (B). Both molecules in their soluble, V5-His-tagged form were expressed in transiently transfected HeLa cells. 1 day post transfection, cells were metabolically labeled using ³⁵S for 2 hours (see Fig. 20A). Cell lysates were incubated with indicated antibodies either precipitating the soluble proteins, sgp34wt or sgp34mtrp, via C-terminal His-Tag or using human IgG-Fc-fragment to precipitate protein capable for Fc-binding. Precipitation of EndoH-deglycosylated samples was analyzed using SDS-gradient gel and Phospholmage software for development. Results confirmed comparable expression of both molecules, but drastically impaired Fc-binding for sgp34mtrp. Impaired IgG-Fc-binding was confirmed in binding ELISA using immobilized human Fc-fragment (see Fig. 20B). Brief, human Fc-fragment (Rockland; at 4 µg/ml) was immobilized on ELISA plates. Soluble proteins were added to blocked wells and binding detected using anti-His antibody recognizing C-terminal tagged sgp34wt, sgp34mtrp or sICOSL, used as a non-binding control.

Since gp34wt protein shows in addition to human IgG, also binding potential to various mouse IgG subclasses, we decided to use also gp34mtrp for immunization to guarantee a successful generation of anti-gp34 mAbs. Cells were transfected for expression of sgp34wt and sgp34mtrp proteins and analyzed for their binding to various IgG molecules including human IgG and various mouse subclasses. As expected, sgp34mtrp is not only impaired for binding to human, but also to mouse IgG molecules and should therefore not interfere with the immunization process.

It was questionable in the beginning of this process whether it was possible to generate mAbs using sgp34wt protein as antigen that would truly recognize the vFcyR gp34 or if such antibodies would only be "false-positive" due to binding by gp34wt to these antibodies via their Fc-part. Therefore, it was speculated to gain at least antibodies using the gp34mtrp variant, structurally forming monomers and dimers, also recognizing the native protein.

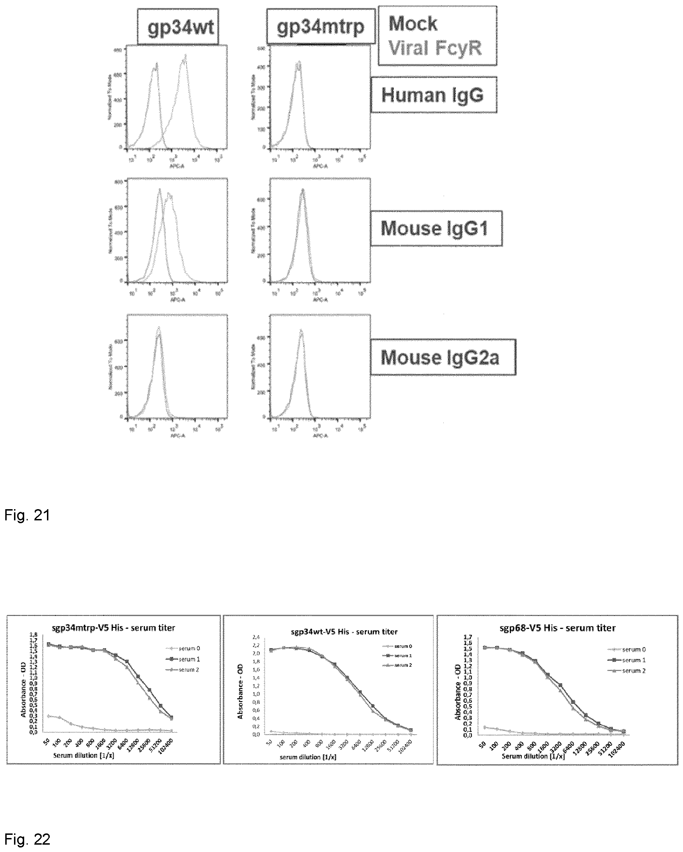

Fig. 21 illustrates the binding of vFcyR gp34 to mouse IgG subclasses. While sgp34wt protein shows binding to the Fc-part of human IgG and at least mouse IgG1, gp34mtrp protein is impaired for IgG binding in general, independent on the origin of IgG-species used.

### Immunization of female BALB/c mice

The sequences of gp34wt and gp68wt correspond to the laboratory HCMV strain AD169. Nethertheless, both proteins are highly conserved between HCMV strains, showing only few amino acid variations.

For immunization, BALB/c mice were injected with 50 µg of the immunogen (either sgp68-V5-His, sgp34wt-V5-His or sgp34mtrp-V5-His) in complete Freund's adjuvant (CFA) followed by 50 µg of the same immunogen in incomplete Freund's adjuvant (IFA) at day 14 post first immunization. Next, ELISA analyzes of the sera for anti-gp68, anti-sgp34wt or anti-gp34mtrp antibody titers were performed. The mice with the highest titer were boosted with another 50 µg of the respective immunogen in PBS.

Fig. 22: immunization titers of BALB/c mice immunized with sgp34mtrp, sgp34wt or sgp68. Serum titers for sgp34mtrp, sgp34wt and sgp68 immunization are shown. Sera tested at different intervals for antigen-specificity in ELISA. Dependent on serum-titers compared to non-immunized individuals, mice were killed and splenocytes used for fusion.

After three days, the spleen of immunized mice was taken and, after lysis of red blood cells, the splenocytes were fused with SP2/0 cell line. The potential hybridoma cells were seeded in 20 % RPMI 1640 medium containing hypoxanthine, aminopterin, and thymidine (HAT) for selection of stable hybridoma cell lines.

To obtain anti-gp68 antibodies, 564 wells with hybridoma cells were screened for antibody secretion by ELISA. Since all proteins gp68, sgp34wt, sgp34mtrp and slCOSL were C-terminal V5 and poly-His tagged one has to test and exclude those clones only raised against the protein-Tag itself.

Then, 59 hybridoma cells from wells in which the supernatants were positive on the gp68-V5His coated ELISA plates, were re-tested for their positivity. In parallel, a cross-reactivity test was performed on an irrelevant His-tagged protein. This resulted in 17 hybridoma cells that secreted antibodies specifically recognizing gp68 protein. Moreover, none of these antibodies showed cross-reactivity to the sgp34mtrp protein (data not shown) or the irrelevant control protein (sICOSL-V5His).

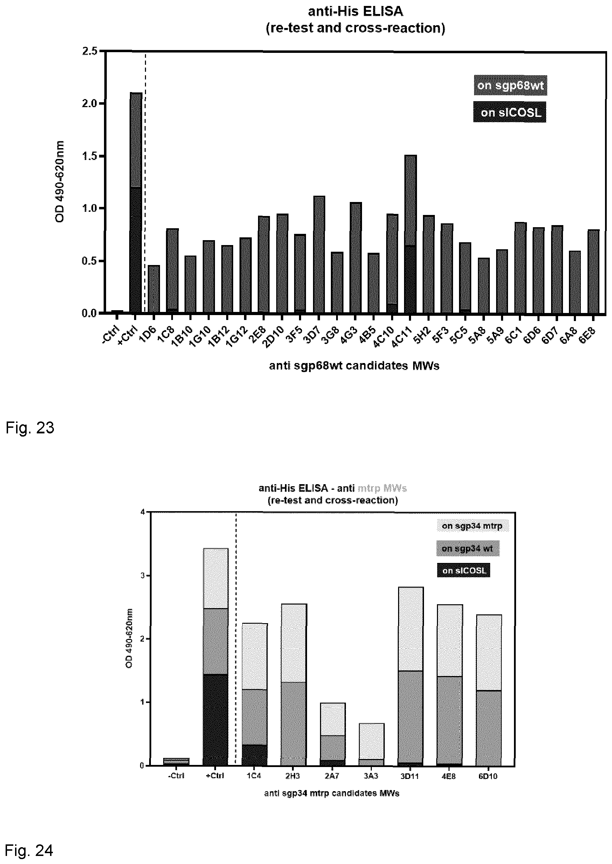

Fig. 23: First screening candidates anti-vFcyR gp68 mAbs. Supernatants positive after first round of screening were re-tested for antigenic specificity and tested on control protein to exclude antibodies raised against V5-His-Tag only. Supernatants of cell cultures of various hybridomas were analyzed on immobilized sgp68 (V5-His tagged) or control protein (sICOSL, also V5-His-tagged). ELISA values for detection of sgp68 (red part of the bars) or slCOSL (blue part of the bars) are shown additive. Clone names are indicated as origin of mother well (MW) of mAb candidates.

Out of 17 hybridoma cells, 4 were further selected due to their capacity to block the binding of the human Fc-fragment to the gp68 protein and sub-cloned. At this point, 8 stable clonal cell lines, summarized in Tab. 2, were selected for large scale production and purification (gp68.01, gp68.02, gp68.03, gp68.04, gp68.05, gp68.06, gp68.07, gp68.08). Thereby, always to pairs originated from one mother-well (MW) and may most likely result in identical sub-clones.

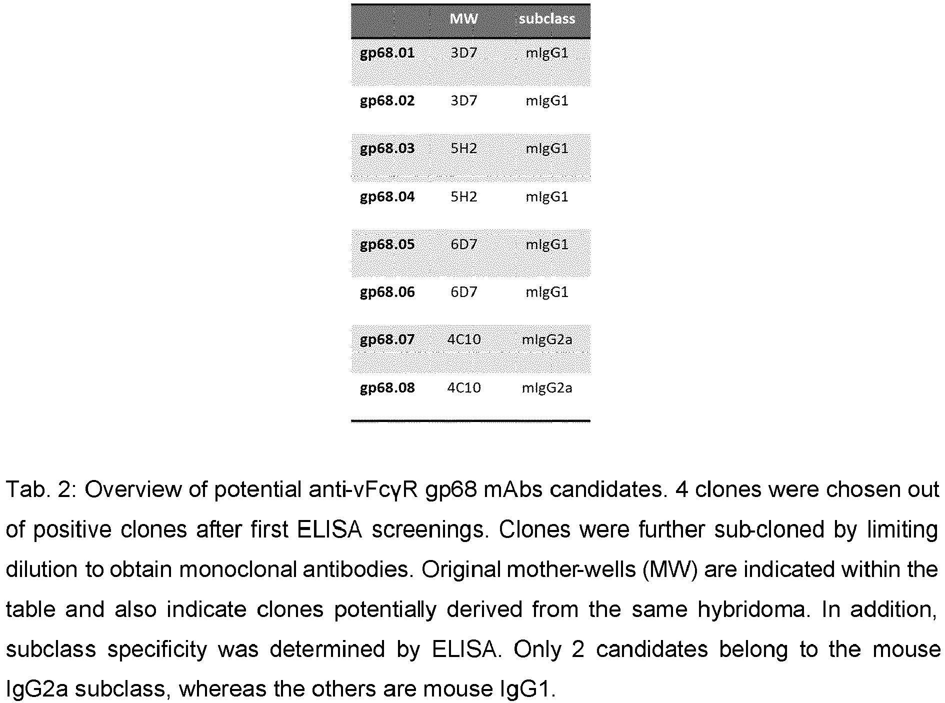

Next, a large-scale antibody production was performed and all monoclonal antibodies were purified from the serum free medium, using GE ÄKTA Prime Plus Liquid Chromatography System and HiTrap Protein G columns, in an amount of few milligrams. Finally, all 8 leading candidates showed a strong binding capacity to the purified protein but moreover to the native HCMV gp68 protein in infected cells.

Next, to obtain anti-gp34wt antibodies, 564 wells with hybridoma cells were screened for antibody secretion by ELISA. Then, 34 hybridoma cells from wells in which the supernatants were positive on the sgp34wt coated ELISA plates, were re-tested for their positivity. In parallel, a cross-reactivity test was performed on an irrelevant V5-His-tagged protein (e.g. sICOLS-V5-His). This resulted in 14 hybridoma cells that secreted antibodies specifically recognizing sgp34wt protein. Moreover, none of these antibodies showed cross-reactivity to the sgp68 protein (data not shown). Interestingly, no antibody obtained using sgp34wt protein for immunization recognized the sgp34mtrp protein having only one amino acid altered. This tempted us to speculate that gp34wt mAbs recognize structural epitopes on gp34 since its mtrp version is impeded for oligomerization.

Out of 14 hybridoma cell lines, 5 were further selected for their highest absorbance and sub-cloned. At this point, 9 stable clonal cell lines, were selected for large scale production and purification (gp34wt.07, gp34wt.08, gp34wt.09, gp34wt.10, gp34wt.11, gp34wt.12, gp34wt.13, gp34wt.14, gp34wt.15). Thereby, always two pairs originated from one mother-well (MW) and may result in identical sub-clones. mAb gp34wt.15 is unpaired.

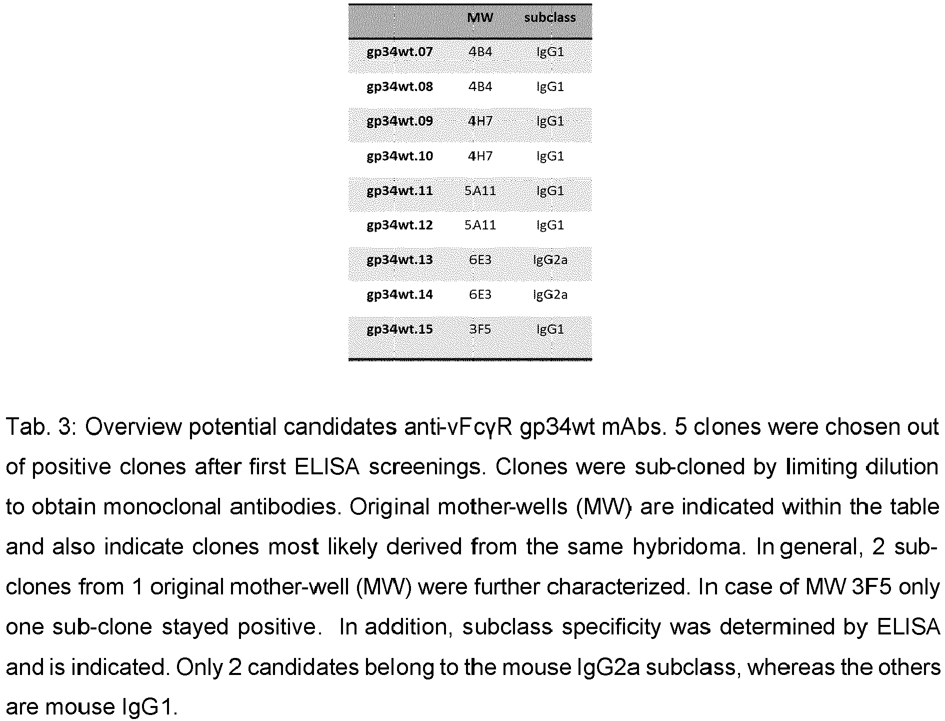

Next, a large-scale antibody production was performed and all monoclonal antibodies were purified from the serum free medium, using GE ÄKTA Prime Plus Liquid Chromatography System and HiTrap Protein G columns, in an amount of few milligrams. Finally, all 9 leading candidates showed a strong binding capacity to the native HCMV gp34 protein in infected cells.

Finally, to obtain anti-gp34mtrp antibodies, 1128 wells with hybridoma cell were screened for antibody secretion by ELISA. Then, 12 hybridoma cells from wells in which the supernatants were positive on the sgp34mtrp coated ELISA plates, were re-tested for their positivity. In parallel, a cross-reactivity test was performed on an irrelevant His-tagged protein (sICOSL-V5His). This resulted in 5 hybridoma cell lines that secreted antibodies specifically recognizing sgp34mtrp protein. Moreover, all 5 antibodies showed cross-reactivity to the sgp34wt protein. Out of 5 hybridoma cell lines, 3 were further selected for their highest absorbance and sub-cloned. At this point, 6 stable clonal cell lines, were selected for large scale production and purification (gp34mtrp.01, gp34mtrp.02, gp34mtrp.03, gp34mtrp.04, gp34mtrp.05, gp34mtrp.06). Next, a large-scale antibody production was set up and all monoclonal antibodies were purified from the serum free medium, using GE ÄKTA Prime Plus Liquid Chromatography System and HiTrap Protein G columns, in an amount of few milligrams. Finally, all 6 leading candidates showed a strong binding capacity to the native HCMV gp34 protein in infected cells.

Fig. 24: First screening anti-vFcyR gp34 mAbs candidates, derived from immunization using sgp34mtrp as immunogen. Supernatants positive after first round of screening were re-tested for antigenic specificity and tested on control protein to exclude antibodies raised against V5-His-Tag only and tested for recognition of sgp34mtrp as well as sgp34wt protein. Supernatants of cell cultures of various hybridomas were analyzed on immobilized sgp34wt (V5-His tagged), sgp34mtrp (V5-His tagged) or control protein (sICOSL, also V5-His-tagged). ELISA values for detection of sgp34wt (green bars), sgp34mtrp (yellow bars) or slCOSL (blue bars) are shown additive. Clone names are indicated as origin of mother-well (MW) of mAb candidates.

Although not many positive candidates derived from the immunization were obtained using sgp34mtrp protein as antigen, nearly all of them recognize gp34mtrp as good as they recognize native gp34 protein.

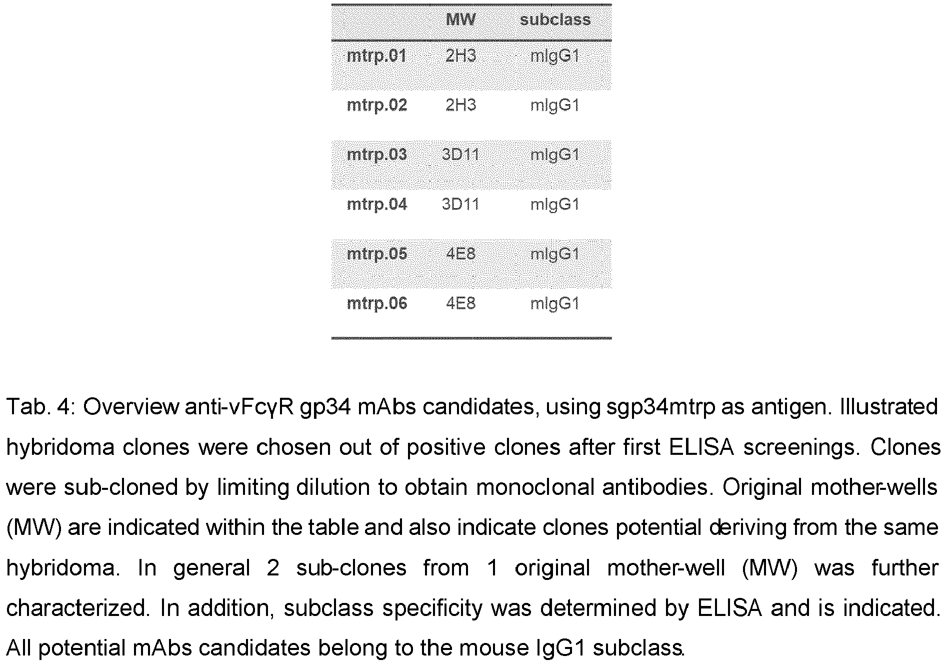

In summary, we generated various monoclonal antibodies recognizing vFcγRs gp34 or gp68 that were further characterized and functional analyzed. All candidates, including the mouse IgG subclass belonging to, are summarized in following table (Tab. 5).

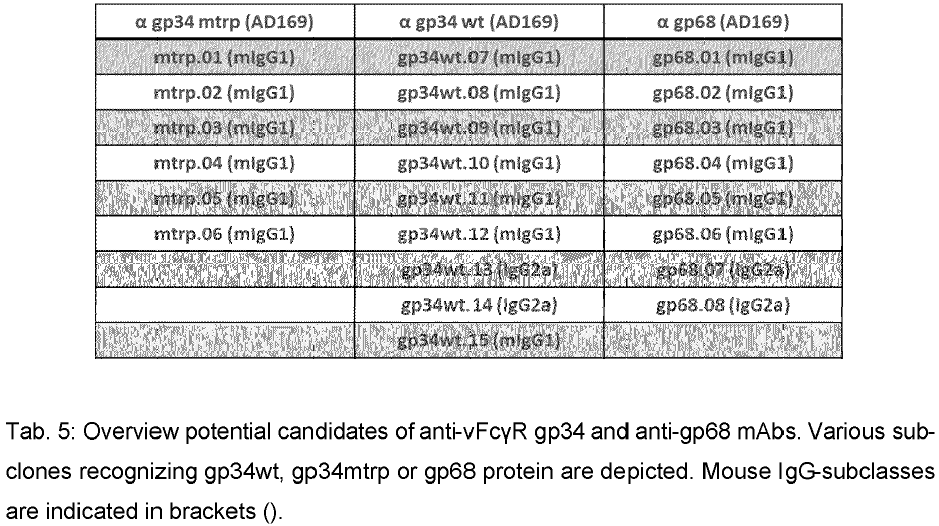

### Maintenance and expansion of hybridomas

Hybridomas were tested in ELISA for specific antibody production. Positive clones were sub-cloned and further tested. When clones remained positive in ELISA, they were cultured and transferred from 96-well plates to larger vessels and later to flasks/dishes. Cells were expanded in RPMI, 10% FCS media to desired cell density. For antibody production and collection, the FCS percentage in the culture medium was reduced from 10% to 0% (Starvation media contains no FCS at all). For starvation, cells were re-suspended after centrifugation into ISF-1 medium (F 9061-01 from Biochrom) without any additional supplements. Cells were routinely checked for viability under microscopy. After 2-3 weeks, the conditioned medium was collected by centrifugation (4.000g, 40 mins, 4°C) and sterile-filtered (0.45µm in size] into flasks until the antibody purification on protein G sepharose (use HiTrap^{®} Protein G HP GE Healthcare 1 ml columns) was down.

### Freezing hybridomas

Hybridoma cells and corresponding sub-clones were frozen for long-time storage in liquid nitrogen. For this, first the cell number was determined by counting them with trypan-blue staining. Then, 0.5 - 1x 107 cells/ml were re-suspended in fresh medium (RPMI medium with 10% FCS) and diluted 1:2 in 2x freezing medium. Sample preparation was performed on ice. Samples were stored on liquid nitrogen.

### Purification of monoclonal antibodies by protein-G sepharose

In general, antibodies were purified using 1 ml protein-G sepharose, packed into a column and attached to the GE Healthcare ÄKTA System device. Before loading the column with the antibody solution, the resin was pre-equilibrated. A four-fold volume of binding buffer was passed through the column with a flow rate of 0.5 ml/min. Then the antibody solution was loaded onto the protein G sepharose column at the same flow rate. Non-specifically bound proteins were washed off with binding buffer until a baseline UV absorbance was reached monitored at 280 nm. Elution was performed at 0.5 ml/min with elution buffer (0.1 M glycine pH 3.1) and samples were collected in 1.5 ml Eppendorf tubes, in 200 µl fractions. Immediately after elution, samples were neutralized with 1 M Tris pH 11, already pipetted into wells for elution. After purification, eluted samples were dialyzed and concentrated against PBS using Amicon columns.

Afterwards, purified antibodies were analyzed on SDS-PAGE (coomassie stain) and in WB. Protein concentrations of dialyzed samples were determined using a PIERCE BCA kit. Aliquots of antibodies were stored for long term at -80°C. To re-use the protein-G sepharose column, the resin was regenerated by washing first with 6 M Urea, washing and flushing with 20% ethanol and afterwards stored at +4°C. Each HiTrap^{®} Protein G HP GE Healthcare 1 ml column was used monoclonal antibody-specific, always the same column for the same hybridoma.

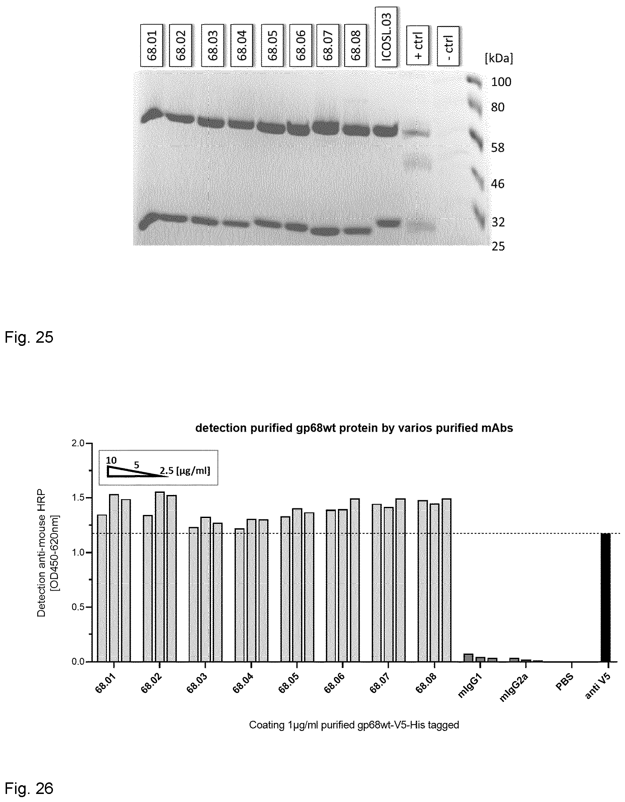

Fig. 25 shows purified anti-vFcyR gp68 mAbs. Exemplarily, all generated anti-gp68 mAbs after NiNTA purification from hybridoma cell culture supernatants under starvation conditions (no FCS) were analyzed in coomassie stain. 5 µg of each mAb was loaded on SDS-page and stained. In addition, one representative mAb raised against recombinant, soluble human ICOSL (ICOSL.03) was investigated. Commercial available mouse IgG1 isotype antibody (Invitrogen) served as positive control and cell lysate from non-antibody producing cells (SP2/0 hybridoma cells) were loaded as negative control.

### Characterization mAbs

### Detection of vFcyRs gp68 in ELISA using mAbs

To further characterize newly generated mAbs raised against vFcyRs gp34 and gp68, purified mAbs were tested for binding and recognition of the appropriate antigen, e.g. gp34 or gp68. First, mAbs were tested on immobilized recombinant, soluble gp68-V5-His protein that was used for immunization. The antigen was immobilized on ELISA plates and mAb in a dose-dependent manner were added and allowed for binding. Bound anti-gp68 specific mAbs were detected using goat-anti-mouse IgG-HRP secondary antibodies. ELISA results confirmed specific recognition of gp68 antigen by all generated anti-gp68 mAbs.

Fig. 26 shows the detection of immobilized sgp68-V5-His by various anti-gp68 mAbs. Purified anti-gp68 mAbs were further analyzed for binding towards sgp68-V5-His protein in a dose-dependent manner. 10 µg/ml, 5 µg/ml or 2.5 µg/ml of each mAb was used for the detection of 1µg/ml immobilized recombinant, soluble gp68 protein in ELISA. Commercial available mouse IgG1 and IgG2a isotype antibody (Invitrogen) was used as negative control, as well as PBS only. Using an antibody detecting the V5-Tag on sgp68-V5-His served as a positive control for loading of coated antigen. All anti-gp68 mAbs specifically and similar recognize recombinant vFcyR gp68.

To determine differences among those mAbs ELISA-binding assays were repeated with further titration of anti-gp68 mAbs to identify most potent of them with regard to gp68 recognition.

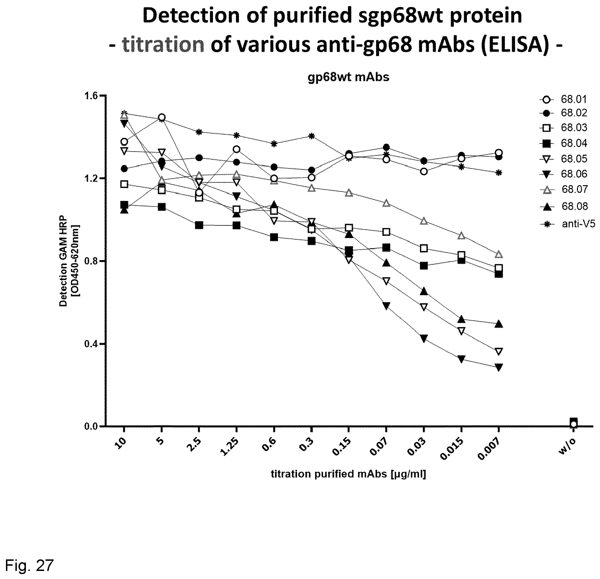

Fig. 27 shows the titration anti-gp68 mAbs on immobilized recombinant sgp68. Purified anti-gp68 mAbs were further analyzed for binding towards sgp68-V5-His protein in titration. 10 µg/ml mAbs was further titrated using log2 dilutions over 12 steps (last well without mAbs) for the detection of 1µg/ml immobilized recombinant, soluble gp68 protein in ELISA. Using an antibody detecting the V5-Tag on sgp68-V5-His served as a positive control for loading of coated antigen. All anti-gp68 mAbs specifically recognize recombinant vFcyR gp68. In addition, now clearly, dose-dependent variations especially in lower concentration become obvious.

Based on those ELISA data mAbs were selected for best binding affinity towards recombinant gp68, efficient recognizing the antigen also in very low mAb concentrations. This may also provide advantages when antigen-concentrations will be at low detection limit.

### Detection of vFcyRs gp34 in ELISA using mAbs

To further characterize newly generated mAbs, raised against vFcyRs gp34, were tested for binding and recognition of the appropriate recombinant antigen. First, mAbs were tested on immobilized recombinant, soluble gp34mtrp-V5-His protein that was used for immunization. The antigen was immobilized on ELISA plates and mAb in a dose-dependent manner were added and allowed for binding. Bound anti-gp34mtrp specific mAbs were detected using goat-anti-mouse IgG-HRP secondary antibodies. To our surprise, ELISA results confirmed specific recognition of gp34mtrp protein, only by anti-gp34mtrp mAbs. As a reminder, gp34mtrp-mAbs were generated by immunizing mice using the sgp34mtrp. This protein is impaired for IgG-Fc-binding and is structurally restricted to monomers and dimers, lacking the ability to form higher oligomers.

None of the anti-gp34wt mAbs (native sgp34wt was used for immunization) is capable to recognize gp34mtrp protein suggesting that those mAbs recognize a structural determinant of gp34 only formed and accessible by the native gp34 wild-type protein.

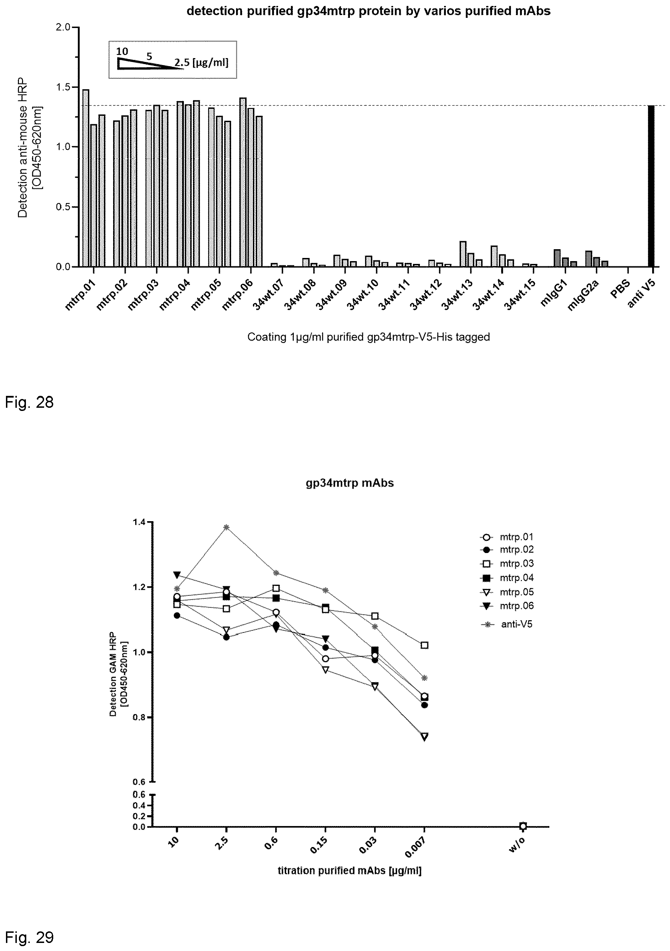

Fig. 28 shows the detection of immobilized sgp34mtrp-V5-His by various anti-gp34wt and anti-gp34mtrp mAbs. Purified anti-gp34 mAbs were further analyzed for binding towards sgp34mtrp-V5-His protein in a dose-dependent manner. 10 µg/ml, 5 µg/ml or 2.5 µg/ml of each mAb was used for the detection of 1 µg/ml immobilized recombinant, soluble gp34mtrp protein in ELISA. Commercial available mouse IgG1 and IgG2a isotype antibody (Invitrogen) was used as negative control, as well as PBS only. Using a antibody detecting the V5-Tag on sgp68-V5-His served as a positive control for loading of coated antigen. All anti-gp34mtrp mAbs specifically and similar recognize recombinant sgp34mtrp protein but none of the anti-gp34wt mAbs recognize sgp34mtrp protein.

To determine differences among those mAbs ELISA-binding assays were repeated with further titration of anti-gp34mtrp mAbs to identify most potent of them with regard to gp34mtrp protein recognition.

Fig. 29 shows the titration of anti-gp34mtrp mAbs on immobilized recombinant sgp34mtrp. Purified anti-gp34mtrp mAbs were further analyzed for binding towards sgp34mtrp-V5-His protein in titration. 10 µg/ml mAbs was further titrated using log4 dilutions over 7 steps (last well without mAbs) for the detection of 1 µg/ml immobilized recombinant, soluble gp34mtrp protein in ELISA. Using an antibody detecting the V5-Tag on sgp34mtrp-V5-His served as a positive control for loading of coated antigen. All anti-gp34mtrp mAbs specifically recognize recombinant sgp34mtrp protein. In addition, now, dose-dependent variations especially in lower concentration become obvious.

To further characterize newly generated mAbs raised against vFcyRs gp34, purified mAbs were tested for binding and recognition of the appropriate recombinant antigen. Next, mAbs were tested on immobilized recombinant, soluble sgp34wt-V5-His protein that was used for immunization. The antigen was immobilized on ELISA plates and mAb in a dose-dependent manner were added and allowed for binding. Bound anti-gp34wt specific mAbs were detected using goat-anti-mouse IgG-HRP secondary antibodies. ELISA data revealed, all anti-gp34mtrp mAbs and all anti-gp34wt mAbs recognize sgp34wt protein. As a reminder, gp34wt-mAbs were generated by immunizing BALB/c mice using the native sgp34wt protein. This protein is fully functional with regard to IgG-Fc-binding and structurally forms monomers, dimers and higher oligomers.

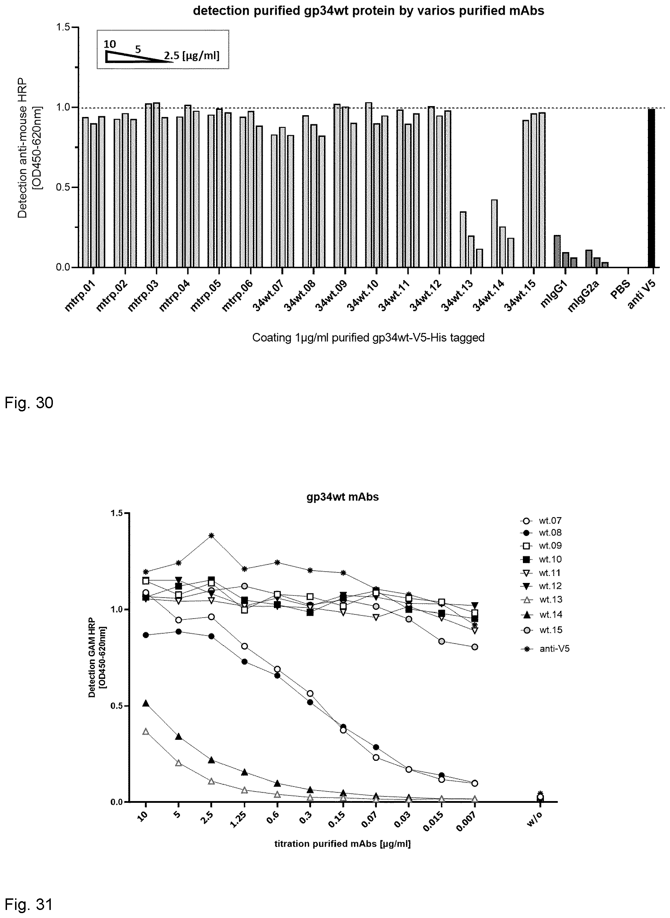

Fig. 30 shows the detection of immobilized sgp34wt-V5-His by various anti-gp34wt and anti-gp34mtrp mAbs. Purified anti-gp34 mAbs were further analyzed for binding towards sgp34wt-V5-His protein in a dose-dependent manner. 10 µg/ml, 5 µg/ml or 2.5 µg/ml of each mAb was used for the detection of 1 µg/ml immobilized recombinant, sgp34wt protein in ELISA. Commercial available mouse IgG1 and IgG2a isotype antibody (Invitrogen) was used as negative control, as well as PBS only. Using an antibody detecting the V5-Tag on sgp34wt-V5-His served as a positive control for loading of coated antigen. All anti-gp34mtrp mAbs and all anti-gp34wt mAbs specifically and similar recognize recombinant sgp34wt protein. Only mAbs 34wt.13 (IgG2a subtype) and 34wt.14 (IgG2a subtype) are different and show only low affinity binding towards recombinant sgp34wt protein.

To determine differences among those mAbs, ELISA-binding assays were repeated with further titration of anti-gp34wt mAbs and anti-gp34mtrp mAbs to identify most potent of them with regard to sgp34wt protein recognition.

Fig. 31 illustrates the titration of anti-gp34wt mAbs on immobilized recombinant sgp34wt. Purified anti-gp34wt mAbs were further analyzed for binding towards sgp34wt-V5-His protein in titration. 10 µg/ml mAbs was further titrated using log2 dilutions over 11 steps (last well without mAbs) for the detection of 1µg/ml immobilized recombinant, sgp34wt protein in ELISA. Using an antibody detecting the V5-Tag on sgp34mtrp-V5-His served as a positive control for loading of coated antigen. All anti-gp34wt mAbs specifically recognize recombinant sgp34wt protein. In addition, now drastically, dose-dependent variations especially in lower concentration become obvious. Again, mAbs 34wt.13 (IgG2a subtype) and 34wt.14 (IgG2a subtype) exhibit only low affinity binding towards recombinant sgp34wt protein.

To exclude, differences in recognition are based on immobilization of antigen due to direct protein coating, ELISA binding experiments were repeated and antigens were pre-bound on Nickel-coated plates. Results obtained were the same as in direct-antigen coating ELISAs previously.

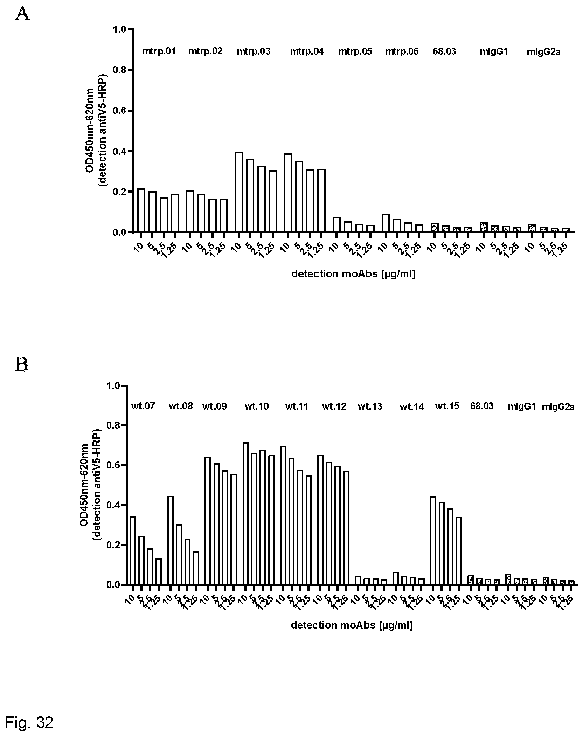

Fig. 32 is the detection of sgp34wt-V5-His on Nickel-coated plates by anti-gp34 mAbs. To exclude structural differences in antigen-presentation, instead of direct coating, sgp34wt-V5-His protein was incubated on Nickel-coated wells. 10 µg/ml, 5 µg/ml or 2.5 µg/ml of each mAb was added for the detection of 1 µg/ml pre-bound recombinant, soluble sgp34wt protein in ELISA. Detection was either performed with mtrp-mAbs (A) or with wt-mAbs (B). Commercial available mouse IgG1 and IgG2a isotype antibody (Invitrogen) was used as negative control, as well as one of the anti-gp68 specific novel mAbs. All tested anti-gp34mtrp mAbs and all anti-gp34wt mAbs specifically recognize recombinant sgp34wt protein, except for mAbs 34wt.13 (IgG2a subtype) and 34wt.14 (IgG2a subtype) as shown before. Especially anti-gp34wt mAbs exhibit broader differences in binding affinities.

Same experiments were done using sgp34mtrp-V5-His tagged protein pre-incubated on Nickel-coated ELISA plates. Similar results were generated as seen in direct antigen coating before. None of the anti-gp34wt mAbs does recognize the mtrp-variant of gp34 confirming that antibodies derived from immunization with the native, multimeric form of vFcyR gp34 recognize epitopes within the protein either not present in the structurally impaired mtrp-variant or those epitopes are hidden under such conditions and therefore not accessible.

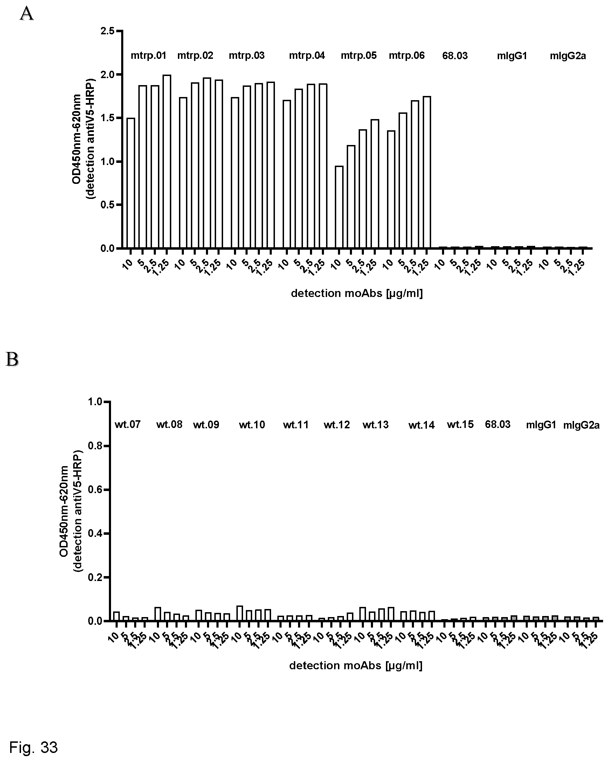

Fig. 33 shows the detection of sgp34mtrp-V5-His on Nickel-coated plates by anti-gp34 mAbs. To exclude structural differences in antigen-presentation, instead of direct coating, sgp34mtrp-V5-His protein was incubated on Nickel-coated wells. 10 µg/ml, 5 µg/ml or 2.5 µg/ml of each mAb was added for the detection of 1 µg/ml pre-bound recombinant, soluble gp34mtrp protein in ELISA. Detection was either performed with mtrp-mAbs (A) or with wt-mAbs (B). Commercial available mouse IgG1 and IgG2a isotype antibody (Invitrogen) was used as negative control, as well as one of the anti-gp68 specific novel mAbs. All tested anti-gp34mtrp mAbs recognize sgp34mtrp bound to Nickel-coated wells. Again, none of the anti-gp34wt mAbs do recognize sgp34mtrp protein, neither in direct-antigen coating nor pre-bound to Nickel-opsonized plates.

### Detection vFcyRs gp68 by mAbs in WB

In addition to functional characterization of novel anti-gp34 and anti-gp68 mAbs for detection of their appropriate antigen (recombinant or in the virus context), mAbs were further tested for specificity in western blot (WB) analysis.

Recombinant sgp68wt protein, or slCOSL as non-binding control protein were separated on SDS-page and western blot was performed as described elsewhere. Membrane-bound antigens were incubated with anti-gp68 mAbs and binding was visualized using goat-anti mouse IgG-HRP secondary antibody and chemiluminescent signals measured by incubation of the membranes in substrate. Results are showing following.

Two mAbs derived originally from the same motherwell (MW) by sub-cloning, showed the strongest signal for western blot detection of recombinant sgp68 protein (used also for immunization), whereas, all the other anti-gp68 mAbs recognize only to a lower level higher molecular weight form of gp68. One mAb (gp68.06) at least in western blot shows only a very weak detection signal if positive at all.

None of the anti-gp68 mAbs recognizes loaded slCOSL control-protein, indicating that those mAbs does bind to the gp68 ECD and not to the V5-His-Tag.

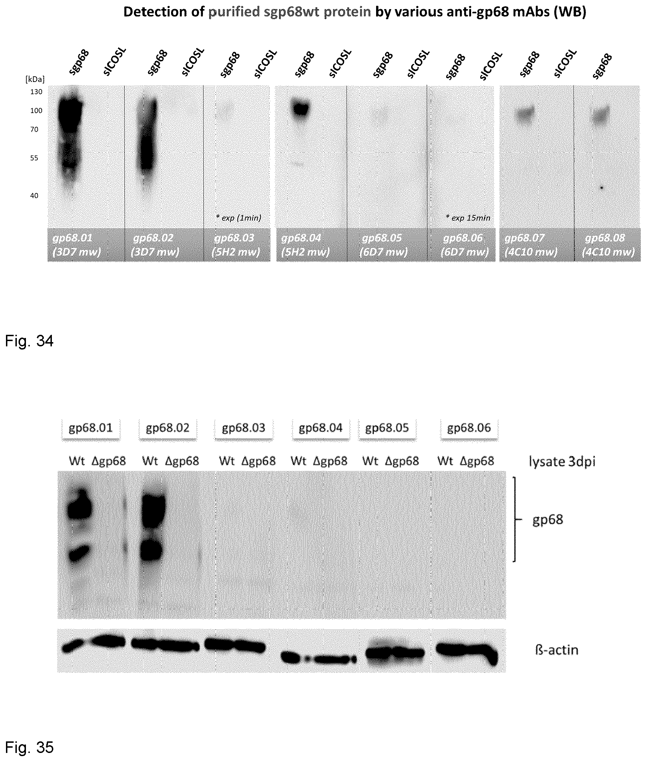

Fig. 34 shows the detection of recombinant sgp68 by anti-gp68 mAbs. Recombinant proteins sgp68wt or slCOSL were separated on SDS-page and transferred onto a membrane for western blot analysis. Membrane pieces containing one lane with sgp68 and one lane slCOSL each were incubated with indicated mAbs raised against vFcyR gp68. mAbs 68.01 and 68.02 show strongest detection signals for recombinant gp68, followed by other anti-gp68 mAbs recognizing at least higher glycosylated form of gp68 in western blot, but with lower signals. None of the anti-gp68 mAbs cross-react with recombinant slCOSL protein.

Western blot analysis were repeated using this time cell lysates from cells infected either with HCMV AD169 wild-type strain or a AD169 virus mutant lacking vFcyR gp68 (Δgp68). In virus-context now, two mAbs are able to detect gp68 also in virus context: gp68.01 and gp68.02. Both mAbs showed also the strongest effect on binding in western blot before using recombinant protein.

Fig. 35 shows the detection of vFcyR gp68 by anti-gp68 mAbs in infection. MRC5 fibroblast were infected with MOI 2. Three days post infection cell lysates were generated. Next, lysates were analyzed in western blot for detection of vFcyR gp68 by novel mAbs. Again, only mAbs 68.01 and 68.02 shows specific detection of gp68 now also in infection, indicating that those two mAbs indeed recognize vFcyR gp68 and can be used for western blot analysis. Membrane pieces containing one lane with lysate from AD169wt infected cells and one lane containing lysate from AD169 Δgp68 infected cells were incubated with indicated mAbs. As observed before, only mAbs 68.01 and 68.02 detect vFcyR gp68 in western blot. No detection is observed in lysates lacking gp68 demonstrating specificity of those mAbs. β-actin stain was performed as loading control, indicating that comparable amounts of cell lysates were loaded onto the gel.

### Detection by immuno-precipitation using lysates of HCMV-infected cells

Finally, anti-vFcyR gp68 mAbs were used for immunoprecipitation experiments. MRC5 fibroblasts were infected with HCMV AD169wt strain or a virus mutant lacking vFcyR gp68 (Δgp68) for 4 days with MOI 3. Infected cells were metabolically labeled with ³⁵S for 2 hours at 37°C, washed and lysed using NP40-lysis buffer. Lysates were incubated with indicated mAbs or human IgG Fc-fragment to pulldown vFcyR gp68 specifically or both HCMV-encoded vFcyRs gp34, gp68 using human IgG-Fc-fragment to precipitated Fc-binding proteins. Samples were deglycosylated by EndoH and separated on SDS-page gradient gel. In summary, all anti-gp68 mAbs recognize and bind vFcyR gp68 from infected cells. To some extend also vFcyR gp34 is detected probably due to the strong binding of vFcyR gp34 towards mouse IgGs, stronger to mouse IgG1 than to mouse IgG2a (68.07 and 68.08). Therefore this small background binding to gp34 is absent when using 68.07 and 68.08 mAbs.

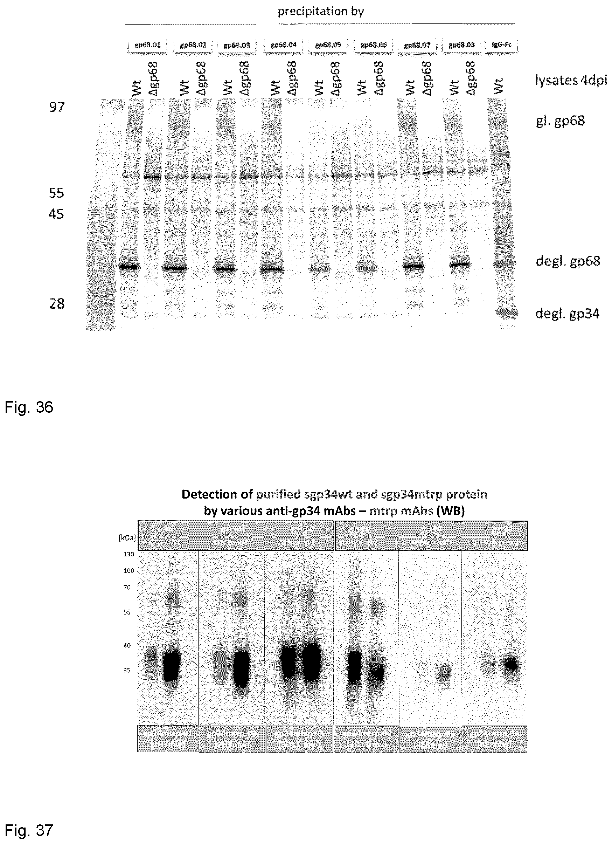

Fig. 36 shows the precipitation of vFcyR gp68 from infected cells by anti-gp68 mAbs. MRC5 fibroblast were infected with MOI 2. Four days post infection cells were metabolically labelled and following lysates were incubated with indicated anti-gp68 mAbs or human IgG-Fc-fragment for precipitation of vFcyR gp68. Indeed, all anti-gp68 mAbs precipitated vFcyR gp68 from AD169 infected cells. Signals were absent in lysates lacking vFcyR gp68 demonstrating specificity. Except for mAbs 68.05 and 68.06 also EndoH-resistant, glycosylated gp68.

### Detection vFcγR gp34 by various mAbs in WB

In addition to functional characterization of novel anti-gp34wt- and anti-gp34mtrp- mAbs for detection of their appropriate antigen in ELISA, mAbs were further tested for their specificity in western blot (WB) analysis. Recombinant sgp34wt or sgp34mtrp protein were loaded onto SDS-PAGE and detected in western blot using anti-gp34mtrp mAbs. Most of them (mtrp.01-mtrp.04) recognize sgp34wt and gp34mtrp protein, including dimers for the sgp34wt protein. mAbs mtrp.05 and mtrp.06 are less efficient to recognize sgp34wt/mtrp protein in western blot. Results confirm binding affinities seen before in ELISA.

Fig. 37 shows the detection of recombinant sgp34wt or sgp34mtrp by anti-gp34 mAbs. Recombinant proteins sgp68 or slCOSL were separated on SDS-page and transferred onto a membrane for western blot analysis. Membrane pieces containing one lane with sgp68 and one lane slCOSL each were incubated with indicated mAbs raised against vFcyR gp68. mAbs 68.01 and 68.02 show strongest detection signals for recombinant gp68, followed by other anti-gp68 mAbs recognizing at least higher glycosylated form of gp68 in western blot, but with lower signals. None of the anti-gp68 mAbs cross-react with recombinant slCOSL protein.

In addition, mAbs raised against gp34mtrp were used for precipitation of vFcyR gp34 from infected cells. For comparison and to demonstrate specificity, lysates from HCMV AD169 wild-type cells were used as well as from cells infected with a HCMV AD169 mutant lacking vFcyR gp34 (Δgp34). Although not to similar levels, all mAbs raised against sgp34mtrp protein are able to recognize vFcyR gp34 in infected cells.

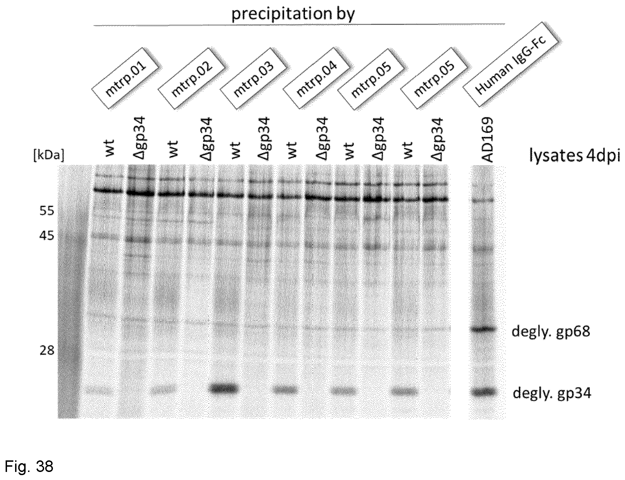

Fig. 38 shows the precipitation of vFcyR gp34 from infected cells by anti-gp34mtrp mAbs. MRC5 fibroblast were infected with MOI 2. Four days post infection cells were metabolically labelled and following lysates were incubated with indicated anti-gp34mtrp mAbs for precipitation of vFcyR gp34 or human IgG-Fc-fragment for precipitation of both, vFcyRs gp34 and gp68. Indeed, all anti-gp34mtrp mAbs efficiently precipitated vFcyR gp34 from HCMV AD169 infected cells. Signals were absent in lysates lacking vFcyR gp34 demonstrating specificity.

Next, mAbs raised against sgp34wt protein were investigated for their specificity in western blot (WB) analysis. Recombinant sgp34wt, sgp34mtrp or slCOSL (non-binding control) protein were loaded onto SDS-PAGE and detected in western blot using anti-gp34wt mAbs. Most of them (except mAb 34wt.15) recognize sgp34wt protein; again, none of them is able to detect sgp34mtrp protein. In addition, no unspecific binding on membranes loaded with slCOSL protein was observed. Positive control for proteins used in this experiment was done using anti-His antibodies or one of the functional anti-gp34mtrp mAbs from before. Results confirm binding affinities seen before in ELISA with some interesting exceptions. First, mAbs exhibits strong binding in ELISA 34wt.15, but at least in this assay was not working. This needs to be further investigated. In addition, mAbs wt.13 and wt.14 showed only weak detection signals in ELISA but seem to be comparable with other mAbs in this western blot.

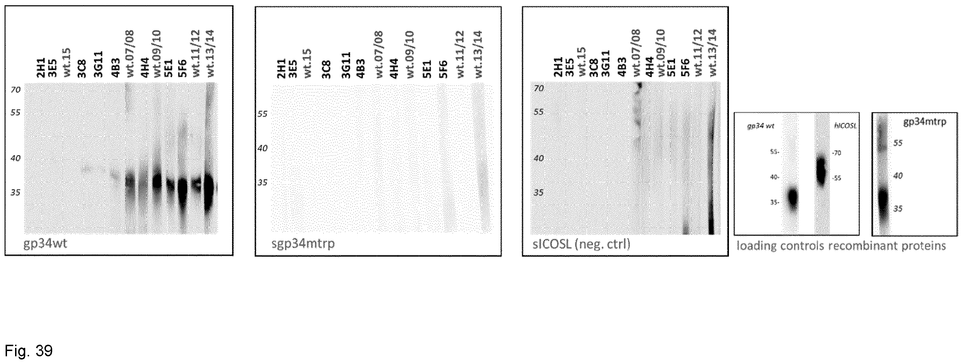

Fig. 39: detection of recombinant sgp34wt or sgp34mtrp by anti-gp34wt mAbs. Recombinant proteins sgp34wt, sgp34mtrp or slCOSL were separated on SDS-page and transferred onto a membrane for western blot analysis. Membrane lanes containing recombinant proteins were stained with indicated SN from hybridomas of mAbs. Results verified results of ELISA binding studies: mAbs anti-gp34wt do not recognize recombinant sgp34mtp protein.

Next, regions within the antigen were determined that are recognized by appropriate mAbs. For this purpose, truncation variants of sgp34wt protein ECD were transiently transfected into HEK-293T cells. One day after transfection, cells were metabolically labeled by ³⁵S, 2hrs 37°C, lysed and precipitation experiments were done incubating indicated lysates with indicated anti-gp34mtrp mAbs. To the end, three versions of sgp34wt ECD were used: (i) sgp34wt protein consisting of wildtype sequence covering amino acids 1-179 (complete ECD; used for immunization), (ii) sgp34wt protein consisting of wildtype sequence covering amino acids 1-156 (loosing two predicted O-glycosylation sites) and (iii) sgp34wt protein consisting of wildtype sequence covering amino acids 1-124 (complete Ig-like domain). Previous studies by our group demonstrated, that the region necessarily for IgG-Fc-binding involves amino acids of the ECD including complete Ig-like domain and further amino acids. IgG-Fc-binding experiments done earlier confirmed, that sgp34wt (1-124aa) does not bind any longer to IgG-Fc.

The results indicate at least (i) mAbs mtrp.01 and mtrp.02 recognize all three soluble variants of sgp34wt, (ii) mAbs mtrp.03 and mtrp.04 show strongest binding to the variant of sgp34wt containing the complete ECD, but do not recognize the variant covering amino acids 1-124 and (iii) mAbs mtrp.05 and mtrp.06 show similar binding to the variants of sgp34wt containing 1-156aa and also 1-179aa, but also do not recognize the variant covering amino acids 1-124.

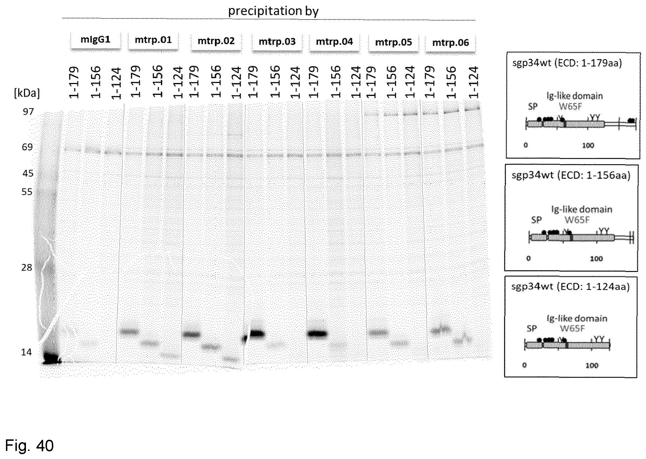

Fig. 40: epitope mapping of mAbs anti-gp34mtrp. To further determine binding epitopes recognized by various anti-gp34mtrp mAbs, truncated versions of sgp34wt protein were transiently transfected in HEK-293-T cells. One day post transfection cells were metabolically labeled with ³⁵S and lysates used for precipitation by indicated mAbs or mouse IgG1 isotype control. Samples were separated on SDS-gradient gel and analyzed on phosphoplate. Results confirmed data from ELISA binding studies: all mAbs anti-gp34mtrp recognize sgp34wt protein, whereby mtrp.01 and mtrp.02 in addition also recognize the shortest truncation variant comprising amino acids 1-124.

In summary, precipitation experiments confirmed again, that all anti-gp34mtrp mAbs recognize recombinant sgp34wt protein and can be used for pulldown experiments.

In line with that, we also wanted to determine regions within the antigen that are recognized by anti-gp34wt mAbs. As before, truncation variants of sgp34wt protein ECD were transiently transfected into HEK-293T cells. One day after transfection, cells were metabolically labeled by ³⁵S, 2hrs 37°C, lysed and precipitation experiments were done incubating indicated lysates with indicated anti-gp34wt mAbs. To the end, three versions of sgp34wt ECD were used: (i) sgp34wt protein consisting of wildtype sequence covering amino acids 1-179 (complete ECD; used for immunization), (ii) sgp34wt protein consisting of wildtype sequence covering amino acids 1-156 (losing two predicted O-glycosylation sites) and (iii) sgp34wt protein consisting of wildtype sequence covering amino acids 1-124 (complete Ig-like domain). Previous studies by our group demonstrated, that the region necessarily for IgG-Fc-binding involves amino acids of the ECD including complete Ig-like domain and further amino acids. IgG-Fc-binding experiments done earlier confirmed, that sgp34wt (1-124aa) does not bind any longer to IgG-Fc.

The results indicate at least (i) all anti-gp34wt mAbs recognize sgp34wt variants including amino acids 1-156 and the amino acids 1-179, (ii) all mAbs detect the recombinant soluble truncation variants of sgp34wt protein to a similar extend, except mAbs wt.13 and wt.14 (both mouse IgG2a) and (iii) none of the anti-gp34wt mAbs precipitated truncation variant 1-124 amino acids.

Interesting, one can clearly see non-specific precipitation by the mouse IgG1 subtype, demonstrating remaining binding capacity of vFcyR gp34 to mouse IgG1 as mentioned earlier.

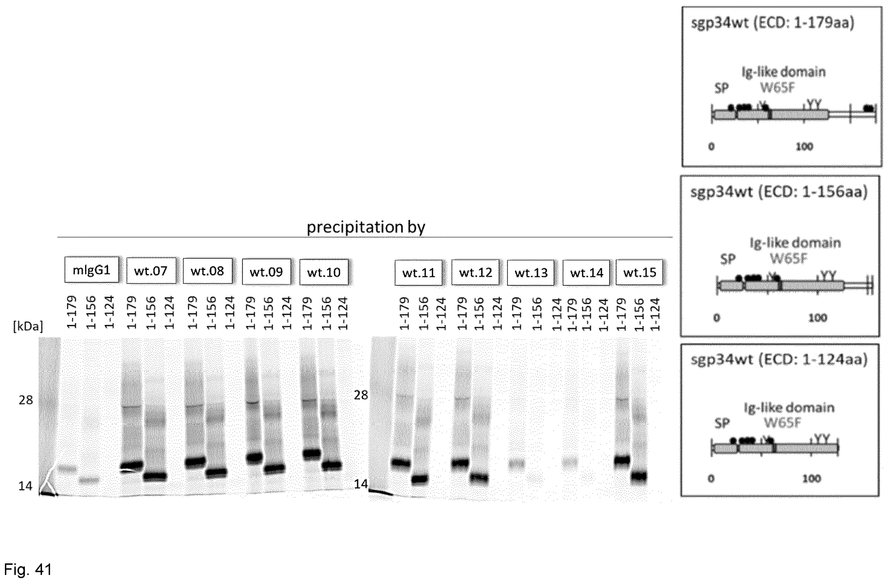

Fig. 41: epitope mapping of mAbs anti-gp34wt. To further determine binding epitopes recognized by various anti-gp34wt mAbs, truncated versions of sgp34wt protein were transiently transfected in HEK-293-T cells. One day post transfection cells were metabolically labeled with ³⁵S and lysates used for precipitation by indicated mAbs or mouse IgG1 isotype control. Samples were separated on SDS-gradient gel and analyzed on phosphoplate. Results confirmed data from ELISA binding studies: all mAbs anti-gp34wt recognize sgp34wt protein, whereby wt.13 and wt.14 exhibit weakest interaction. None of the mAbs recognizes the shortest truncation variant, comprising amino acids 1-124.

### Blocking capacity of anti-vFcyR gp34 and gp68 mAbs

Glycoproteins gp34 and gp68 are HCMV encoded type-I transmembrane molecules able to bind to the Fc-part of human immunoglobulin G (IgG).

By this, gp34 and gp68 can interact with IgG, as their human counterparts the human FcyRs. Through this interaction, they antagonize IgG-dependent host FcyR activation e.g. ADCC mediated by FcγRIII+-NK cells.

Since IgG-binding by vFcyRs gp34 and gp68 is mandatory for their inhibitory effect on host FcyRs we next wanted to identify the ability of our novel generated anti-vFcyR mAbs with respect to their capacity to block IgG binding.

### IgG-blocking capacity of anti-vFcγR gp68-specific mAbs

To test if anti-gp68 specific mAbs can interfere with IgG-binding ability of gp68 and human IgG. Therefore, recombinant, soluble gp68 was pre-incubated with indicated anti-gp68 specific mAbs, mouse IgG isotype or PBS only. Next, antibody bound sgp68 was incubated on IgG-immobilized ELISA plates. IgG-binding by sgp68 in presence or absence of potential blocking mAbs was determined detecting bound glycoprotein sgp68-V5-His using anti-V5 antibody.

Results indicated, that all anti-gp68 mAbs dose-dependently block IgG-binding ability of sgp68 in the following hierarchy: 68.01 > 68.03 > 68.07 / 68.08 / 68.04> 68.02 / 68.05 / 68.06.

Incubation with mouse IgG1 and IgG2a isotypes only, did not influence IgG-binding by sgp68.

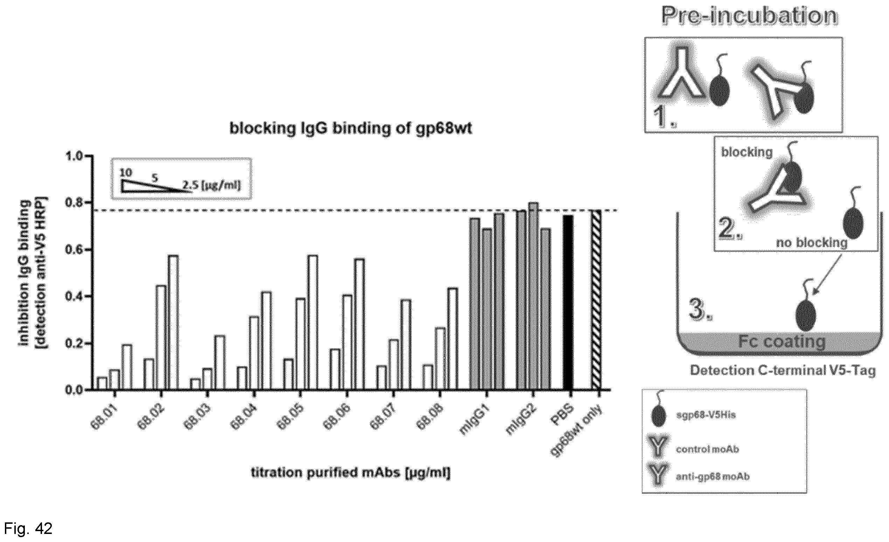

Fig. 42: IgG blocking ability by anti-gp68 mAbs. vFcγR gp68 is an HCMV encoded IgG-Fc-binding. mAbs were pre-incubated with recombinant, sgp68 and next added to IgG-immobilized ELISA plates. Bound sgp68 in absence or presence of blocking mAbs is detected using anti-V5-HRP antibody recognizing IgG-bound sgp68-V5-His. All anti-gp68 specific mAbs have the potential to block vFcγR gp68 IgG-binding in a dose-dependent manner. Effects by non-specific mouse IgG isotypes are indicated.

Finally, summarized in table 6 (Tab. 6), characteristics and potentials of anti-vFcγR gp68-specific mAbs are highlighted.

In the end, 8 anti-gp68 mAbs were provided, all of them specifically recognizing vFcyR gp68 as soluble, recombinant protein but also in lysates of HCMV AD169 infected cells. More important, all of them have the potential to interfere with IgG-binding ability of gp68.

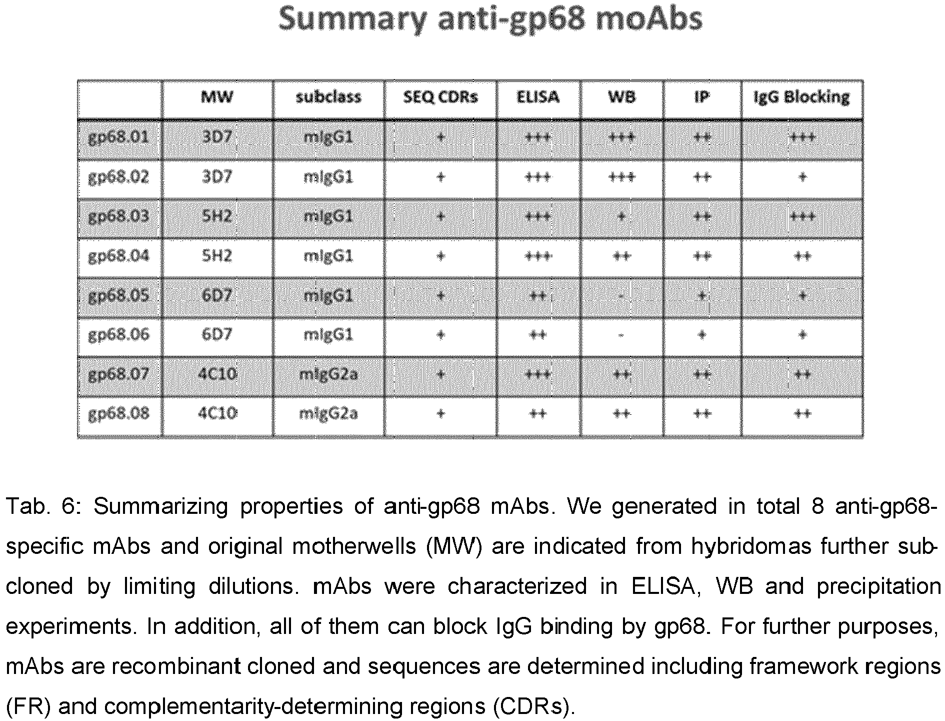

### IgG-blocking capacity of anti-vFcγR gp34-specific mAbs

First, binding capacity by various anti-gp34wt and anti-gp34mtrp mAbs towards recombinant sgp34wt protein will be recapitulated. Therefore, constant immobilized sgp34wt protein detection by constant concentration by indicated mAbs was measured in ELISA, highlighting differences upon antigen binding that might also affect blocking capacities. Bound mAbs were detected using goat anti-mouse IgG-HRP secondary antibody.

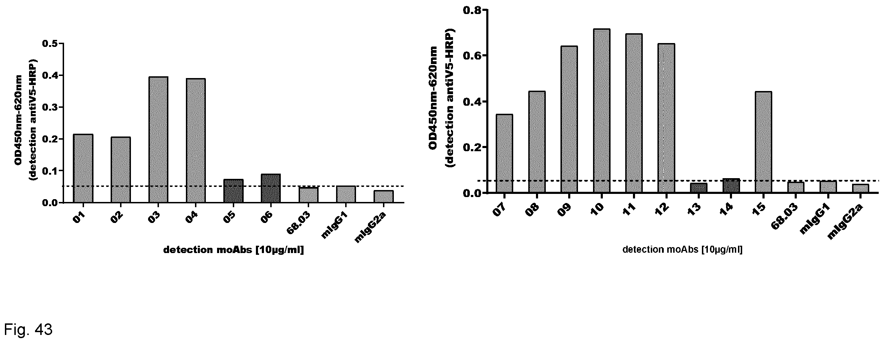

Fig. 43 shows the detection of immobilized sgp34wt-V5-His by various anti-gp34wt and anti-gp34mtrp mAbs. Purified anti-gp34 mAbs (both subgroups in comparison) at 10 µg/ml concentration were analyzed for binding of 1 µg/ml immobilized sgp34wt-V5-His protein in ELISA. Commercial available mouse IgG1 and IgG2a isotype antibody (Invitrogen) were used as negative control, as well as one anti-gp68-specific mAbs. Tested anti-gp34mtrp mAbs and all anti-gp34wt mAbs specifically recognize recombinant sgp34wt protein, except wt.13 and wt.14 and only to some less extend mtrp.05 and mtrp.06.

First, the blocking potential of anti-gp34mtrp mAbs (mtrp.01 - mtrp.06) was investigated and afterwards anti-gp34wt mAbs (wt.07 - wt.15).

To test if anti-gp34mtrp specific mAbs can interfere with IgG-binding ability of gp34 and human IgG, recombinant, sgp34wt (gp34mtrp is impaired for IgG-Fc-binding) was pre-incubated with indicated anti-gp34mtrp specific mAbs, mouse IgG isotype or PBS only. Next, antibody bound sgp34wt was incubated on IgG-immobilized ELISA plates. IgG-binding by sgp34wt in presence or absence of potential blocking mAbs was determined detecting bound glycoprotein sgp34wt-V5-His using anti-V5 antibody.

Results indicated, not all but some anti-gp34mtrp mAbs dose-dependently can block IgG-binding ability of sgp34wt in the following hierarchy: mtrp.03 / mtrp.04 > mtrp.01 / mtrp.02 > mtrp.05 / mtrp.06.

Incubation with mouse IgG1 affects to some extend sgp34wt binding to immobilized human IgG, since gp34wt also shows binding ability predominantly to mouse IgG1. Mouse IgG2a isotype only, did not influence IgG binding by sgp34wt. Same holds true for PBS only. Nethertheless, blocking potential of anti-gp34mtrp mAbs is much stronger compared to mouse IgG1 isotype alone.

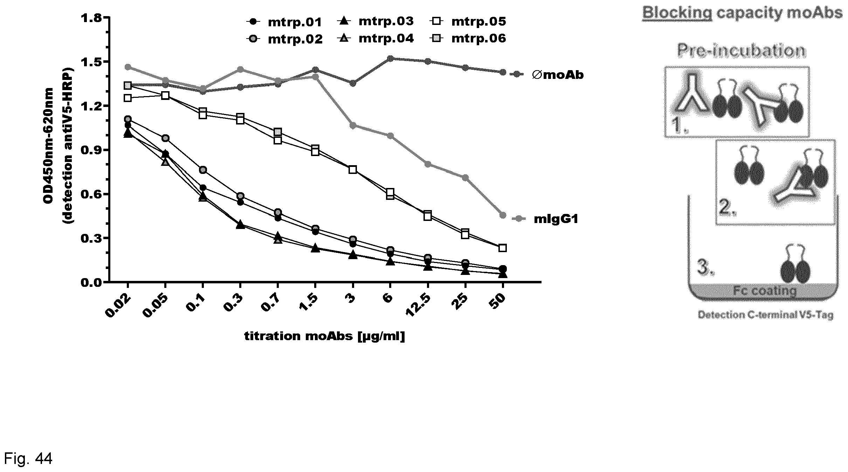

Fig. 44: IgG blocking ability by anti-gp34mtrp mAbs. vFcγR gp34 is an HCMV encoded IgG-Fc-binding. Anti-gp34mtrp-specific mAbs (in titration) were pre-incubated with recombinant, sgp34wt (constant amount 1 µg/ml) and added to IgG-immobilized ELISA plates. Bound sgp34wt in absence or presence of blocking mAbs is detected using anti-V5-HRP antibody recognizing IgG-bound sgp34wt-V5-His. All anti-gp34mtrp-specific mAbs have the potential to block vFcyR gp34 IgG-binding but with variations in efficiency. Dose-dependent potential of mAbs used for blocking is indicated. Effect by non-specific mouse IgG isotype IgG1 is shown. In addition, binding of sgp34wt to immobilized IgG in absence of any mAb is depicted.

Next, the blocking potential of anti-gp34wt mAbs (wt.07 - wt.15) was investigated. To test if anti-gp34wt specific mAbs can interfere with IgG-binding ability of gp34 and human IgG. Therefore, recombinant, sgp34wt was pre-incubated with indicated anti-gp34wt specific mAbs, mouse IgG isotype or PBS only. Next, antibody bound sgp34wt was incubated on IgG-immobilized ELISA plates. IgG-binding by sgp34wt in presence or absence of potential blocking mAbs was determined detecting bound glycoprotein sgp34wt-V5-His using anti-V5 antibody.

Results indicated, not all but some anti-gp34wt mAbs dose-dependently can block IgG-binding ability of gp34 in the following hierarchy: wt.09 / wt.10 / wt.11 / wt.12 > wt.15 > wt.07 / wt.08.

mAbs wt.13 and wt.14 (both mouse IgG2a) does not significantly influence sgp34wt binding to immobilized IgG, even though mouse IgG1 shows stronger effects.

Incubation with mouse IgG1 affects to some extend sgp34wt binding to immobilized human IgG, since gp34wt also shows binding ability predominantly to mouse IgG1. Mouse IgG2a isotype only, did not influence IgG-binding by sgp34wt. Same holds true for PBS only. Nethertheless, blocking potential of anti-gp34wt mAbs is much stronger compared to mouse IgG1 isotype alone.

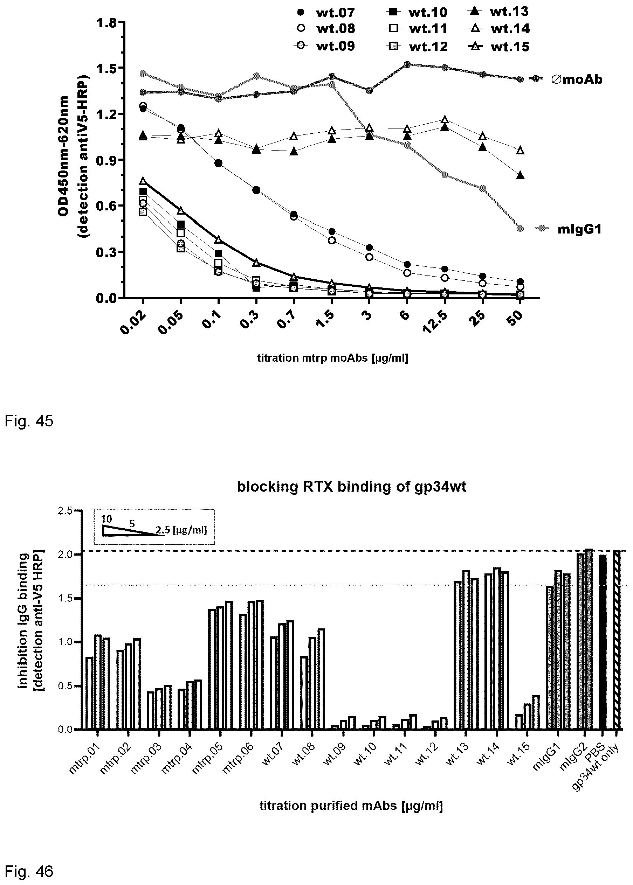

Fig. 45 shows the IgG blocking ability by anti-gp34wt mAbs. vFcγR gp34 is an HCMV encoded IgG-Fc-binding. Anti-gp34wt-specific mAbs (in titration) were pre-incubated with recombinant, sgp34wt (constant amount 1 µg/ml) and added to IgG-immobilized ELISA plates. Bound sgp34wt in absence or presence of blocking mAbs is detected using anti-V5-HRP antibody recognizing IgG-bound sgp34wt-V5-His. Most anti-gp34wt-specific mAbs have the potential to block vFcyR gp34 IgG-binding but with strong variations in efficiency. Dose-dependent potential of mAbs used for blocking is indicated. Also, effect of non-specific mouse IgG1 isotype is shown, highlighting that mAbs wt. 13 and wt. 14 cannot blocksgp34wt-IgG-binding. In addition, binding of sgp34wt to immobilized IgG in absence of any mAb is depicted.

Finally, IC50 values for blocking capacities by various anti-gp34 mAbs are calculated and summarized in table 7 (Tab. 7).

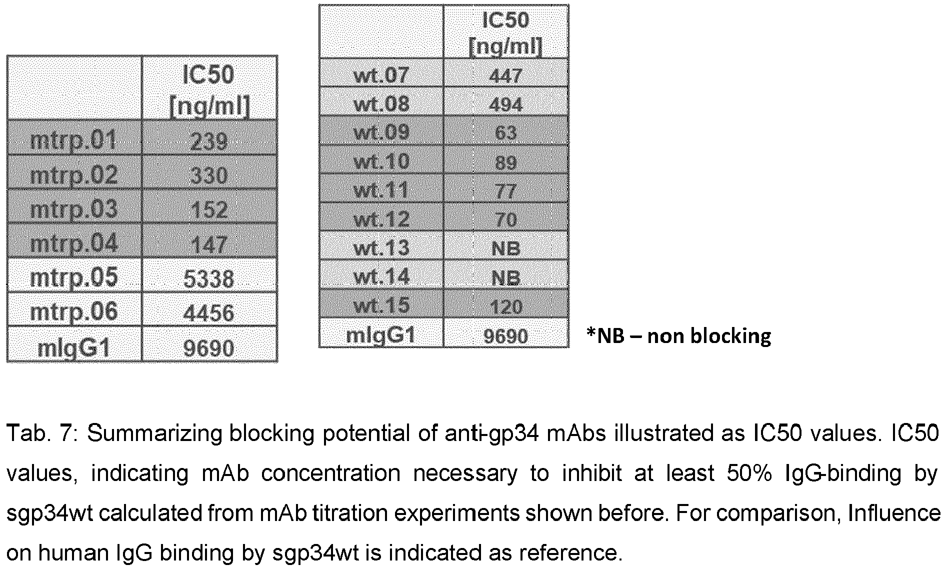

Finally, summarized in Tab. 8, characteristics and potentials of anti-vFcyR gp68-specific mAbs are highlighted.

In the end, we generated 15 anti-gp34wt mAbs (6 mAbs raised against sgp34mtrp protein: anti-gp34mtrp mAbs and 9 mAbs raised against sgp34wt protein: anti-gp34wt mAbs).

All of them specifically recognized vFcyR gp34wt as soluble, recombinant protein but also in lysates of cells infected with HCMV AD169 strain. Most importantly, all of them have the potential to interfere with IgG-binding ability of gp34wt.

Especially anti-gp34wt mAbs wt.09, wt.10, wt.11, wt.12 and t.15 show most promising blocking potential to interfere with vFcyR gp34 binding towards human IgG.

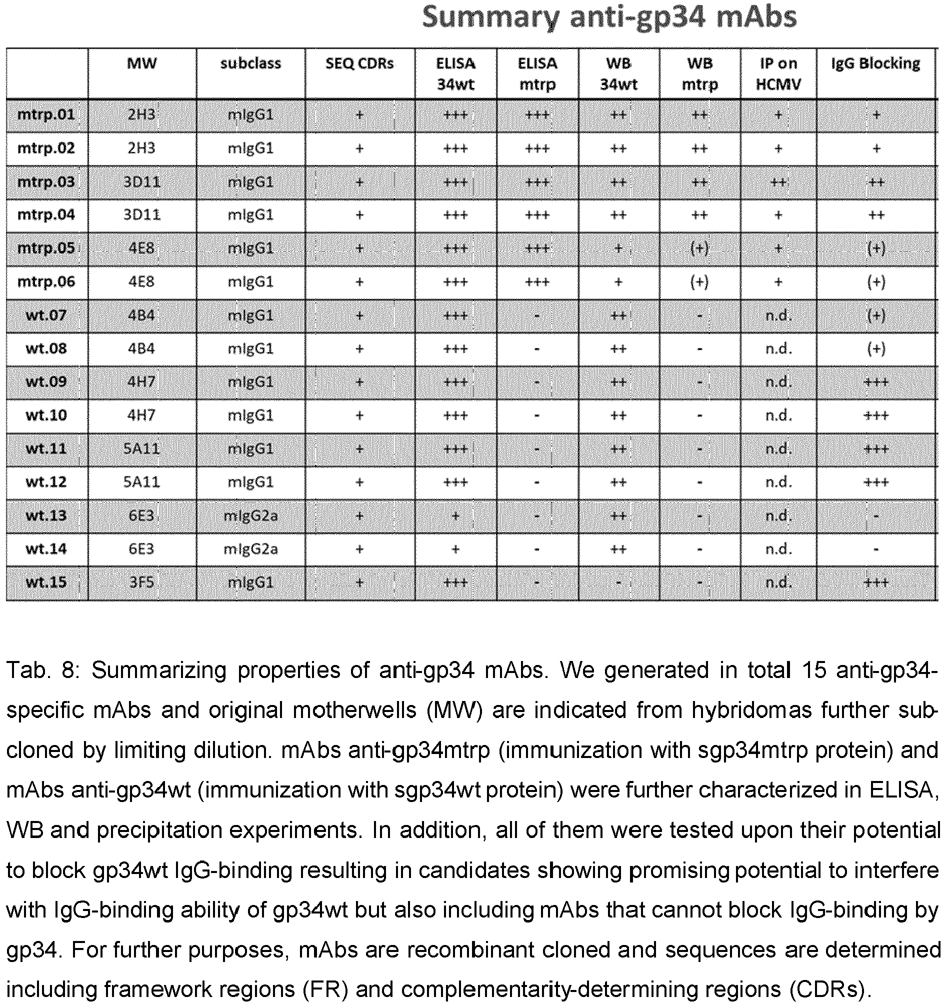

Only recently, we identified additional interaction partners of vFcγR gp34: (i) interaction with the membrane-bound B-cell receptor (BCR) of IgG+ memory B-cells, (ii) C-reactive protein (CRP and (iii) the neonatal Fc-Receptor (FcRn). Interaction and binding of vFcγR gp34 was demonstrated earlier in this study. While in the beginning mAbs anti-vFcγR gp68 and gp34 were generated to potentially be able to block IgG-mediated antagonization by this molecules we tested them also now for their ability to interact with gp34-CRP and gp34-FcRn interaction.

First, one experiment was performed demonstrating interference between gp34 and immobilized IgG as already shown before, this time testing anti-gp34wt and anti-gp34mtrp mAbs within the same experimental setup. Results obtained reflect previous results.

Fig.46 shows the IgG blocking ability by anti-gp34-specific mAbs. Anti-gp34wt-specific mAbs (10 µg/ml; 5 µg/ml or 2.5 µg/ml) were pre-incubated with recombinant, sgp34wt (constant amount of 1 µg/ml) and added to IgG-immobilized ELISA plates. Bound sgp34wt-V5-His in absence or presence of blocking mAbs is detected using anti-V5-HRP antibody. Most anti-gp34wt-specific mAbs have the potential to efficiently block vFcyR gp34 IgG-binding but with strong variations in efficiency. In addition, some gp34mtrp mAbs show a tendency for inhibition, while some mAbs do not essentially affect IgG-binding by gp34. Mouse IgG1 and IgG2a isotypes were used for reference and should not specifically influence IgG-interaction. In addition, binding of sgp34wt to immobilized IgG in absence of any mAb is depicted.

Next, sgp34wt and CRP interaction was analyzed, included various anti-gp34 mAbs, and identified their potential to block this interaction. Experiments were performed as described before for IgG-blocking ELISA, but this time using immobilized CRP as gp34-ligand.

Those mAbs exhibiting the strongest blocking effect on IgG-binding did not at all block the interaction towards CRP. In contrast, mAbs raised against the sgp34mtrp variant, impaired for IgG-binding and structurally impeded to form higher oligomers at least block gp34-CRP-binding by ~50%. Interestingly, also two mAbs raised against sgp34wt (wt.07 and wt.08) have the same blocking capacity as those mAbs. Based on phylogenetic tree analysis this makes sense, since they show closest relationship on protein level of their respective Fab-sequences (data not shown).

Further, this led to the assumption, that different epitopes of gp34wt protein are necessary for binding of vFcyR gp34 to IgG, compared to CRP. Moreover, this is underlined by unpublished data from our group (also shown here in this study): while gp34mtp protein is impaired for IgG-binding it remains binding ability towards CRP, although not to the same extend like gp34wt protein.

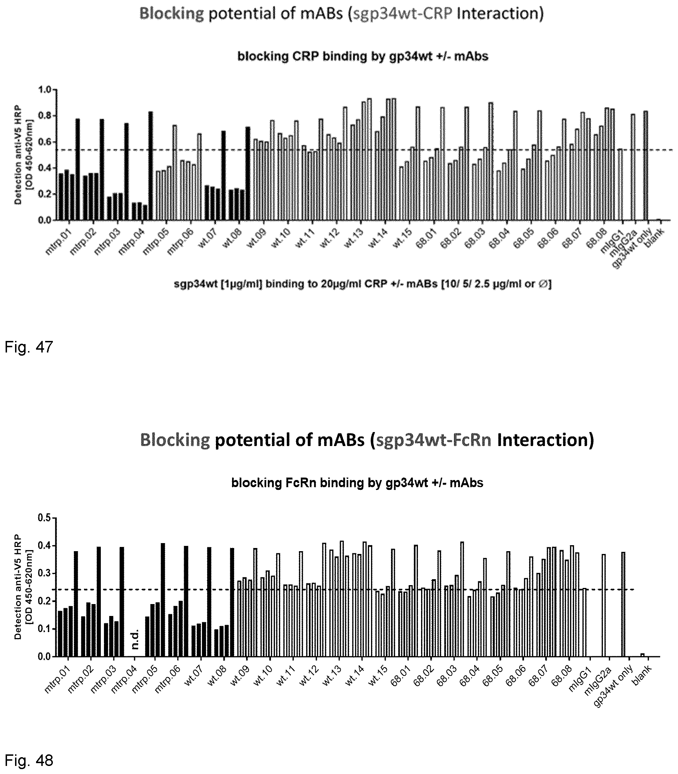

Fig. 47 shows the CRP blocking ability by anti-gp34-specific mAbs. Anti-gp34wt-specific mAbs (10 µg/ml; 5 µg/ml or 2.5 µg/ml or without any mAb) were pre-incubated with recombinant, sgp34wt (constant amount of 20 µg/ml) and added to CRP-immobilized ELISA plates. Bound sgp34wt-V5-His in absence or presence of blocking mAbs is detected using anti-V5-HRP antibody. Most anti-gp34mtrp-specific mAbs have the potential to block vFcyR gp34 CRP-binding up to 50% but with strong variations in efficiency. Here, mtrp.05 and mtrp.06 show less efficient blocking ability. Only wt.07 and wt.08 mAbs raised against sgp34wt protein have an effect on CRP-binding while all the other gp34wt mAbs and anti-gp68 mAbs (as control) does not block this interaction. Mouse IgG1 and IgG2a isotype controls were used for reference and should not influence CRP-interaction. In addition, binding of sgp34wt to immobilized CRP in absence of any mAb is depicted.

Furthermore, the gp34wt and FcRn interaction was investigated whereby various anti-gp34 mAbs were analyzed and their potential to block this interaction was identified. Experiments were performed as described before for IgG-blocking ELISA, but this time using immobilized FcRn as a novel gp34-ligand. Interaction of gp34 and FcRn was illustrated earlier.

Those mAbs exhibiting the strongest blocking effect on IgG-binding did not at all block the interaction towards FcRn. In contrast, mAbs raised against the gp34mtrp variant, impaired for IgG-binding and structurally impeded to form higher oligomers at least block gp34-CRP-binding by ~50%. Interestingly, also two mAbs (wt.07 and wt.08) have the same blocking capacity as those mAbs.

Moreover, those mAbs showing effects on the gp34 interaction with FcRn are also affecting the gp34-CRP interaction. This led us to speculate that those epitopes implicated in the gp34 interaction with CRP might be overlapping with binding sites for gp34 and FcRn interaction but distant from regions necessary for binding of vFcyR gp34 to IgG.

Fig. 48 shows the FcRn blocking ability by anti-gp34-specific mAbs. Anti-gp34wt-specific mAbs (10 µg/ml; 5 µg/ml or 2.5 µg/ml or without any mAb) were pre-incubated with recombinant, sgp34wt (constant amount of 1µg/ml) and added to FcRn-immobilized ELISA plates. Bound sgp34wt-V5-His in absence or presence of blocking mAbs is detected using anti-V5-HRP antibody. All anti-gp34mtrp-specific mAbs have the potential to block vFcγR gp34 FcRn-binding up to 50% with similar efficiencies. Only wt.07 and wt.08 mAbs raised against sgp34wt protein have an effect on FcRn-binding while all the other anti-gp34wt mAbs and anti-gp68 mAbs (as control) does not block this interaction. Mouse IgG1 and IgG2a isotypes were used for reference and should not influence FcRn-interaction. In addition, binding of sgp34wt to immobilized FcRn in absence of any mAb is depicted.

In conclusion, mAbs raised against HCMV encoded vFcyRs gp34 and gp68 are provided, exhibiting various interesting properties and even more important have the potential to interfere with IgG-binding abilities of vFcyR gp68.

Regarding vFcyR gp34, powerful mAbs are provided interfering with binding partners of gp34 involving IgG, CRP and FcRn.

The properties and blocking potentials of the anti-vFcyR gp34 mAbs are shown in Table 9.

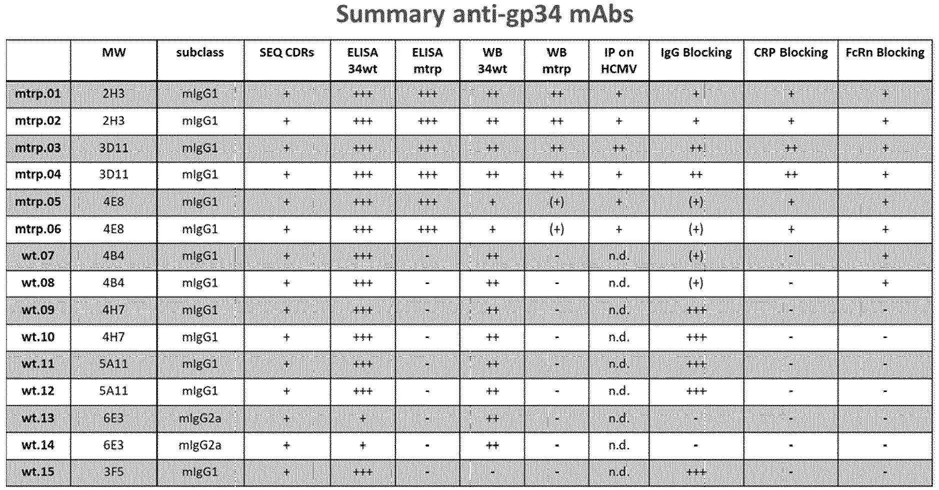

Tab. 9: Summarizing properties of anti-gp34 mAbs with respect to IgG, CRP and FcRn. We generated in total 15 anti-gp34-specific mAbs and original motherwells (MW) are indicated from hybridomas further sub-cloned by limiting dilutions. mAbs anti-gp34mtrp (immunization with sgp34mtrp protein) and mAbs anti-gp34wt (immunization with sgp34wt protein) were further characterized in ELISA, WB and precipitation experiments. Finally, all of them were tested upon their potential to block gp34wt CRP-binding and FcRn-binding resulting in candidates showing promising potential to interfere with IgG-binding, CRP-binding and/ or FcRn-binding ability of gp34wt.

### Potential to use anti-vFcyR mAbs for diagnostic applications

Both vFcyRs gp34 and gp68 are present in the HCMV virion. Therefore, it was investigated whether vFcyR gp34 can be detected as indicator for HCMV virions. These experiments were carried out using anti-gp34 mAbs according to the present invention.

The data indicate a potential for usage of anti-vFcyR mAbs for diagnostic applications. Since vFcyR gp34 represents a glycoprotein binding to human IgG, human IgG-Fc-fragment was immobilized before incubated with sera of various patient samples.

Presence of gp34 was verified using anti-gp34 mAb mtrp.04 for detection. HCMV virions were further determined using commercial anti-gB antibody. Glycoprotein B is one important surface protein of HCMV, present also within the virion.

The data indicate a potential to use anti-gp34 mAbs for detection of HCMV positive sera. Data will be further analyzed since not all HCMV-positive sera were also positive for vFcyR gp34 indicating higher gp34 levels under different circumstances e.g. re-activation of the virus. All tested sera from diagnosed HCMV-negative patients were also negative in our ELISAs using mAb anti-gp34 for detection.

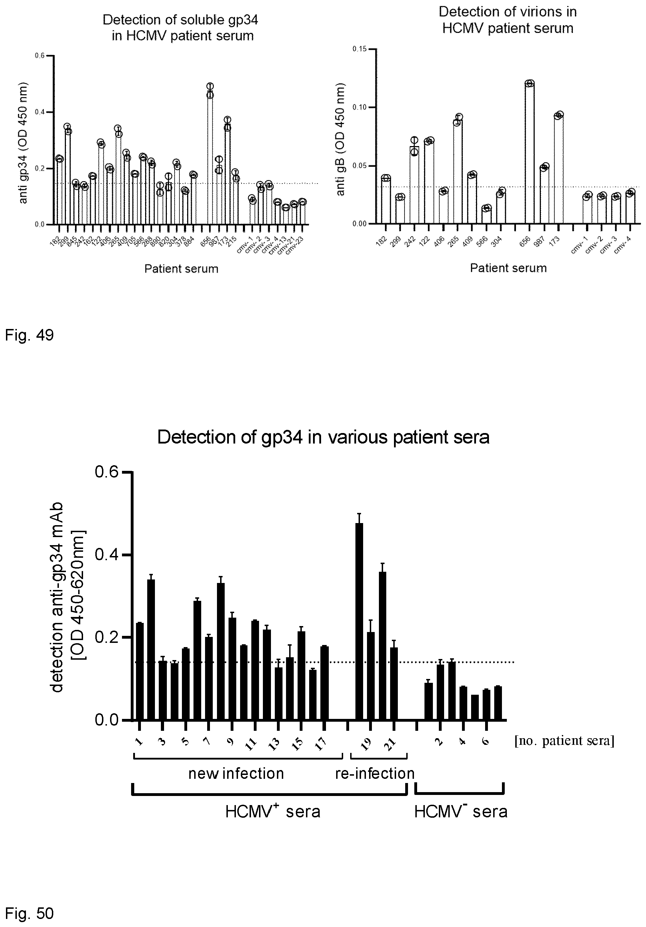

Fig. 49: Detection of vFcyRs gp34 by anti-gp34mtrp-specific mAb mtrp.04 in various patient sera. Ultra-centrifuged (at 100,000 g for 45 minutes at 4°C) sera from various donors were investigated for their HCMV-sero-positivity by usage of either commercially available anti-HCMV gB antibody or using our anti-gp34 mAb mtrp.04. Respective secondary antibody (goat-anti-mouse IgG HRP) was added to detect any gp34 or gB present in each serum indicating HCMV seropositive donors. Sera from HCMV-negative donors served as control.

Next, the data were validated further and used different sera from HCMV donors after primary infection and sera from donors after re-activation or re-infection. Again, sera from HCMV negative patients were used as negative control. This time, ELISA was performed using only anti-gp34 mAb for detection. Not all, but many sera correlated with gp34 expression and sero-positivity. This needs to be further validated and viral load within these sera has to be measured to validate if it correlates with gp34 levels.

Fig. 50: Detection of vFcyRs gp34 by anti-gp34-specific mAb mtrp.04 in HCMV+ vs. HCMV-patient sera. Ultra-centrifuged (at 100,000 g for 45 minutes at 4°C) sera from HCMV+ or HCMV- donors were incubated on an IgG-Fc coated (4 µg/ml) ELISA plate overnight at 4 degrees. Only supernatants after ultracentrifugation were analyzed, while the sediment should contain virions, exosomes and cell membranes.

The plate was blocked and incubated with antigp34mtrp mAb mtrp.04 at room temperature for 4 hrs. A secondary antibody (goat-anti-mouse IgG-HRP) was used to detect any gp34 present in each serum by spectrophotometry.

### Recombinant Fab cloning

For further analysis Fab fragments were provided based on the methodology as known to the skilled person.

In brief, hybridomas of mAbs were cultivated. RNA was isolated and transcribed into cDNA. This cDNA was used for amplification of either variable light chain or variable heavy chain und further ligated into plasmids containing either mouse IgG1 heavy chain with an intact Poly-His-Tag (pOPINVH plasmid for variable heavy chain) at the C-terminus or mouse IgG kappa light chain (pOPINVL plasmid for variable light chain). Within the light chain plasmid, a stop codon is introduced, resulting in a frame shift leading to a non-functional poly-His-Tag. Therefore, only functional heavy chain fab-fragments expresses a poly-His-Tag. In addition, heavy chains only stabilized and secreted into cell culture supernatants if connected to their respective light chains.

Recombinant Fab-fragments of anti-vFcyR gp34 and anti-gp68 mAbs were cloned and verified (i) for their secretion into supernatants after double transfection of pOPINVL- and pOPINVH-plasmids in 293-T cells detected by anti-His, (ii) their specific recognition of their appropriate antigen (comparison with full length mAbs) and (iii) recombinant fab-fragments were send for sanger-sequencing. By help of sequencing and online tools (e.g. IgBLAST from NCBI) nucleotides and amino acids of framework regions (FR1-4) and complementarity-determining regions (CDRs 1-3) of each individual mAb were determined. Sequences were applied for phylogenetic tree analysis using Genious to identify relationship and similarities of all generated anti-vFcyR mAbs (data not shown).

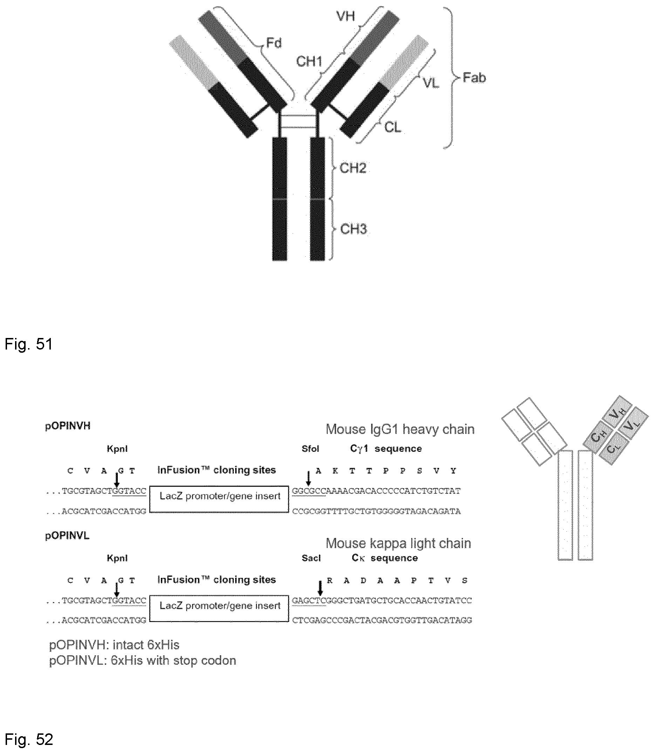

Fig. 51 is a schematic representation of human IgG1. Human immunoglobulin 1 subclass is illustrated and Fab regions are indicated comprising VH (variable heavy chain), VL (variable light chain). VH and VL of respective mAbs were amplified by PCR from hybridoma cDNA. Sequences were generated by in-Fusion cloning and inserted into pOPINVL or pOPINVH plasmids, containing in-frame mouse IgG1 heavy chain or mouse IgG kappa light chain, respectively.

Fig. 52: In-fusion cloning using pOPINVL and pOPINVH plasmids. Detailed sequencing informations of pOPINVL or pOPINVH plasmids are depicted. Those include informations like restriction enzymes used for in-fusion ligation and in-frame added elements for both plasmids: either mouse IgG1 heavy chain (pOPINVH, with intact poly-His-Tag) or mouse IgG kappa light chain (pOPINVL, with non-functional poly-His-Tag due to stop mutation introduced into the plasmids in frame in front of the original poly-His-Tag).

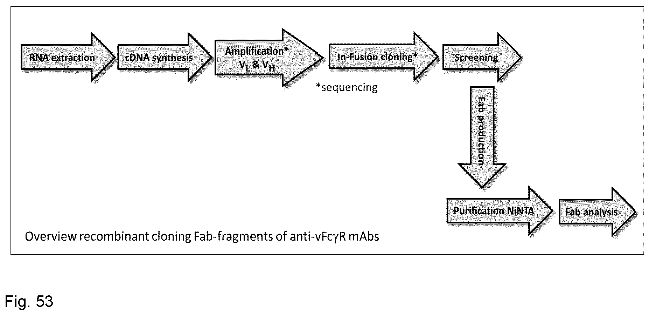

Fig. 53: Workflow generation recombinant fab-fragments of anti-vFcyR mAbs. Essential steps in cloning Fab-fragments (Fabs) of anti-vFcyR mAbs are shown, including RNA isolation from hybridoma cell culture; PCR-amplification of VL and VH fragments using hybridoma cDNAs. Constructs were cloned by in-fusion technology. Correct and functional Fab-fragments were tested for antigen-recognition and fab-expression after co-transfection of VL and VH plasmids into HEK-293-T cells. Appropriate amounts of cell culture supernatants were collected under starvation conditions (no FCS within the media) and sub following purified using NiNTA chromatography. Fab fragment were further analyzed by sequencing and functional studies.

Within a first experiment, a selection of newly generated Fab-fragments was analyzed if they remain their blocking capacity regarding IgG binding by vFcyR gp34.

Binding of sgp34wt to immobilized IgG was investigated in the presence or absence of blocking candidate. Within the experiment, full length mAbs were compared to their respective recombinant, purified Fab-fragment.

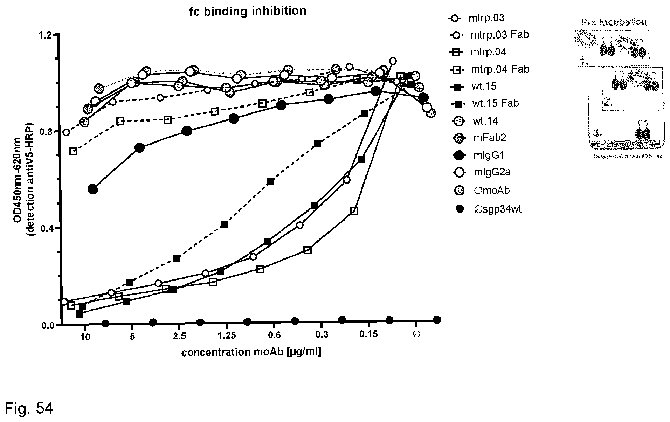

Fig. 54: IgG blocking ability by anti-gp34 mAbs. Anti-gp34-specific mAbs (memerbs of both groups) or cloned recombinant Fab-fragments thereof were pre-incubated with recombinant, soluble sgp34wt (constant amount 1µg/ml) and added to IgG-immobilized ELISA plates. Bound sgp34wt in absence or presence of blocking full-length mAbs or fab-fragments only is detected using anti-V5-HRP antibody recognizing IgG-bound sgp34wt-V5-His. Most tested anti-gp34wt-specific full length mAbs (except wt.13 and wt.14) have the potential to block vFcyR gp34 IgG-binding and for the Fab-fragment of anti-gp34wt mAb wt.15 blocking capacity remains although with a bit reduced efficiency. Whereas, blocking capacity by the fab-fragment derived from anti-gp34mtrp mAbs mtrp.03 and mtrp.04, which were sufficient before to block gp34wt-IgG binding as full length mAb, is gone. Dose-dependent IgG-blocking potential is indicated. Also, non-specific effect of total mouse IgG1, as well as mouse IgG1 fab-fragment isotype control is shown. In addition, binding of sgp34wt to immobilized IgG in absence of any full-length mAb or fab-fragment is depicted.

The first experiment verified that anti-gp34 mAb wt.15 still remains its blocking ability regarding the sgp34wt-IgG interaction, although it lost a bit of its potential. In contrast, mtrp.03 and mtrp04 mAb as Fab only fragments completely seemed to lack their blocking capacity. This result indicates that blocking effects seen before in our ELISAs were maybe due to the Fc-part. Nevertheless, experiments need to be repeated and results further analyzed in more detail. Also, mtrp mAbs, compared to some of the sgp34wt mAbs never had such a drastically blocking effect up to nearly no sgp34wt-Fc-binding in presence of those mAbs.

By cloning all respective Fab-fragments further analysis can be done. In addition, by sequencing our plasmids containing variable heavy and light chain fragments of our mAbs, we were able to determine their sequences and predict their framework regions (FR) and complementary-determining-regions (CDRs), both are essential for humanization of mAbs. Following, one representative example is shown, illustrating sequence analysis (using GeneWiz) and predictions of variable regions (using IgBlast).

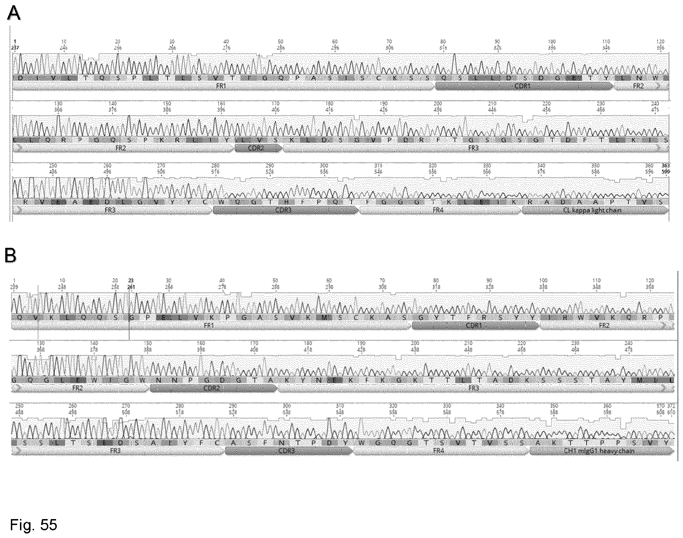

Fig. 55 shows representative variable light chain (VL) and variable heavy chain (VH) of anti-gp34 wt.15. Fab-fragment consist of one variable light chain (A) and one variable heavy chain (B). Variable light chain of hybridoma gp34wt.15 was cloned and send for sequencing (Fig. 52A). Obtained sequences were analyzed using Geneious software and IgBlast. Framework-regions (FR) as well as complementarity-determining-regions (CDRs) are indicated. Also, mouse IgG kappa light chain is indicated, contained in-frame on pOPINVL plasmids. (B) Variable heavy chain of hybridoma gp34wt.15 was cloned and send for sequencing. Obtained sequences were analyzed using Geneious software and IgBlast. Framework-regions (FR) as well as complementarity-determining-regions are indicated. Also, mouse IgG1 heavy chain is indicated, contained in-frame on pOPINVH plasmids. Also, heavy chain fragments contain a function poly-His-Tag on the C-terminus, used for detection and purification approaches.

Since all anti-vFcyR mAbs were successfully cloned, sequences were available and were analyzed e.g. by phylogenetic tree analysis comparing protein sequences to decipher relationships (data not shown).

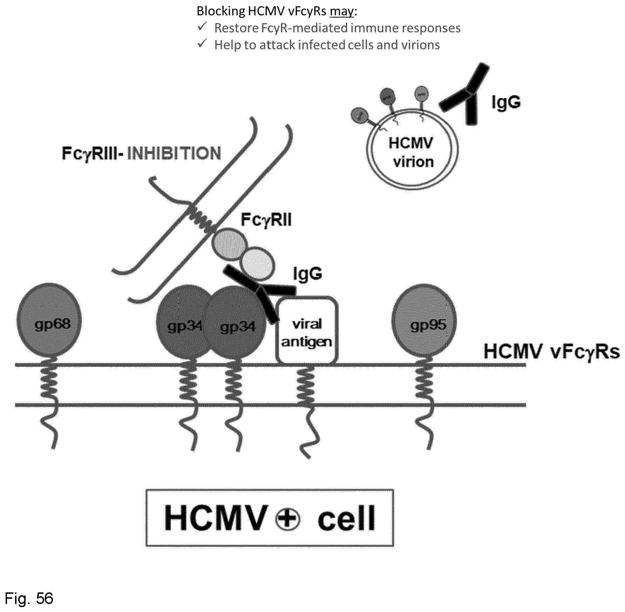

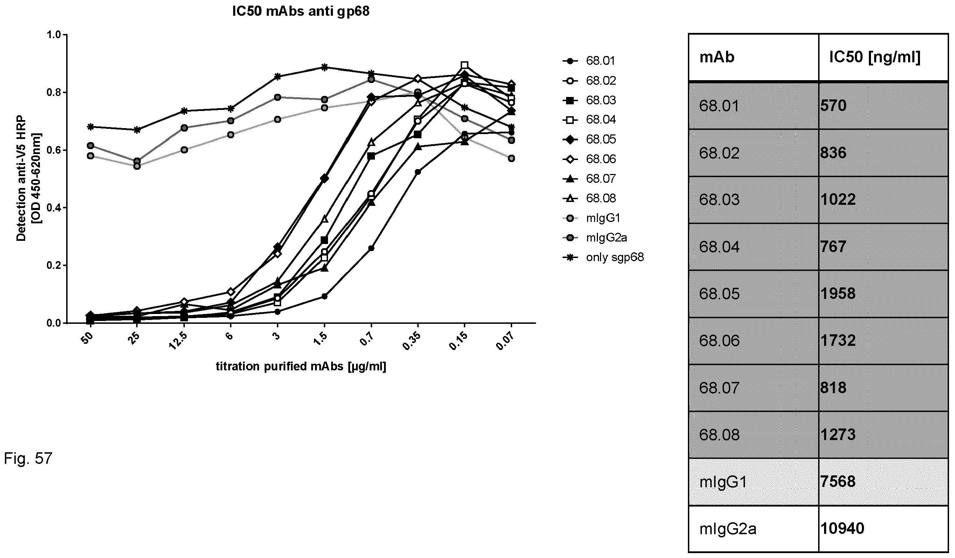

Fig. 56 shows schematically the immune evasion mechanism performed by HCMV encoded vFcyRs. HCMV-encoded vFcyRs like gp34 and gp68 expressed during infection mediate immune evasion from IgG-triggered immune responses by antagonizing host FcyR-activation. This is enabled by their property to bind human IgG-Fc thereby counteracting simultaneously interactions of host FcyRs. At least, vFcyRs gp34 and gp68 are also part of the HCMV virion, potentiallyprotecting the virion from Antibody-mediated immune-recognition.

The amino acid sequences of the preferred monoclonal antibodies are provided as follows:

Sequences including CRDs of monoclonal anti-gp68 and anti-gp34 antibodies:
1) Anti gp34 clone mtrp.01
   Heavy chain variable region polypeptide sequence, 145 amino acids
      FR1 -CDR1-FR2-CDR2-FR3-CDR3-FR4 
SEQ mtrp.01 HC1_.ab1:
MGILPSPGMPALLSLVSLLSVLLMGCVAQVKLEESGAELVKPGASVKLSCTGSGFNIKDIY
MDWVKQRPEQGLEWIGRIDPANGNSKYDPKFQGKATITVDTSSNTAYLQLSSLTSEDTA
VYYCASYYGSSYNYWGQGTTLTVSS (SEQ ID NO:14)
   Light chain variable region polypeptide sequence, 149 amino acids
      FR1 -CDR1 -FR2-CDR2-FR3 -CDR3 -FR4 
SEQ mtrp.01 LC15_ab1:
MGILPSPGMPALLSLVSLLSVLLMGCVADIVITQTPPSVPVTPGESVSISCRSSKSLLHSNG
NTYLYWFLQRPGQSPQLLIYRMSNLASGVPDRFSGSGSGTAFTLRISRVEAEDVGVYYC
MQHLEYPLTFGAGTKLELKRADAAPTVS (SEQ ID NO:15)
2) Anti gp34 clone mtrp.02
   Heavy chain variable region polypeptide sequence, 145 amino acids
      FR1 -CDR1-FR2-CDR2-FR3-CDR3-FR4 
SEQ mtrp.02 HC3_.ab1:

MGILPSPGMPALLSLVSLLSVLLMGCVAQVKLEQSGAELVKPGASVKLSCTGSGFNIKDIY
MDWVKQRPEQGLEWIGRIDPANGNSKYDPKFQGKATITVDTSSNTAYLQLSSLTSEDTA
VYYCASYYGSSYNYWGQGTTLTVSS (SEQ ID NO:16)
   Light chain variable region polypeptide sequence, 149 amino acids
      FR1 -CDR1 -FR2-CDR2-FR3 -CDR3 -FR4 
SEQ mtrp.02 LC5_.ab1:

MGILPSPGMPALLSLVSLLSVLLMGCVADIVITQTPPSVPVTPGESVSISCRSSKSLLHSNG
NTYLYWFLQRPGQSPQLLIYRMSNLASGVPDRFSGSGSGTAFTLRISRVEAEDVGVYYC
MQHLEYPLTFGAGTKLELKRADAAPTVS (SEQ ID NO:17)
3) Anti gp34 clone mtrp.03
   Heavy chain variable region polypeptide sequence, 143 amino acids
      FR1 -CDR1-FR2-CDR2-FR3-CDR3-FR4 
SEQ mtrp.03 HC1_.ab1:

MGILPSPGMPALLSLVSLLSVLLMGCVAEVQLEESGTVLARPGASVKMSCKASGYTFTSY
WMHWVKQRPGQGLEWIGAIYPGNSDTGYNQKFKGKARLTAVTSTSTAYLELSSLTDEDS
AVYFCTRLGYGDYWGQGTTLTVSS (SEQ ID NO:18)
   Light chain variable region polypeptide sequence, 135 amino acids 
SEQ_ mtrp.03 LC1_.ab1:

FR1 -CDR1 -FR2-CDR2-FR3 -CDR3 -FR4

MGILPSPGMPALLSLVSLLSVLLMGCVADIVLTQTPSSMYASLGERVTITCKASQDINSYLT
WFQQKPGKSPKTLIYRANRLVDGVPSRFSGSGSGQDYSLTINSLDYEDMGIYYCLQYDE
FPPTFGGGTKLEIK (SEQ ID NO:19)
4) Anti gp34 clone mtrp.04
   Heavy chain variable region polypeptide sequence, 143 amino acids
      FR1 -CDR1-FR2-CDR2-FR3-CDR3-FR4 
SEQ mtrp.04_HC1_.ab1:

MGILPSPGMPALLSLVSLLSVLLMGCVAEVKLVQSGTVLARPGASVKMSCKASGYTFTSY
WMHWVKQRPGQGLEWIGAIYPGNSDTGYNQKFKGKARLTAVTSTSTAYLELSSLTDEDS
AVYFCTRLGYGDYWGQGTTLTVSS (SEQ ID NO:20)
   Light chain variable region polypeptide sequence, 135 amino acids 
SEQ_ mtrp.04 LC3_.ab1:

FR1 -CDR1 -FR2-CDR2-FR3 -CDR3 -FR4

MGILPSPGMPALLSLVSLLSVLLMGCVADIVMTQTPSSMYASLGERVTITCKASQDINSYL
TWFQQKPGKSPKTLIYRANRLVDGVPSRFSGSGSGQDYSLTINSLDYEDMGIYYCLQYD
EFPPTFGGGTKLEIK (SEQ ID NO:21)
5) Anti gp34 clone mtrp.05
   Heavy chain variable region polypeptide sequence, 145 amino acids
      FR1 -CDR1-FR2-CDR2-FR3-CDR3-FR4 
SEQ mtrp.05_HC3_.ab1:

MGILPSPGMPALLSLVSLLSVLLMGCVAEVKLLQSGPEVKKPGETVKISCKASGYTFTNYG
MSWLRQAPGKGLKWMGWINTYTGEPTYADDFKGRFAFSLETSASTAYFQINNLKDEDTT
TYFCARRAFGGLDYWGQGTSVTVSS (SEQ ID NO:22)
   Light chain variable region polypeptide sequence, 140 amino acids 
SEQ_ mtrp.05 LC4_.ab1:

FR1 -CDR1 -FR2-CDR2-FR3 -CDR3 -FR4

MGILPSPGMPALLSLVSLLSVLLMGCVADIVMTQSPLSLPVSLGDQASISCRSSQSIVYTN
GNTYLEWYLQKPGQSPKLLIYKVSNRFSGVPDRFSGSGSGTDFTLKISRVEAEDLGVYYC
FQGSHVPWTFGGGTKLEIK (SEQ ID NO:23)
6) Anti gp34 clone mtrp.06
   Heavy chain variable region polypeptide sequence, 145 amino acids
      FR1 -CDR1-FR2-CDR2-FR3-CDR3-FR4 
SEQ mtrp.06_HC1_.ab1:

MGILPSPGMPALLSLVSLLSVLLMGCVAEVQLLQSGPEVKKPGETVKISCKASGYTFTNY
GMSWLRQAPGKGLKWMGWINTYTGEPTYADDFKGRFAFSLETSASTAYFQINNLKNEDT
TTYFCARRAFGGLDYWGQGTSVTVSS (SEQ ID NO:24)
   Light chain variable region polypeptide sequence, 140 amino acids 
SEQ_ mtrp.06 LC1_.ab1:

FR1 -CDR1 -FR2-CDR2-FR3 -CDR3 -FR4

MGILPSPGMPALLSLVSLLSVLLMGCVADIVLTQSPLSLPVSLGDQASISCRSSQSIVYTNG
NTYLEWYLQKPGQSPKLLIYKVSNRFSGVPDRFSGSGSGTDFTLKISRVEAEDLGVYYCF
QGSHVPWTFGGGTKLEIK (SEQ ID NO:25)
7) Anti gp34 clone wt.07
   Heavy chain variable region polypeptide sequence, 149 amino acids
      FR1 -CDR1-FR2-CDR2-FR3-CDR3-FR4 
SEQ wt.07 HC2_.ab1:

MGILPSPGMPALLSLVSLLSVLLMGCVAQVKLEESGPELVKTGASVKISCKASGYSFTSYQ
IYWVKQSHGKSLEWIGHISCYNGATSYNQKFKDKATFTVDTSSSTAYMQFNSLTSEDSAV
YFCARYDYAPDYYAMDYWGQGTSVTVSS (SEQ ID NO:26)
   Light chain variable region polypeptide sequence, 139 amino acids 
SEQ_ wt.07 LC2_.ab1:

FR1 -CDR1 -FR2-CDR2-FR3 -CDR3 -FR4

MGILPSPGMPALLSLVSLLSVLLMGCVADIVLTQTPASLAVSLGQRATISCRASESVDSYG
NSFMHWYQQKPGQPPKLLIYLASNLESGVPARFSGSGSRTDFTLTIDPVEAEDAAIYYCQ
QNNEDPLTFGGGTKLELK (SEQ ID NO:27)
8) Anti gp34 clone wt.08
   Heavy chain variable region polypeptide sequence, 149 amino acids
      FR1 -CDR1-FR2-CDR2-FR3-CDR3-FR4 
SEQ wt.08 HC3_.ab1.

MGILPSPGMPALLSLVSLLSVLLMGCVAEVQLEQSGPELVKTGASVKISCKASGYSFTSY
QIYWVKQSHGKSLEWIGHISCYNGATSYNQKFKDKATFTVDTSSSTAYMQFNSLTSEDSA
VYFCARYDYAPDYYAMDYWGQGTSVTVSS (SEQ ID NO:28)
   Light chain variable region polypeptide sequence, 139 amino acids 
SEQ_ wt.08 LC3_.ab1:

FR1 -CDR1 -FR2-CDR2-FR3 -CDR3 -FR4

MGILPSPGMPALLSLVSLLSVLLMGCVADIVMTQTPASLAVSLGQRATISCRASESVDSYG
NSFMHWYQQKPGQPPKLLIYLASNLESGVPARFSGSGSRTDFTLTIDPVEAEDAAIYYCQ
QNNEDPLTFGGGTKLELK (SEQ ID NO:29)
9) Anti gp34 clone wt.09
   Heavy chain variable region polypeptide sequence, 143 amino acids
      FR1 -CDR1-FR2-CDR2-FR3-CDR3-FR4 
SEQ wt.09 HC4_.ab1.

MGILPSPGMPALLSLVSLLSVLLMGCVAEVQLEQSGPELVKPGASVKMSCKASGYTFTRP
YIHWVKQRPGQGLEWIGWIYPGDGITKYNEKFKGKITLTADKSSSAAYMLLSSLTSEDSAI
YFCASFTTPVYWGQGTSVTVSS (SEQ ID NO:30)
   Light chain variable region polypeptide sequence, 140 amino acids 
SEQ_ wt.09 LC4_.ab1:

FR1 -CDR1 -FR2-CDR2-FR3 -CDR3 -FR4

MGILPSPGMPALLSLVSLLSVLLMGCVADIVMTQTPLTLSVTIGQPASISCKSSQSLLDSDG
KTYLNWLFQRPGQSPKRLIYLVSKLDSGVPDRFTGSGSGTDFTLKISRVEAEDLGVYYCW
QGTHFPQTFGGGTKLEIK (SEQ ID NO:31)
10) Anti gp34 clone wt.10
   Heavy chain variable region polypeptide sequence, 143 amino acids
      FR1 -CDR1-FR2-CDR2-FR3-CDR3-FR4 
SEQ wt.10 HC1_.ab1:

MGILPSPGMPALLSLVSLLSVLLMGCVAEVKLEQSGPELVKPGASVKMSCKASGYTFTRP
YIHWVKQRPGQGLEWIGWIYPGDGITKYNEKFKGKITLTADKSSSAAYMLLSSLTSEDSAI
YFCASFTTPVYWGQGTSVTVSS (SEQ ID NO:32)
   Light chain variable region polypeptide sequence, 140 amino acids 
SEQ_ wt.10 LC1_.ab1:

FR1 -CDR1 -FR2-CDR2-FR3 -CDR3 -FR4

MGILPSPGMPALLSLVSLLSVLLMGCVADIVLTQTPLTLSVTIGQPASISCESSQSLLDSDG
KTYLNWLFQRPGQSPKRLIYLVSKLDSGVPDRFTGSGSGTDFTLKICRVEAEDLGVYYC
WQGTHFPQTFGGGTKLEIK (SEQ ID NO:33)
11) Anti gp34 clone wt.11
   Heavy chain variable region polypeptide sequence, 143 amino acids
      FR1 -CDR1-FR2-CDR2-FR3-CDR3-FR4 
SEQ wt.11 HC1_.ab1:

MGILPSPGMPALLSLVSLLSVLLMGCVAQVQLEESGPELVKPGASVKMSCKASGYTVTRS
YIHWVKQRPGQGLEWIGWIYPGDGSTKYNEKFKGKTTLTVDKSSSTAYMLLSSLTSEDSA
IYFCASFTTPVYWGQGTSVTVSS (SEQ ID NO:34)
   Light chain variable region polypeptide sequence, 140 amino acids 
SEQ_wt.11 LC1_.ab1:

FR1 -CDR1 -FR2-CDR2-FR3 -CDR3 -FR4

MGILPSPGMPALLSLVSLLSVLLMGCVADIVLTQSPLTLSVTIGQPASISCKSSQSLLDSDG
ETYLNWLLQRPGQSPKRLIYLVSKLDSGVPDRFTGSGSGTDFTLKISRVEAEDLGVYYCW
QGTHFPQTFGGGTKLEIK (SEQ ID NO:35)
12) Anti gp34 clone wt.12
   Heavy chain variable region polypeptide sequence, 143 amino acids
      FR1 -CDR1-FR2-CDR2-FR3-CDR3-FR4 
SEQ wt.12 HC4_.ab1.

MGILPSPGMPALLSLVSLLSVLLMGCVAEVKLQESGPELVKPGASVKMSCKASGYTVTRS
YIHWVKQRPGQGLEWIGWIYPGDGSTKYNEKFKGKTTLTVDKSSSTAYMLLSSLTSEDSA
IYFCASFTTPVYWGQGTSVTVSS (SEQ ID NO:36)
   Light chain variable region polypeptide sequence, 140 amino acids 
SEQ_wt.12 LC1_.ab1:

FR1 -CDR1 -FR2-CDR2-FR3 -CDR3 -FR4

MGILPSPGMPALLSLVSLLSVLLMGCVADIVLTQTPLTLSVTIGQPASISCKSSQSLLDSDG
ETYLNWLLQRPGQSPKRLIYLVSKLDSGVPDRFTGSGSGTDFTLKISRVEAEDLGVYYCW
QGTHFPQTFGGGTKLEIK (SEQ ID NO:37)
13) Anti gp34 clone wt.13
   Heavy chain variable region polypeptide sequence, 145 amino acids
      FR1 -CDR1-FR2-CDR2-FR3-CDR3-FR4 
SEQ wt.13 HC2_.ab1:

MGILPSPGMPALLSLVSLLSVLLMGCVAEVQLVESGPELKKPGETVKISCKASGYAFTNY
GMNWVKQTPGKGLKWMGWINTYTGKPTYADDFKGRFAFSLETSASSAYLQINNLKNED
TATYFCARRRGNSFDYWGQGTTLTVSS (SEQ ID NO:38)
   Light chain variable region polypeptide sequence, 141 amino acids 
SEQ_ wt.13 LC4_.ab1:

FR1 -CDR1 -FR2-CDR2-FR3 -CDR3 -FR4

MGILPSPGMPALLSLVSLLSVLLMGCVADIVLTQSPSSLTVTAGEKVTMSCKSSQSLLNSG
NQENCLTWYQQKPGQPPRLLIYWASTRESGVPDRFTGSGSGTDFTLTISSVQAEDLAVY
YCQNDYTYPLTFGAGTKLELK (SEQ ID NO:39)
14) Anti gp34 clone wt.14
   Heavy chain variable region polypeptide sequence, 145 amino acids
      FR1 -CDR1-FR2-CDR2-FR3-CDR3-FR4 
SEQ wt.14 HC4_.ab1:

MGILPSPGMPALLSLVSLLSVLLMGCVAQVKLLQSGPELKKPGETVKISCKASGYAFTNY
GMNWVKQTPGKGLKWMGWINTYTGKPTYADDFKGRFAFSLETSASSAYLQINNLKNED
TATYFCARRRGNSFDYWGQGTTLTVSS (SEQ ID NO:40)
   Light chain variable region polypeptide sequence, 141 amino acids 
SEQ_ wt.14 LC1_.ab1.

FR1 -CDR1 -FR2-CDR2-FR3 -CDR3 -FR4

MGILPSPGMPALLSLVSLLSVLLMGCVADIVITQSPSSLTVTAGEKVTMSCKSSQSLLNSG
NQENCLTWYQQKPGQPPRLLIYWASTRESGVPDRFTGSGSGTDFTLTISSVQAEDLAVY
YCQNDYTYPLTFGAGTKLELK (SEQ ID NO:41)
15) Anti gp34 clone wt.15
   Heavy chain variable region polypeptide sequence, 143 amino acids
      FR1 -CDR1-FR2-CDR2-FR3-CDR3-FR4 
SEQ wt.15 HC1_.ab1:

MGILPSPGMPALLSLVSLLSVLLMGCVAQVKLQQSGPELVKPGASVKMSCKASGYTFRS
YYIHWVKQRPGQGLEWIGWNNPGDGTAKYNEKFKGKTTLTADKSSSTAYMLLSSLTSED
SAIYFCASFNTPDYWGQGTSVTVSS (SEQ ID NO:42)
   Light chain variable region polypeptide sequence, 140 amino acids 
SEQ_ wt.15 LC1_.ab1:

FR1 -CDR1 -FR2-CDR2-FR3 -CDR3 -FR4

MGILPSPGMPALLSLVSLLSVLLMGCVADIVLTQSPLTLSVTIGQPASISCKSSQSLLDSDG
ETYLNWLLQRPGQSPKRLIYLVSKLDSGVPDRFTGSGSGTDFTLKISRVEAEDLGVYYCW
QGTHFPQTFGGGTKLEIK (SEQ ID NO:43)
16) Anti gp68 clone 68.01
   Heavy chain variable region polypeptide sequence, 146 amino acids
      FR1 -CDR1-FR2-CDR2-FR3-CDR3-FR4 
SEQ 68.01 HC2_.ab1:

MGILPSPGMPALLSLVSLLSVLLMGCVAEVKLLESGPELVKPGSSVKISCKASGYSFADHII
LWVKQSHGKSLEWIGNINPYSGNTIYNLNFKGKATLTVDKSSSTAYMQLNSLTSEDSAVY
YCARIEGYYSMDYWGQGTSVTVSS (SEQ ID NO:44)
   Light chain variable region polypeptide sequence, 140 amino acids 
SEQ_68.01 LC4_.ab1:

FR1 -CDR1 -FR2-CDR2-FR3 -CDR3 -FR4

MGILPSPGMPALLSLVSLLSVLLMGCVADIVMTQTPLSLPVSLGDQASISCRSSQSIVHSN
GNTYLEWYLQKPGQSPKVLIYKVSKRFSGVPDRFSGSGSGTDFTLKISRVEAEDMGVYY
CFQGSHVPLTFGAGTKLELK (SEQ ID NO:45)
17) Anti gp68 clone 68.02
   Heavy chain variable region polypeptide sequence, 146 amino acids
      FR1 -CDR1-FR2-CDR2-FR3-CDR3-FR4 
SEQ 68.02 HC2_.ab1:

MGILPSPGMPALLSLVSLLSVLLMGCVAEVKLLQSGPELVKPGSSVKISCKASGYSFADHII
LWVKQSHGKSLEWIGNINPYSGNTIYNLNFKGKATLTVDKSSSTAYMQLNSLTSEDSAVY
YCARIEGYYSMDYWGQGTSVTVSS (SEQ ID NO:46)
   Light chain variable region polypeptide sequence, 140 amino acids 
SEQ_ 68.02 LC2_.ab1:

FR1 -CDR1 -FR2-CDR2-FR3 -CDR3 -FR4

MGILPSPGMPALLSLVSLLSVLLMGCVADIVLTQTPLSLPVSLGDQASISCRSSQSIVHSNG
NTYLEWYLQKPGQSPKVLIYKVSKRFSGVPDRFSGSGSGTDFTLKISRVEAEDMGVYYC
FQGSHVPLTFGAGTKLELK (SEQ ID NO:47)
18) Anti gp68 clone 68.03
   Heavy chain variable region polypeptide sequence, 146 amino acids
      FR1 -CDR1-FR2-CDR2-FR3-CDR3-FR4 
SEQ 68.03 HC2_.ab1.

MGCVAEVKLEESGPEVKKPGETVKISCKASGYTFTNYGMSWLRQAPGKGLKWMGWINT
YTGEPTYADDFKGRFAFSLETSASTAYFQINNLKDEDTTTYFCARRAFGGLDYWGQGTS
VTVFSAKTTPPSVY (SEQ ID NO:48)
   Light chain variable region polypeptide sequence, 149 amino acids 
SEQ_ 68.03 LC8_.ab1:

FR1 -CDR1 -FR2-CDR2-FR3 -CDR3 -FR4

MGILPSPGMPALLSLVSLLSVLLMGCVADIVLTQTPLSLPVSLGDQASISCRSSQSIVHSNG
NTYLEWYLQKPGQSPKVLIYKVSKRFSGVPDRFSGSGSETDFTLKISRVEAEDMGVYYCF
QGSHVPLTFGAGTKLELKRADAAPTVS (SEQ ID NO:49)
19) Anti gp68 clone 68.04
   Heavy chain variable region polypeptide sequence, 154 amino acids
      FR1 -CDR1-FR2-CDR2-FR3-CDR3-FR4 
SEQ 68.04 HC4 1/3_.ab1:

MGILPSPGMPALLSLVSLLSVLLMGCVAEVQLQESGPEVKKPGETVKISCKASGYTFTNY
GMSWLRQAPGKGLKWMGWINTYTGEPTYADDFKGRFAFSLETSASTAYFQINNLKNEDT
TTYFCARRAFGGLDYWGQGTSVTVSSAKTTPPSVY (SEQ ID NO:50)
   Light chain variable region polypeptide sequence, 146 amino acids
      FR1 -CDR1-FR2-CDR2-FR3-CDR3-FR4
      SEQ 68.04 LC3_.ab1: 
MGILPSPGMPALLSLVSLLSVLLMGCVADIVLTQTPLSLPVSLGDQASISCRSSQNIVHSNG
NTYLEWYLQKPGQSPKLLIYKVSNRFSGVPDRFSGSGSGTDFTLKISRVEAEDLGVYYCF
QGSHVPYTFGGGTKLEVKRADAAPTVS (SEQ ID NO:51)
20) Anti gp68 clone 68.05
   Heavy chain variable region polypeptide sequence, 158 amino acids
      FR1 -CDR1-FR2-CDR2-FR3-CDR3-FR4
      SEQ 68.05 HC8_.ab1: 
MGILPSPGMPALLSLVSLLSVLLMGCVAQVKLEESGAELMKPGASVKISCTATGYTFSGY
WIEWVKQRPGHGLEWIGEILPGSGGTNYNEKFKGKATFTADTSSNTAYMQLSSLTSEDS
AVYYCARGIHYFGYYYFDYWGQGTTLTVSSAKTTPPSVY (SEQ ID NO:52)
   Light chain variable region polypeptide sequence, 144 amino acids
      FR1 -CDR1-FR2-CDR2-FR3-CDR3-FR4
      SEQ 68.05 LC1_.ab1: 
MGILPSPGMPALLSLVSLLSVLLMGCVADIVLTQTPSSLSASLGDRVTISCRASQDISNYLN
WYQLKPDGTVKLLIYYTSRLHSGVPSRFSGSGSGTDYSLTISNLEQEDIATYFCQQGNTL
PPTFGGGTKLEIKRADAAPTVS (SEQ ID NO:53)
21) Anti gp68 clone 68.06
   Heavy chain variable region polypeptide sequence, 158 amino acids
      FR1 -CDR1-FR2-CDR2-FR3-CDR3-FR4
      SEQ 68.06 HC8_.ab1: 
MGILPSPGMPALLSLVSLLSVLLMGCVAQVKLEQSGAELMKPGASVKISCTATGYTFSGY
WIEWVKQRPGHGLEWIGEILPGSGGTNYNEKFKGKATFTADTSSNTAYMQLSSLTSEDS
AVYYCARGIHYFGYYYFDYWGQGTTLTVSSAKTTPPSVY (SEQ ID NO:54)
   Light chain variable region polypeptide sequence, 144 amino acids
      FR1 -CDR1-FR2-CDR2-FR3-CDR3-FR4
      SEQ 68.06 LC2_.ab1: 
MGILPSPGMPALLSLVSLLSVLLMGCVADIVLTQSPSSLSASLGDRVTISCRASQDISNYLN
WYQLKPDGTVKLLIYYTSRLHSGVPSRFSGSGSGTDYSLTINNLEQEDIATYFCQQGNTL
PPTFGGGTKLEIKRADAAPTVS (SEQ ID NO:55)
22) Anti gp68 clone 68.07
   Heavy chain variable region polypeptide sequence, 159 amino acids
      FR1 -CDR1-FR2-CDR2-FR3-CDR3-FR4
      SEQ 68.07 HC1_.ab1: 
MGILPSPGMPALLSLVSLLSVLLMGCVAEVKLVQSGGGLVQPGGSLRLSCATSGFTFSDF
YMEWVRQPPGKRLEWIAASRNKANDYATEYSASVKGRFIVSRDTSQSILYLQMNALRAE
DTAIYYCARDAGDYWGYFNVWGAGTTVTVSSAKTTPPSVY (SEQ ID NO:56)
   Light chain variable region polypeptide sequence, 150 amino acids
      FR1 -CDR1-FR2-CDR2-FR3-CDR3-FR4 
SEQ 68.07 LC1_.ab1:

MGILPSPGMPALLSLVSLLSVLLMGCVADIVMTQTPSSLGVSVGEKVTMSCKSSQSLLYS
SNQKNYLAWYQQKPGQSPKLLIYWASTRESGVPDRFTGSGSGTDFTLTISSVKAEDLAV
YYCQQYYSYPLTFGAGTKLELKRADAAPTVS (SEQ ID NO:57)
23) Anti gp68 clone 68.08
   Heavy chain variable region polypeptide sequence, 159 amino acids
      FR1 -CDR1-FR2-CDR2-FR3-CDR3-FR4 
SEQ 68.08 HC8_.ab1:

MGILPSPGMPALLSLVSLLSVLLMGCVAQVQLVESGGGLVQPGGSLRLSCATSGFTFSDF
YMEWVRQPPGKRLEWIAASRNKANDYATEYSASVKGRFIVSRDTSQSILYLQMNALRAE
DTAIYYCARDAGDYWGYFNVWGAGTTVTVSSAKTTPPSVY (SEQ ID NO:58)
   Light chain variable region polypeptide sequence, 150 amino acids
      FR1 -CDR1-FR2-CDR2-FR3-CDR3-FR4

SEQ 68.08 LC4_ab1:

MGILPSPGMPALLSLVSLLSVLLMGCVADIVITQSPSSLGVSVGEKVTMSCKSSQSLLYSS
NQKNYLAWYQQKPGQSPKLLIYWASTRESGVPDRFTGSGSGTDFTLTISSVKAEDLAVY
YCQQYYSYPLTFGAGTKLELKRADAAPTVS (SEQ ID NO:59).

In the above represented sequences the CDRs are underlined and the amino acids forming the CDRs are printed in bold. From the sequences it is evident for a person skilled in the art how the CDRs are oriented. Moreover, it goes without saying that it is possible to combine the CDRs as shown in the above sequences with each other. The sequences and the CDRs must match together. The above provided sequences represent preferred embodiments of the invention.

Figure 57

## Claims

1. Antibody which binds to the extracellular domain of an Fc-gamma-binding glycoprotein of human cytomegalovirus.

2. Antibody according to claim 1 wherein the Fc-gamma-binding glycoprotein is gp34.

3. Antibody according to claim 2 which binds to the extracellular domain of glycoprotein gp34 having SEQ ID NO:1.

4. Antibody according to claim 2 which binds to mutated extracellular domain of glycoprotein gp34 having SEQ ID NO:2, (mtrp).

5. Antibody according to any of claims 1 to 4 which blocks the binding of glycoprotein gp34 to the Fc part of immunoglobulin gamma or to the IgG B cell receptor.

6. Antibody according to any of claims 1 to 5 which blocks the binding of glycoprotein gp34 to C-reactive protein.

7. Antibody according to claim 6 whereby the blocking of glycoprotein gp34 to C-reactive protein restores the function of C-reactive protein.

8. Antibody according to any of claims 1 to 5 which blocks the binding of glycoprotein gp34 to the neonatal Fc receptor.

9. Antibody according to claim 8 wherein the antibody blocking the binding of glycoprotein gp34 to the neonatal Fc receptor restores the natural function of the neonatal Fc receptor.

10. Antibody according to claim 1 wherein the Fc-gamma-binding glycoprotein is gp68.

11. Antibody according to claim 10 which binds to the extracellular domain of glycoprotein gp68 (SEQ ID NO:3), sgp68.

12. Antibody according to claim 11 which blocks the binding of glycoprotein gp68 to the Fc part of immunoglobulin gamma.

13. Humanized antibody which is derived from any of the antibodies according to claims 1 to 12.

14. Humanized antibody according to claim 13 having the CDRs of any of the antibodies according to claims 1 to 12.

15. Method of detecting the presence of human cytomegalovirus by reacting a biological sample derived from a potentially infected subject with an antibody according to any of claims 1 to 14 whereby the antibodies or antigen binding fragments thereof are labeled and wherein subsequently unbound antibodies or antigen binding fragments thereof are separated from the sample and causing the label to form a signal which is subsequently identified quantitatively or qualitatively whereby the signal correlates with the Fc-gamma-binding glycoprotein.
